# EUROPEAN PATENT SPECIFICATION

(11) **EP 3 317 424 B1**
(45) Date of publication and mention of the grant of the patent: **06.09.2023**
(21) Application number: 16738066.6
(22) Date of filing: 01.07.2016
(51) Int. Cl.: C12Q 1/6806, C12Q 1/6809

(54) **METHOD FOR ANALYSIS OF AN RNA MOLECULE**
VERFAHREN ZUR ANALYSE EINES RNA-MOLEKÜLS
PROCÉDÉ D'ANALYSE D'UNE MOLÉCULE D'ARN

(30) Priority: 01.07.2015 WO PCT/EP2015/001336
(43) Date of publication of application: 09.05.2018
(60) Divisional application: 23169549.5
(73) Proprietor: CureVac Manufacturing GmbH, 72076 Tübingen (DE)
(72) Inventor: WOCHNER, Aniela, 72706 Tübingen (DE); EBER, Fabian Johannes, 72706 Tübingen (DE)
(74) Representative: Graf von Stosch, Andreas
(86) International application number: PCT/EP2016/001121
(87) International publication number: WO 2017/001058

(56) References cited:
- WO-A1-00/70039
- WO-A1-96/40906
- WO-A1-2008/077592
- WO-A1-2014/144039
- WO-A1-2014/152659
- WO-A1-2015/101416
- UHLENBECK O C: "A small catalytic oligoribonucleotide.", NATURE 1987 AUG 13-19, vol. 328, no. 6131, 13 August 1987 (1987-08-13), pages 596-600, XP002755436, ISSN: 0028-0836
- YEN L ET AL: "SEQUENCE-SPECIFIC CLEAVAGE OF HUNTINGTIN MRNA BY CATALYTIC DNA", ANNALS OF NEUROLOGY, JOHN WILEY AND SONS, BOSTON, US, vol. 46, no. 3, 1 September 1999 (1999-09-01), pages 366-373, XP008031730, ISSN: 0364-5134, DOI: 10.1002/1531-8249(199909)46:3<366::AID-ANA 12>3.0.CO;2-D
- LEIBER A ET AL: "SELECTION OF EFFICIENT CLEAVAGE SITES IN TARGET RNAS BY USING A RIBOZYME EXPRESSION LIBRARY", MOLECULAR AND CELLULAR BIOLOGY, AMERICAN SOCIETY FOR MICROBIOLOGY, WASHINGTON, US, vol. 15, no. 1, 1 January 1995 (1995-01-01), page 540/541, XP001015446, ISSN: 0270-7306
- DENMAN R B: "CLEAVAGE OF FULL-LENTH BETAAPP MRNA BY HAMMERHEAD RIBOZYMES", NUCLEIC ACIDS RESEARCH, INFORMATION RETRIEVAL LTD, GB, vol. 21, no. 17, 25 August 1993 (1993-08-25), pages 4119-4125, XP000567877, ISSN: 0305-1048
- TCHEREPANOVA IRINA Y ET AL: "Ectopic expression of a truncated CD40L protein from synthetic post-transcriptionally capped RNA in dendritic cells induces high levels of IL-12 secretion", BMC MOLECULAR BIOLOGY, BIOMED CENTRAL LTD, GB, vol. 9, no. 1, 17 October 2008 (2008-10-17), page 90, XP021042442, ISSN: 1471-2199, DOI: 10.1186/1471-2199-9-90 cited in the application
- THEUS S A ET AL: "A simple assay for determining the capping efficiencies of RNA polymerases used for in vitro transcription", BIOTECHNIQUES, INFORMA HEALTHCARE, US, vol. 9, no. 5, 1 November 1990 (1990-11-01), pages 610-612,614, XP009179072, ISSN: 0736-6205
- KORE A R ET AL: "Synthesis and evaluation of 2'-O-allyl substituted dinucleotide cap analog for mRNA translation", BIOORGANIC & MEDICINAL CHEMISTRY, PERGAMON, GB, vol. 18, no. 22, 15 November 2010 (2010-11-15), pages 8061-8065, XP027452470, ISSN: 0968-0896, DOI: 10.1016/J.BMC.2010.09.013 [retrieved on 2010-09-19]
- KORE A R ET AL: "Synthesis and biological validation of N<7>-(4-chlorophenoxyethyl) substituted dinucleotide cap analogs for mRNA translation", BIOORGANIC & MEDICINAL CHEMISTRY, PERGAMON, GB, vol. 21, no. 15, 1 August 2013 (2013-08-01), pages 4570-4574, XP002729762, ISSN: 0968-0896, DOI: 10.1016/J.BMC.2013.05.041 [retrieved on 2013-06-01]
- SINGH R ET AL: "Capping of mammalian U6 small nuclear RNA in vitro is directed by a conserved stem-loop and AUAUAC sequence: conversion of a noncapped RNA into a capped RNA", MOLECULAR AND CELLULAR BIOLOGY, AMERICAN SOCIETY FOR MICROBIOLOGY, WASHINGTON, US, vol. 10, no. 3, 1 March 1990 (1990-03-01), pages 939-946, XP002729763, ISSN: 0270-7306, DOI: 10.1128/MCB.10.3.939
- MCCALL M J ET AL: "Small, efficient hammerhead ribozymes", MOLECULAR BIOTECHNOLOGY, HUMANA PRESS, INC, US, vol. 14, no. 1, 1 January 2000 (2000-01-01), pages 5-17, XP002740393, ISSN: 1073-6085, DOI: 10.1385/MB:14:1:5
- KORE ET AL: "Synthesis and biological evaluation of trimethyl-substituted cap analogs", BIOORGANIC & MEDICINAL CHEMISTRY LETTERS, PERGAMON, AMSTERDAM, NL, vol. 18, no. 3, 4 January 2008 (2008-01-04), pages 880-884, XP022475650, ISSN: 0960-894X, DOI: 10.1016/J.BMCL.2007.12.049
- SCHUBERT S ET AL: "Ribozyme- and deoxyribozyme-strategies for medical applications", CURRENT DRUG TARGETS, BENTHAM SCIENCE PUBLISHER, US, vol. 5, no. 8, 1 November 2004 (2004-11-01), pages 667-681, XP009180178, ISSN: 1389-4501
- JEMIELITY J ET AL: "Novel anti-reverse cap analogs with superior translational properties", RNA, COLD SPRING HARBOR LABORATORY PRESS, US, vol. 9, no. 9, 1 September 2003 (2003-09-01), pages 1108-1122, XP002378472, ISSN: 1355-8382, DOI: 10.1261/RNA.5430403
- H. SCHURER ET AL: "A universal method to produce in vitro transcripts with homogeneous 3' ends", NUCLEIC ACIDS RESEARCH, vol. 30, no. 12, 15 June 2002 (2002-06-15) , pages 56e-56, XP055514558, DOI: 10.1093/nar/gnf055
- SRIRAM B ET AL: "Targeted cleavage of hepatitis E virus 3' end RNA mediated by hammerhead ribozymes inhibits viral RNA replication", VIROLOGY, ELSEVIER, AMSTERDAM, NL, vol. 312, no. 2, 1 August 2003 (2003-08-01), pages 350-358, XP004446044, ISSN: 0042-6822, DOI: 10.1016/S0042-6822(03)00259-9

## Description

The present invention relates to the field of RNA analysis. In particular, the present disclosure concerns the use of a catalytic nucleic acid molecule for the analysis of an RNA molecule and/or of a population of RNA molecules. In one aspect, the invention concerns methods as defined by the attached claims for analyzing mRNA molecules having at least one cleavage site for at least one catalytic nucleic acid molecule. In particular, the invention concerns a method according to the claims for determining the presence and/or integrity of a homopolymeric sequence in the 3'-UTR of an mRNA molecule or of a population of mRNA molecules. Moreover, the present invention provides novel uses of a catalytic nucleic acid molecule for analyzing mRNA molecules as defined in the attached claims. In particular, the invention relates to the use of a catalytic nucleic acid molecule in a method for analyzing mRNA molecules according to the claims, wherein the resulting 3' terminal RNA fragment and optionally the at least one optional central RNA fragment are analyzed.

Therapeutic RNA molecules represent an emerging class of drugs. RNA-based therapeutics include mRNA molecules encoding antigens for use as vaccines. mRNA vaccines combine desirable immunological properties with the flexibility of genetic vaccines. In addition, mRNA is considered to be a safer vector than DNA-based vectors because RNA cannot integrate into genomic DNA possibly leading to insertional mutagenesis. In addition, it is envisioned to use mRNA therapeutics for replacement therapies, e.g. providing missing proteins such as growth factors or enzymes to patients (Schlake et al., 2012. RNA Biol. 9(11):1319-30). Furthermore, other RNA molecules such as antisense RNA, small interfering (si)RNA, ribozymes, aptamers, immunostimulating RNA etc. are envisioned as therapeutics.

Successful protein expression from transfected RNA depends on transfection efficiency, RNA stability and translation efficiency. The 5' terminal as well as the 3' terminal region of an RNA molecule are known to be involved in the regulation of the mRNA stability and translation efficiency. For example, the 5' cap structure and the 3' poly(A) tail are important features for the efficient translation of mRNA and protein synthesis in eukaryotic cells. Singh et al. described a method for capping U6 small nuclear RNA in vitro (Singh et al.: "Capping of Mammalian U6 Small Nuclear RNA In Vitro Is Directed by a Conserved Stem-Loop and AUAUAC Sequence: Conversion of a Noncapped RNA into a Capped RNA", Molecular and Cellular Biology 1990, vol. 10, no. 3: 939-946). Several techniques were described for analyzing the capping efficiencies of RNA polymerases and of certain cap structures (see e.g. Theus S.A. and Charles D.L.: "A Simple Assay for Determining the Capping Efficiencies of RNA Polymerases Used for In Vitro Transcription", BioTechniques 1990, vol. 9, no. 5: 610-615 or Kore A.R. and Irudaya C.: "Synthesis and evaluation of 2'-O-allyl substituted dinucleotide cap analog for mRNA translation", Bioorganic & Medicinal Chemistry 2010, 18: 8061-8065).However, also 5'-untranslated regions (5'-UTR's) and 3'-untranslated regions (3'-UTR's) were found to play similar roles in the regulation of mRNA stability and translation efficiency.

Short RNA molecules can be synthesized by chemical methods, whereas long RNAs are typically produced by *in vitro* transcription using suitable DNA templates with a promoter and RNA polymerases, for example bacteriophage SP6, T3 or T7 RNA polymerases.

Small catalytic oligoribonucleotides were described, which can cause specific cleavage of a 24-nucleotide RNA fragment (Uhlenbeck O.C.: "A small catalytic oligoribonucleotide", Nature 1987, vol. 328, no. 6131: 596-600). Another report described a strategy for expression of ribozymes and for the selection of efficient cleavage sites in target RNAs (Lieber A. and Strauss M.: "Selection of Efficient Cleavage Sites in Target RNAs by Using a Ribozyme Expression Library", Molecular and Cellular Biology 1995, vol. 15, no. 1: 540-551). The use of catalytic DNA for sequence-specific cleavage of Huntingtin mRNA was further reported (Yen et al.: "Sequence-Specific Cleavage of Huntingtin mRNA by Catalytic DNA", Annals of Neurology 1999, vol. 46, no. 3: 366-373). Hammerhead ribozymes were further described, which cleave Alzheimer amyloid precursor mRNA (Denman R.B.: "Cleavage of Full-Length ßApp mRNA by Hammerhead Ribozymes", Nucleic Acids Research 1993, vol. 21, no. 17: 4119-4125). Another report described the cleavage of chimpanzee mitochondrial tRNA^{Phe} by the hepatitis delta virus ribozyme (Schürer et al.: "A universal method to produce in vitro transcripts with homogeneous 3'ends", Nucleic Acids Research 2002, vol. 30, no. 12: 1-5). Sriram et al. used hammerhead ribozymes for cleaving the genomic RNA of hepatitis E virus in order to inhibit viral replication (Sriram et al.: "Targeted cleavage of hepatitis E virus 3' end RNA mediated by hammerhead ribozymes inhibits viral RNA replication", Virology 2003, 312: 350-358). The international patent application WO 00/70039 further described a method to modify an RNA population, in particular a method for subtracting cDNAs by suppressing the synthesis of specifically targeted mRNAs.

For any application of RNA in a scientific or therapeutic setting, it is highly desired or mandatory to use RNA with a defined sequence that can be reproduced in a reliable manner.

Particularly for therapeutic purposes it is requested by the authorities to control the composition of the drug. Therefore it is highly desired or mandatory to control the identity and/or integrity of the RNA molecules or of the RNA population comprised in the drug. Several methods for characterizing mRNA molecules during mRNA production were described in international patent application WO 2014/144039. But currently no quick, cheap and reliable method is available to analyze the identity and/or integrity of an RNA molecule or an RNA population. Only sequencing of cDNA synthesized from the RNA or RT (Reverse-Transcription)-PCR can be conducted, which implicates several problems. Mutations can be introduced into the cDNA due to the error rate of the reverse transcription leading to wrong results in the control of the RNA identity and/or integrity. One further problem of cDNA sequencing is that homopolmyer structures such as a poly(A) sequence, a poly(C) sequence or repeat sequences (e.g. tandem repeats in open reading frames) cannot be analyzed correctly by sequencing. Therefore the determination of the sequence identity of such sequences is a major problem, particularly if homopolymer structures such as a poly(A) and/or poly(C) sequence are present in the 3' terminal region of the RNA molecule. Additionally, such an indirect method for the analysis of the sequence identity and/or integrity (e.g. cDNA sequencing) is time-consuming and therefore it is not possible to get a result of the analysis in the short term, parallel to the production process. Thus, it is desired to have a quick and cheap and reliable method in place to analyze the sequence identity and/or integrity of the RNA molecule or of the RNA molecules comprised in the RNA population.

It is thus one of the objects of the present invention as defined by the amended claims to provide a method for analyzing mRNA, particularly for analyzing the (sequence) identity and/or integrity of a mRNA molecule. In particular, a method shall be provided, which is suitable for use in quality control during or following production of RNA, especially of mRNA, which is intended to be used for diagnostic or therapeutic purposes. Furthermore, it is an object of the present invention to provide a method for analyzing a mixture of mRNA molecules or a mRNA population. It is further a particular object of the present invention to provide a method for analyzing mRNA, wherein at least one mRNA fragment e.g. the 3' terminal fragments and/or optional central RNA fragments can be analyzed.

The objects underlying the present invention are solved by the claimed subject-matter.

The present invention as defined by the claims relates to a method for determining the presence and/or integrity of a homopolymeric sequence in the 3'-UTR of an mRNA molecule, the mRNA molecule having at least one cleavage site for at least one catalytic nucleic acid molecule, wherein the mRNA molecule is derived from an in vitro transcription reaction and wherein at least one cleavage site is located 5' of the homopolymeric sequence, the method comprising the steps of:
a) providing the mRNA molecule having at least one cleavage site for at least one catalytic nucleic acid molecule, wherein at least one cleavage site is located 5' of the homopolymeric sequence in the 3'-UTR of the mRNA molecule,
b) cleaving the mRNA molecule with the at least one catalytic nucleic acid molecule into a 5' terminal RNA fragment, a 3' terminal RNA fragment and optionally into at least one central RNA fragment by contacting the mRNA molecule with the at least one catalytic nucleic acid molecule under conditions allowing the cleavage of the mRNA molecule, and
c) determining the presence and/or the integrity of the homopolymeric sequence in the 3'-UTR of the mRNA molecule by analyzing the 3' terminal RNA fragment and optionally the at least one optional central RNA fragment obtained in step b), wherein step c) comprises separating the 3' terminal RNA fragment by denaturing gel electrophoresis or liquid chromatography, determining the mass and/or the length of the 3' terminal RNA fragment and comparing the determined mass and/or length of the 3' terminal RNA fragment with a respective mass and/or length of a reference RNA fragment.

In further non-claimed embodiments, the present disclosure also provides, *inter alia,* a method for analyzing an RNA molecule having at least one cleavage site for at least one catalytic nucleic acid molecule, the method comprising the steps of:
a) providing an RNA molecule having at least one cleavage site for at least one catalytic nucleic acid molecule,
b) cleaving the RNA molecule with the at least one catalytic nucleic acid molecule into a 5' terminal RNA fragment, a 3' terminal RNA fragment and optionally into at least one central RNA fragment by contacting the RNA molecule with the at least one catalytic nucleic acid molecule under conditions allowing the cleavage of the RNA molecule,
c) determining a physical property of the RNA molecule by analyzing the 5' terminal RNA fragment, the 3' terminal RNA fragment and/or the at least one optional central RNA fragment.

In particular, the RNA molecule comprises at least 1, 2, 3, 4, 5, 6, 7, 8, 9 or 10 cleavage sites for at least one catalytic nucleic acid molecule.

Additionally, it is preferred that the RNA molecule comprises cleavage sites for at least 1, 2, 3, 4, 5, 6, 7, 8, 9 or 10 different catalytic nucleic acid molecules.

Furthermore, the RNA molecule preferably comprises at least one cleavage site for a first catalytic nucleic acid molecule and at least one cleavage site for a second catalytic nucleic acid molecule.

The RNA molecule preferably comprises two or three cleavage sites, more preferably one cleavage site, for a catalytic nucleic acid. In certain embodiments, the RNA molecule comprises one, two or three cleavage sites for a first catalytic nucleic acid molecule and one, two or three cleavage sites for a second or further catalytic nucleic acid molecule.

More preferably, the RNA molecule comprises one cleavage site for a first catalytic nucleic acid molecule and one cleavage site for a second or further catalytic nucleic acid molecule.

The RNA molecule preferably comprises at least one cleavage site for at least one catalytic nucleic acid molecule, wherein the cleavage site is a unique cleavage site with respect to the at least one catalytic nucleic acid molecule. In this context, the term 'unique cleavage site' typically refers to a cleavage site, which is cleaved by a catalytic nucleic acid molecule and which is present only once in the RNA molecule to be analyzed.

The present disclosure relates to a method for analyzing an RNA molecule having at least one cleavage site for at least one catalytic nucleic acid molecule, the method comprising the steps of:
a) providing an RNA molecule having at least one cleavage site for at least one catalytic nucleic acid molecule,
b) cleaving the RNA molecule with the at least one catalytic nucleic acid molecule into a 5' terminal RNA fragment, a 3' terminal RNA fragment and optionally into at least one central RNA fragment by contacting the RNA molecule with the at least one catalytic nucleic acid molecule under conditions allowing the cleavage of the RNA molecule,
c) determining a physical property of the RNA molecule by analyzing the 3' terminal RNA fragment and/or the at least one optional central RNA fragment.

The present disclosure relates to a method for analyzing an RNA molecule having at least two cleavage sites for at least one catalytic nucleic acid molecule, the method comprising the steps of:
a) providing an RNA molecule having at least two cleavage sites for at least one catalytic nucleic acid molecule,
b) cleaving the RNA molecule with the at least one catalytic nucleic acid molecule into a 5' terminal RNA fragment, a 3' terminal RNA fragment and into at least one central RNA fragment by contacting the RNA molecule with the at least one catalytic nucleic acid molecule under conditions allowing the cleavage of the RNA molecule,
c) determining a physical property of the RNA molecule by analyzing the 5' terminal RNA fragment or the 3' terminal RNA fragment and the at least one optional central RNA fragment.

In this context it is particularly preferred that the RNA molecule has at least two cleaveage sites for at least two different catalytic nucleic acid molecules, preferably all cleavage sites in the RNA molecule are recognized by different catalytic nucleic acid molecules.

The present disclosure relates, to a method for analyzing an RNA molecule having a cleavage site for a catalytic nucleic acid molecule, the method comprising the steps of:
a) providing an RNA molecule having a cleavage site for a catalytic nucleic acid molecule,
b) cleaving the RNA molecule with the catalytic nucleic acid molecule into a 3' terminal RNA fragment and a 5' terminal RNA fragment by contacting the RNA molecule with the catalytic nucleic acid molecule under conditions allowing the cleavage of the RNA molecule,
c) determining a physical property of the RNA molecule by analyzing the 5' terminal RNA fragment or the 3' terminal RNA fragment, preferably the 3' terminal RNA fragment.

The method disclosed herein comprises analyzing the 3' terminus, a 3' terminal modification or a 3' terminal fragment of an RNA molecule having at least one cleavage site for at least one catalytic nucleic acid molecule. Preferably, the method for analyzing an RNA molecule disclosed herein comprises determining the identity and/or the integrity of the 3' terminus of an RNA molecule having at least one cleavage site for at least one catalytic nucleic acid molecule, and/or determining the identity and/or the integrity of a 3' terminal RNA fragment obtained by cleavage of said RNA molecule with at least one catalytic nucleic acid molecule. In particularly preferred embodiments, the method disclosed herein comprises analyzing the 3'-UTR of an mRNA or a fragment of the 3'-UTR of an mRNA. More preferably, the method disclosed herein comprises determining the identity and/or the integrity of a nucleic acid sequence in the 3'-UTR of an mRNA.

The method disclosed herein preferably comprises analyzing the 5' terminus, a 5' terminal modification or a 5' terminal fragment of an RNA molecule. Preferably, the method for analyzing an RNA molecule disclosed herein comprises determining the identity and/or the integrity of the 5' terminus of an RNA molecule having at least one cleavage site for a catalytic nucleic acid molecule, and/or determining the identity and/or the integrity of a 5' terminal RNA fragment obtained by cleavage of said RNA molecule with at least one catalytic nucleic acid molecule. In particularly preferred embodiments, the method disclosed herein comprises analyzing the 5'-UTR of an mRNA or a fragment of the 5'-UTR of an mRNA. More preferably, the method disclosed herein comprises determining the identity and/or the integrity of a nucleic acid sequence in the 5'-UTR of an mRNA. In a particularly preferred embodiment, the method disclosed herein comprises determining the presence of a CAP structure or determining the orientation of a CAP structure at the 5' terminus of the RNA molecule having at least one cleavage site for a catalytic nucleic acid molecule. Such a method is already described in PCT/EP2014/003482.

The method disclosed herein does preferably not provide a method for analyzing an RNA molecule having a cleavage site for a catalytic nucleic acid molecule, the method comprising the steps of:
a) providing an RNA molecule having a cleavage site for a catalytic nucleic acid molecule,
b) cleaving the RNA molecule with the catalytic nucleic acid molecule into a 5' terminal RNA fragment and at least one 3' RNA fragment by contacting the RNA molecule with the catalytic nucleic acid molecule under conditions allowing the cleavage of the RNA molecule,
c) determining a physical property of the RNA molecule by analyzing the 5' terminal RNA fragment; and/or
a method for analyzing a population of RNA molecules, wherein the population comprises at least one RNA molecule that has a cleavage site for a catalytic nucleic acid molecule, the method comprising the steps of:
a) providing a sample containing the population of RNA molecules,
b) cleaving the at least one RNA molecule having a cleavage site for the catalytic nucleic acid molecule with the catalytic nucleic acid molecule into a 5' terminal RNA fragment and at least one 3' RNA fragment by contacting the sample with the catalytic nucleic acid molecule under conditions allowing the cleavage of the RNA molecule,
c) determining a physical property of the at least one RNA molecule having a cleavage site by analyzing the at least one 5' terminal RNA fragment obtained in step b), and
d) measuring the relative amount of the at least one 5' terminal RNA fragment obtained in step b), thereby determining the relative amount of RNA molecules having said physical properties in the RNA population.

In this context it is particularly preferred that the present disclosure does not concern a method for analyzing an RNA molecule having a cleavage site for a catalytic nucleic acid molecule or a method for analyzing a population of RNA molecules, wherein the population comprises at least one RNA molecule that has a cleavage site for a catalytic nucleic acid molecule, comprising a step determining a physical property of the at least one RNA molecule having a cleavage site by analyzing the at least one 5' terminal RNA fragment obtained by cleaving the RNA molecule with the catalytic nucleic acid molecule into a 5' terminal RNA fragment and at least one 3' RNA fragment by contacting the RNA molecule with the catalytic nucleic acid molecule under conditions allowing the cleavage of the RNA molecule.

Furthermore, it is particularly preferred that the present disclosure does not concern a method for determining the presence of a CAP structure in an RNA molecule having a cleavage site for a catalytic nucleic acid molecule, a method for determining the capping degree of a population of RNA molecules having a cleavage site for a catalytic nucleic acid molecule, a method for determining the orientation of the cap structure in a capped RNA molecule having a cleavage site for a catalytic nucleic acid molecule and a method for determining relative amounts of correctly capped RNA molecules and reverse-capped RNA molecules in a population of RNA molecules, wherein the population comprises correctly capped and/or reverse-capped RNA molecules that have a cleavage site for a catalytic nucleic acid molecule.

The method disclosed herein preferably comprises the analysis of a population of RNA molecules. Therein, the method preferably comprises determining the relative amounts of RNA molecules having distinct physical properties, such as the relative amount of RNA molecules characterized by a distinct 3' end, a distinct 3' terminal fragment or a distinct 5' end, a distinct 5' terminal fragment or a distinct central RNA fragment.

In particular, the present invention as defined by the attached claims concerns a method for determining the presence and/or integrity of a homopolymeric sequence in the 3'-UTR of a population of mRNA molecules, wherein the population comprises at least one mRNA molecule comprising a 3'-UTR, which comprises at least one homopolymeric sequence, wherein the mRNA molecules are derived from in vitro transcription reactions and wherein the mRNA molecule has at least one cleavage site for at least one catalytic nucleic acid molecule, wherein at least one cleavage site is located 5' of the homopolymeric sequence, the method comprising the steps of:
a) providing the sample containing the population of mRNA molecules, wherein the at least one mRNA molecule comprising a 3'-UTR, which comprises at least one homopolymeric sequence, has at least one cleavage site for at least one catalytic nucleic acid molecule, wherein at least one cleavage site is located 5' of the homopolymeric sequence in the 3'-UTR of the mRNA molecule,
b) cleaving the at least one mRNA molecule having at least one cleavage site for at least one catalytic nucleic acid molecule, wherein at least one cleavage site is located 5' of the homopolymeric sequence, with at least one catalytic nucleic acid molecule into a 3' terminal RNA fragment, a 5' RNA fragment and optionally into at least one central RNA fragment by contacting the sample with at least one catalytic nucleic acid molecule under conditions allowing the cleavage of the mRNA molecule,
c) determining the presence and/or the integrity of the homopolymeric sequence in the 3'-UTR of the at least one mRNA molecule having at least one cleavage site for at least one catalytic nucleic acid molecule by analyzing the 3' terminal RNA fragment, and optionally the at least one optional central RNA fragment obtained in step b), wherein step c) comprises separating the 3' terminal RNA fragment by denaturing gel electrophoresis or liquid chromatography, determining the mass and/or the length of the 3' terminal RNA fragment, comparing the determined mass and/or length of the 3' terminal RNA fragment with a respective mass and/or length of a reference RNA fragment and
d) optionally determining the relative amount of different mRNA molecules in the population.

In another aspect, the present disclosure further provides a novel use of a catalytic nucleic acid molecule for analyzing an RNA molecule or an RNA population as further defined herein. The present invention thus also provides the use of a catalytic nucleic acid molecule in a method as defined in the attached claims.

### Definitions

For the sake of clarity and readability the following definitions are provided. Any technical feature mentioned for these definitions may be read on each and every embodiment disclosed herein. Additional definitions and explanations may be specifically provided in the context of these embodiments as discussed and explained further below.

Population of RNA molecules: In the context of the present disclosure, the phrases "population of RNA molecules" or "RNA population" refers to a plurality of RNA molecules comprised in one mixture or composition. In the context of the present disclosure an RNA population comprises at least one RNA molecule having at least one cleavage site for at least one catalytic nucleic acid molecule. Preferably, the at least one RNA molecule having at least one cleavage site for at least one catalytic nucleic acid molecule is characterized by a distinct property or a structural feature, which may be determined by the method disclosed herien. In addition to the at least one RNA molecule having at least one cleavage site for at least one catalytic nucleic acid molecule, the population may optionally further comprise at least one other RNA molecule that does not have such a cleavage site for a catalytic nucleic acid molecule. A population of RNA molecules may be a plurality of identical RNA molecules having at least one cleavage site for at least one catalytic nucleic acid molecule. A population of RNA molecules preferably comprises at least two distinct RNA molecules having at least one cleavage site for at least one catalytic nucleic acid molecule. Therein, the two distinct RNA molecules are distinct from each other with regard to at least one distinct physical property or structural feature as defined herein. A "population of RNA molecules" in the context of the present disclosure, preferably comprises at least two distinct RNA molecules having at least one cleavage site for at least one catalytic nucleic acid moelcule, wherein the at least two distinct RNA molecules differ from each other only in one physical property or only in one structural feature, which is preferably located close to the 3' terminus of the RNA molecules, more preferably between the most 3' cleavage site for a catalytic nucleic acid molecule and the 3' terminus of the RNA molecules, and wherein the distinct physical property or the structural feature as defined herein may be determined by the method disclosed herein.

According to the present disclosure, said RNA molecules of the population preferably contain at least one cleavage site for at least one catalytic nucleic acid molecule, allowing the cleavage of the RNA molecules into fragments, which can then be separated and detected. In this context, said RNA molecules can be isolated RNA molecules.

The phrase "population of RNA molecules" preferably refers to a plurality of RNA molecules, wherein at least one RNA molecule has at least one cleavage site for at least one catalytic nucleic acid molecule and wherein a physical property of the at least one RNA molecule may be determined by the method disclosed herein.

5' terminal RNA fragment: The 5' terminal RNA fragment is an RNA fragment derived from the RNA molecule comprising at least one cleavage site of at least one catalytic nucleic acid molecule and comprises the 5'-terminus of the RNA molecule comprising at least one cleavage site of at least one catalytic nucleic acid molecule.

3' terminal RNA fragment: The 3' terminal RNA fragment is an RNA fragment derived from the RNA molecule comprising at least one cleavage site of at least one catalytic nucleic acid molecule and comprises the 3'-terminus of the RNA molecule comprising at least one cleavage site of at least one catalytic nucleic acid molecule.

Central RNA fragment: The central RNA fragment is an RNA fragment derived from the RNA molecule comprising at least one cleavage site of at least one catalytic nucleic acid molecule and comprises neither the 5'-terminus nor the 3' terminus of the RNA molecule comprising at least one cleavage site of at least one catalytic nucleic acid molecule.

Catalytic nucleic acid molecule: By "catalytic nucleic acid molecule" it is meant a nucleic acid molecule capable of catalyzing reactions including, but not limited to, site-specific cleavage of other nucleic acid molecules.

The term "catalytic nucleic acid molecule" preferably means a nucleic acid molecule with endonuclease activity. Such a molecule with endonuclease activity may have complementarity in a substrate binding region to a specified binding site in a nucleic acid target, and also has an enzymatic activity that specifically cleaves RNA or DNA in that target at a specific cleavage site. Therefore, the nucleic acid molecule with endonuclease activity is able to intramolecularly (*in cis*) or intermolecularly (*in trans*) cleave RNA or DNA. This complementarity functions to allow sufficient hybridization of the catalytic nucleic acid molecule to the target RNA or DNA and thereby allowing the cleavage of the target RNA or DNA at a specific cleavage site. In this context, 100% complementarity in the substrate binding region of the catalytic nucleic acid molecule to the binding site of the nucleic acid target is preferred, but complementarity of at least 50%, of at least 60%, of at least 70%, more preferably of at least 80 or 90% and most preferably of at least 95% may also be useful in this context. The catalytic nucleic acid molecule may contain modified nucleotides, which may be modified at the base, sugar, and/or phosphate groups. The term catalytic nucleic acid is used interchangeably with phrases such as enzymatic nucleic acid or nucleic acid enzyme. All of these terminologies describe nucleic acid molecules with enzymatic activity. The specific enzymatic nucleic acid molecules described in the instant application are not limiting and those skilled in the art will recognize that all that is important in an enzymatic nucleic acid molecule is that it has a specific substrate binding region which is complementary to one or more binding sites of the target nucleic acid, and that it has nucleotide sequences within or surrounding that substrate binding region, which impart a nucleic acid cleaving activity to the molecule. The term "catalytic nucleic acid molecule" includes ribozymes and DNAzymes as defined below.

Ribozyme: A ribozyme is a catalytic nucleic acid molecule, which is an RNA molecule capable of catalyzing reactions including, but not limited to, site-specific cleavage of other nucleic acid molecules such as RNA molecules. The term ribozyme is used interchangeably with phrases such as catalytic RNA, enzymatic RNA, or RNA enzyme.

In the early 80s natural RNA molecules were discovered, which are capable of catalyzing reactions in the absence of any protein component and these molecules were named ribozymes. Several classes of ribozymes occurring in natural systems have been discovered, most of which catalyse intramolecular splicing or cleavage reactions (reactions *'in cis'*)*.* Since most of the naturally occurring ribozymes catalyse self-splicing or self-cleavage reactions, it was necessary to convert them into RNA enzymes, which can cleave or modify target RNAs without becoming altered themselves (reactions *'in trans'*)*.*

Ribozymes are broadly grouped into two classes based on their size and reaction mechanisms: large and small ribozymes. The first group consists of the self-splicing group I and group II introns as well as the RNA component of RNase P, whereas the latter group includes the hammerhead, hairpin, hepatitis delta ribozymes and varkud satellite (VS) RNA as well as artificially selected nucleic acids. Large ribozymes consist of several hundreds up to 3000 nucleotides and they generate reaction products with a free 3'-hydroxyl and 5'-phosphate group. In contrast, small catalytically active nucleic acids from 30 to ~150 nucleotides in length generate products with a 2'-3'-cyclic phosphate and a 5'-hydroxyl group (Schubert and Kurreck, 2004. Curr. Drug Targets 5(8):667-681).

Group I introns include the self-splicing intron in the pre-ribosomal RNA of the ciliate *Tetrahymena thermophilia.* Further examples of group I introns interrupt genes for rRNAs, tRNAs and mRNAs in a wide range of organelles and organisms. Group I introns perform a splicing reaction by a two-step transesterification mechanism: The reaction is initiated by a nucleophilic attack of the 3'-hydroxyl group of an exogenous guanosine cofactor on the 5'-splice site. Subsequently, the free 3'-hydroxyl of the upstream exon performs a second nucleophilic attack on the 3'-splice site to ligate both exons and release the intron. Substrate specificity of group I introns is achieved by an Internal Guide Sequence (IGS). The catalytically active site for the transesterification reaction resides in the intron, which can be re-engineered to catalyse reactions *in trans.*

Group II introns are found in bacteria and in organellar genes of eukaryotic cells. They catalyse a self-splicing reaction that is mechanistically distinct from group I introns because they do not require a guanosine cofactor. Instead, the 2'-hydroxyl of a specific adenosine at the so-called branch site of the intron initiates the reaction by a nucleophilic attack on the splice-site to form a lariat-type structure.

RNase P was the first example of a catalytic RNA that acts *in trans* on multiple substrates. RNase P can be considered to be the only true naturally occurring trans-cleaving RNA enzyme known to date. However, for full enzymatic activity under *in vivo* conditions the protein component is essential.

The hammerhead ribozyme is found in several plant virus satellite RNAs, viroids and transcripts of a nuclear satellite DNA of newt. This ribozyme is the smallest of the naturally occurring ribozymes and processes the linear concatamers that are generated during the rolling circle replication of circular RNA plant pathogens. The development of hammerhead variants that cleave target RNA molecules *in trans* was a major advancement that made possible the use of ribozyme technology for practical applications. The hammerhead ribozyme motif that has widely been applied since then comprises three helical sections connected via a three-way helical junction.

In hairpin ribozymes the catalytic entity is part of a four-helix junction. A minimal catalytic motif containing approximately 50 nucleotides has been identified that can be used for metal-ion dependent cleavage reactions *in trans.* It consists of two domains, each harbouring two helical regions separated by an internal loop, connected by a hinge region. One of these domains results from the association of 14 nucleotides of a substrate RNA with the ribozyme via base-pairing.

The hepatitis delta virus (HDV) ribozyme is found in a satellite virus of hepatitis B virus. Both the genomic and the antigenomic strand express cis-cleaving ribozymes of ~85 nucleotides that differ in sequence but fold into similar secondary structures. The crystal structure of the ribozyme reveals five helical regions are organized by two pseudoknot structures. The catalytic mechanism of the hepatitis delta virus ribozyme appears to involve the action of a cytosine base within the catalytic centre as a general acid-base catalyst. The hepatitis delta ribozyme displays high resistance to denaturing agents like urea or formamide. Trans-cleaving derivatives of this ribozyme have been developed.

The Varkud Satellite (VS) ribozyme is a 154 nucleotide long and is transcribed from a plasmid discovered in the mitochondria of certain strains of Neurospora. The VS ribozyme is the largest of the known nucleolytic ribozymes.

DNAzyme: A DNAzyme is a catalytic nucleic acid molecule which is a DNA molecule capable of catalyzing reactions including, but not limited to, site-specific cleavage of other nucleic acid molecules such as RNA molecules. The term DNAzyme is used interchangeably with phrases such as catalytic DNA, enzymatic DNA, or DNA enzyme.

DNAzymes are intrinsically more stable than ribozymes made of RNA. Altough DNAzymes have not been found in nature, artificial DNAzymes such as "10-23" DNAzymes have been obtained by using *in vitro* selection methods (Schubert and Kurreck, 2004. Curr. Drug Targets 5(8):667-681).

One of the most active DNAzymes is the RNA-cleaving "10-23" DNAzyme, which was generated by an *in vitro* selection method (Santoro et al., 1997. Proc. Natl. Acad. Sci. USA 94(9):4262-6). 10-23 DNAzymes consist of a catalytic core of about 15 nucleotides and two substrate binding arms of variable length and sequence. The 10-23 DNAzyme cleaves its RNA substrate using divalent ions to yield a 2'-3'-cyclo phosphate and a free 5'-hydroxyl group.

10-23 DNAzymes can be designed and used to cleave almost any target RNA in a sequence-specific manner. Consisting of a catalytic core of 15 nucleotides and two substrate-binding arms of variable length and sequence, they bind the target RNA in a sequence-specific manner and cleave it between a paired pyrimidine base and a free purine base (Schubert et al., 2003. Nucleic Acids Res. 31 (20):5982-92). For example, the DNAzyme cleavage reaction can be performed by incubating the DNAzyme and the substrate RNA in cleavage buffer (10 mM MgCl₂, 50 mM Tris-HCl, pH7.5) at 37°C. Prior to mixing the enzyme and the substrate RNA, both solutions are denatured separately for 5 minutes at 85°C. Methods for the production of DNAzymes are known in the art. For example, DNAzymes can be chemically synthesized using standard DNA synthesis methods (Schubert et al., 2003. Nucleic Acids Res. 31(20):5982-92).

5'-Cap structure: A 5' cap is typically a modified nucleotide, particularly a guanine nucleotide, added to the 5' end of an RNA molecule. Preferably, the 5' cap is added using a 5'-5'-triphosphate linkage. A 5' cap may be methylated, e.g. m7GpppN, wherein N is the terminal 5' nucleotide of the nucleic acid carrying the 5' cap, typically the 5'-end of an RNA. The naturally occurring 5' cap is m7GpppN.

Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety.

Particularly preferred 5' cap structures are CAP1 (methylation of the ribose of the adjacent nucleotide of m7G), CAP2 (methylation of the ribose of the 2^{nd} nucleotide downstream of the m7G), CAP3 (methylation of the ribose of the 3^{rd} nucleotide downstream of the m7G), CAP4 (methylation of the ribose of the 4^{th} nucleotide downstream of the m7G),

A 5' cap structure may be formed by a Cap analog.

Cap analog: A cap analog refers to a non-extendable di-nucleotide that has cap functionality, which means that it facilitates translation or localization, and/or prevents degradation of the RNA molecule when incorporated at the 5' end of the RNA molecule. Non-extendable means that the cap analog will be incorporated only at the 5'terminus because it does not have a 5' triphosphate and therefore cannot be extended in the 3' direction by a template-dependent RNA polymerase.

Cap analogs include, but are not limited to, a chemical structure selected from the group consisting of m7GpppG, m7GpppA, m7GpppC; unmethylated cap analogs (e.g., GpppG); dimethylated cap analog (e.g., m2,7GpppG), trimethylated cap analog (e.g., m2,2,7GpppG), dimethylated symmetrical cap analogs (e.g., m7Gpppm7G), or anti reverse cap analogs (e.g., ARCA; m7,2'OmeGpppG, m7,2'dGpppG, m7,3'OmeGpppG, m7,3'dGpppG and their tetraphosphate derivatives) (Stepinski et al., 2001. RNA 7(10):1486-95).

Further cap analogs have been described previously (US7074596, WO2008/016473, WO2008/157688, WO2009/149253, WO2011/015347, and WO2013/059475). The synthesis of N⁷-(4-chlorophenoxyethyl) substituted dinucleotide cap analogs has been described recently (Kore et al., 2013. Bioorg. Med. Chem. 21 (15):4570-4).

Particularly preferred cap analogs are G[5']ppp[5']G, m⁷G[5']ppp[5']G, m₃^{2,2,7}G[5']ppp[5']G, m₂⁷'^{3'-O}G[5']ppp[5']G (3'-ARCA), m₂^{7,2'-O}GpppG (2'-ARCA), m₂^{7,2'-O}GppspG D1 (β-S-ARCA D1) and m₂^{7,2'-O}GppspG D2 (β-S-ARCA D2).

Nucleic acid: The term nucleic acid means any DNA- or RNA-molecule and is used synonymous with polynucleotide. Furthermore, modifications or derivatives of the nucleic acid as defined herein are explicitly included in the general term "nucleic acid". For example, peptide nucleic acid (PNA) is also included in the term "nucleic acid".

Monocistronic RNA: A monocistronic RNA may typically be an RNA, preferably an mRNA, that comprises only one open reading frame. An open reading frame in this context is a sequence of several nucleotide triplets (codons) that can be translated into a peptide or protein.

Bi-/multicistronic RNA: RNA, preferably mRNA, that typically may have two (bicistronic) or more (multicistronic) open reading frames (ORF). An open reading frame in this context is a sequence of several nucleotide triplets (codons) that can be translated into a peptide or protein.

Nucleotide analogs: Nucleotide analogs are nucleotides structurally similar (analog) to naturally occurring nucleotides, which include phosphate backbone modifications, sugar modifications, or modifications of the nucleobase.

Nucleic acid synthesis: Nucleic acid molecules used according to the present disclosure as defined herein may be prepared using any method known in the art, including synthetic methods such as e.g. solid phase synthesis, *in vivo* propagation (e.g. *in vivo* propagation of viruses), as well as *in vitro* methods, such as *in vitro* transcription reactions.

For preparation of a nucleic acid molecule, especially if the nucleic acid is in the form of an RNA or mRNA, a corresponding DNA molecule may e.g. be transcribed *in vitro.* This DNA template preferably comprises a suitable promoter, e.g. a T7 or SP6 promoter, for *in vitro* transcription, which is followed by the desired nucleotide sequence coding for the nucleic acid molecule, e.g. mRNA, to be prepared and a termination signal for *in vitro* transcription. The DNA molecule, which forms the template of the at least one RNA of interest, may be prepared by fermentative proliferation and subsequent isolation as part of a plasmid which can be replicated in bacteria. Plasmids, which may be mentioned as suitable in the context of the present disclosure, are e.g. the plasmids pT7Ts (GenBank accession number U26404; Lai et al., Development 1995, 121: 2349 to 2360), pGEM^{®} series, e.g. pGEM^{®}-1 (GenBank accession number X65300; from Promega) and pSP64 (GenBank accession number X65327); cf. also Mezei and Starts, Purification of PCR Products, in: Griffin and Griffin (ed.), PCR Technology: Current Innovation, CRC Press, Boca Raton, FL, 2001.

RNA: RNA is the usual abbreviation for ribonucleic acid. It is a nucleic acid molecule, i.e. a polymer consisting of nucleotides. These nucleotides are usually adenosine-monophosphate, uridine-monophosphate, guanosine-monophosphate and cytidine-monophosphate monomers, which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence.

Messenger RNA (mRNA): In eukaryotic cells, transcription is typically performed inside the nucleus or the mitochondria. *In vivo,* transcription of DNA usually results in the so-called premature RNA which has to be processed into so-called messenger RNA, usually abbreviated as mRNA. Processing of the premature RNA, e.g. in eukaryotic organisms, comprises a variety of different posttranscriptional modifications such as splicing, 5'-capping, polyadenylation, export from the nucleus or the mitochondria and the like. The sum of these processes is also called maturation of mRNA. The mature messenger RNA usually provides the nucleotide sequence that may be translated into an amino acid sequence of a particular peptide or protein. Typically, a mature mRNA comprises a 5' cap, a 5'UTR, an open reading frame, a 3'UTR and a poly(A) or a poly(C) sequence. In the context of the present disclosure, an mRNA may also be an artificial molecule, i.e. a molecule not occurring in nature. This means that the mRNA in the context of the present disclosure may, e.g., comprise a combination of a 5'UTR, open reading frame, 3'UTR and poly(A) sequence, which does not occur in this combination in nature.

Open reading frame: An open reading frame (ORF) in the context of the present disclosure may typically be a sequence of several nucleotide triplets, which may be translated into a peptide or protein. An open reading frame preferably contains a start codon, i.e. a combination of three subsequent nucleotides coding usually for the amino acid methionine (ATG or AUG), at its 5'-end and a subsequent region, which usually exhibits a length which is a multiple of 3 nucleotides. An ORF is preferably terminated by a stop codon (e.g., TAA, TAG, TGA). Typically, this is the only stop codon of the open reading frame. Thus, an open reading frame in the context of the present disclosure is preferably a nucleotide sequence, consisting of a number of nucleotides that may be divided by three, which starts with a start codon (e.g. ATG or AUG) and which preferably terminates with a stop codon (e.g., TAA, TGA, or TAG or UAA, UAG, UGA, respectively). The open reading frame may be isolated or it may be incorporated in a longer nucleic acid sequence, for example in a vector or an mRNA. An open reading frame may also be termed "protein coding region" or "coding region".

3'-untranslated region (3'-UTR): Generally, the term "3'-UTR" refers to a part of the artificial nucleic acid molecule, which is located 3' (i.e. "downstream") of an open reading frame and which is not translated into protein. Typically, a 3'-UTR is the part of an mRNA, which is located between the protein coding region (open reading frame (ORF) or coding sequence (CDS)) and the 3' terminus of the mRNA. In the context of the present disclosure, the term 3'-UTR may also comprise elements, which are not encoded in the template, from which an RNA is transcribed, but which are added after transcription during maturation, e.g. a poly(A) sequence (or poly(A) 'tail'). A 3'-UTR of the mRNA is not translated into an amino acid sequence. The 3'-UTR sequence is generally encoded by the gene, which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excise optional introns and modifications of the 3'-end, such as polyadenylation of the 3'-end of the pre-mature mRNA and optional endo-/ or exonuclease cleavages etc. In the context of the present disclosure, a 3'-UTR corresponds to the sequence of a mature mRNA, which is located between the stop codon of the protein coding region, preferably immediately 3' to the stop codon of the protein coding region, and the poly(A) sequence of the mRNA. The term "corresponds to" means that the 3'-UTR sequence may be an RNA sequence, such as in the mRNA sequence used for defining the 3'-UTR sequence, or a DNA sequence, which corresponds to such RNA sequence. In the context of the present disclosure, the term "a 3'-UTR of a gene", such as "a 3'-UTR of a ribosomal protein gene", is the sequence, which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence (both sense and antisense strand and both mature and immature) of the 3'-UTR.

5'-untranslated region (5'-UTR): A 5'-UTR is typically understood to be a particular section of messenger RNA (mRNA). It is located 5' of the open reading frame of the mRNA. Typically, the 5'-UTR starts with the transcriptional start site and ends one nucleotide before the start codon of the open reading frame. The 5'-UTR may comprise elements for controlling gene expression, also called regulatory elements. Such regulatory elements may be, for example, ribosomal binding sites. The 5'-UTR may be post-transcriptionally modified, for example by addition of a 5' cap structure. In the context of the present disclosure, the term "5'-UTR" typically refers to the sequence of an mRNA, which is located between the 5' cap structure and the start codon. Preferably, the 5'-UTR is the sequence, which extends from a nucleotide located 3' to the 5' cap structure, preferably from the nucleotide located immediately 3' to the 5' cap structure, to a nucleotide located 5' to the start codon of the protein coding region (or ORF), preferably to the nucleotide located immediately 5' to the start codon of the protein coding region.

5'-Terminal Oligopyrimidine Tract (TOP): The 5'-terminal oligopyrimidine tract (TOP) is typically a stretch of pyrimidine nucleotides located in the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5' TOP ends one nucleotide 5' to the first purine nucleotide located downstream of the TOP. Messenger RNA that contains a 5'terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present disclosure, a TOP motif is a nucleic acid sequence, which corresponds to a 5'-TOP as defined above. Thus, a TOP motif in the context of the present disclosure is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'-end with the transcriptional start site and ends one nucleotide 5' to the first purin residue in said gene or mRNA. A TOP motif in the sense of the present disclosure is preferably located at the 5'-end of a sequence, which represents a 5'-UTR, or at the 5'-end of a sequence, which codes for a 5'UTR. Thus, preferably, a stretch of 3 or more pyrimidine nucleotides is called "TOP motif" in the sense of the present disclosure if this stretch is located at the 5'-end of a respective sequence, such as the artificial nucleic acid molecule, the 5'-UTR element of the artificial nucleic acid molecule, or the nucleic acid sequence which is derived from the 5'UTR of a TOP gene as described herein. In other words, a stretch of 3 or more pyrimidine nucleotides, which is not located at the 5'-end of a 5'-UTR or a 5'-UTR element but anywhere within a 5'-UTR or a 5'-UTR element, is preferably not referred to as "TOP motif".

TOP gene: TOP genes are typically characterised by the presence of a 5' terminal oligopyrimidine tract. Furthermore, most TOP genes are characterized by a growth-associated translational regulation. However, also TOP genes with a tissue specific translational regulation are known. As defined above, the 5'-UTR of a TOP gene corresponds to the sequence of a 5'-UTR of a mature mRNA derived from a TOP gene, which preferably extends from the nucleotide located 3' to the 5'-CAP to the nucleotide located 5' to the start codon. A 5'-UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'-UTRs of TOP genes are generally rather short. The lengths of 5'-UTRs of TOP genes may vary between 20 nucleotides up to 500 nucleotides, and are typically less than about 200 nucleotides, preferably less than about 150 nucleotides, more preferably less than about 100 nucleotides. Exemplary 5'-UTRs of TOP genes in the sense of the present disclosure are the nucleic acid sequences extending from the nucleotide at position 5 to the nucleotide located immediately 5' to the start codon (e.g. the ATG) in the sequences according to SEQ ID Nos. 1-1363 of the patent application WO2013/143700. In this context, a particularly preferred fragment of a 5'UTR of a TOP gene is a 5'-UTR of a TOP gene lacking the 5'-TOP motif. The terms "5'-UTR of a TOP gene" or "5'-TOP UTR" preferably refer to the 5'-UTR of a naturally occurring TOP gene.

Self-replicating RNA (Replicons): Self-replicating RNA are delivery vectors based on alphaviruses which have been developed from Semliki Forest virus (SFV), Sindbis (SIN) virus, and Venezuelan equine encephalitis (VEE) virus. Alphaviruses are single stranded RNA viruses in which heterologous genes of interest may substitute for the alphavirus' structural genes. By providing the structural genes *in trans,* the replicon RNA is packaged into replicon particles (RP) which may be used for gene therapy purposes or genetic vaccination (see for example Vander Veen et al., 2012. Alphavirus replicon vaccines. Animal Health Research Reviews, p. 1-9). After entry into the host cell, the genomic viral RNA initially serves as an mRNA for translation of the viral nonstructural proteins (nsPs) required for initiation of viral RNA amplification. RNA replication occurs via synthesis of a full-length minusstrand intermediate that is used as the template for synthesis of additional genome-length RNAs and for transcription of a plus-strand subgenomic RNA from an internal promoter. Such RNA may then be considered as self-replicating RNA, since the non-structural proteins responsible for replication (and transcription of the heterologous genes) are still present in such replicon. Such alphavirus vectors are referred to as "replicons."

Sequence of a nucleic acid molecule: The sequence of a nucleic acid molecule is typically understood to be the particular and individual order, i.e. the succession of its nucleotides.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present disclosure, identity of sequences preferably relates to the percentage of nucleotides of a sequence which have the same position in two or more sequences having the same length. Gaps are usually regarded as non-identical positions, irrespective of their actual position in an alignment. In the context of the present disclosure the term "identity of an RNA molecule" is equivalent to the sequence identity of an RNA sequence, which is therefore comprised in the definition of the term "sequence identity".

Analysis of the (sequence) identity of the RNA molecule: In the context of the present disclosure the analysis of the (sequence) identity of an RNA molecule means the determination of a physical property of the RNA molecule (or of a fragment thereof) which can be used to assume the sequence identity (order of nucleotides) of the RNA molecule (or of a fragment thereof). The results of the determination of the physical property of the RNA molecule (or of a fragment thereof) are compared to the expected results and therefore the (sequence) identity can be concluded from this comparison. In the context of the present disclosure the RNA molecule may be cleaved into fragments by at least one catalytic nucleic acid molecule wherein the length of the resulting RNA fragments can be analysed. The resulting fragments can be compared to the expected pattern of fragments and therefore it is possible to conclude the (sequence) identity of the RNA molecule or RNA population. Thus it is not mandatory to determine the order of nucleotides in the RNA molecule or RNA population to conclude the sequence identity of the RNA molecule.

Integrity: Integrity of an RNA molecule means that the RNA molecule has the molecular weight, the mass, and/or the length as expected or compared to a reference RNA. If RNA is produced e.g. by *in vitro* transcription the length of the RNA molecule can be predicted by the length of the template used for *in vitro* transcription. Therefore an RNA molecule does not show integrity if at least one nucleotide is deleted in the RNA molecule or at least one nucleotide is added to the RNA molecule and thus does not correspond to the expected length of the RNA molecule.

Fragment of a sequence: A fragment of a sequence is typically a shorter portion of a full-length sequence of e.g. a nucleic acid sequence or an amino acid sequence. Accordingly, a fragment of a sequence, typically, consists of a sequence that is identical to the corresponding stretch or corresponding stretches within the full-length sequence. A preferred fragment of a sequence in the context of the present disclosure, consists of a continuous stretch of entities, such as nucleotides or amino acids, corresponding to a continuous stretch of entities in the molecule the fragment is derived from, which represents at least 5%, preferably at least 20%, preferably at least 30%, more preferably at least 40%, more preferably at least 50%, even more preferably at least 60%, even more preferably at least 70%, and most preferably at least 80% of the total (i.e. full-length) molecule from which the fragment is derived. It is particularly preferred that the fragment of a sequence is a functional fragment, i.e. that the fragment fulfils one or more of the functions fulfilled by the sequence the fragment is derived from.

Fragments of nucleic acids: "Fragments" of nucleic acid sequences in the context of the present disclosure may comprise a sequence of a nucleic acid as defined herein, which is, with regard to its nucleic acid molecule 5'-, 3'- and/or intrasequentially truncated compared to the nucleic acid molecule of the original (native) nucleic acid molecule. A sequence identity with respect to such a fragment as defined herein may therefore preferably refer to the entire nucleic acid as defined herein.

Transfection: The term 'transfection' refers to the introduction of nucleic acid molecules, such as DNA or RNA (e.g. mRNA) molecules, into cells, preferably into eukaryotic cells. In the context of the present disclosure, the term 'transfection' encompasses any method known to the skilled person for introducing nucleic acid molecules, preferably RNA molecules, into cells, preferably into eukaryotic cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc.

### Detailed Description

The invention as defined by the amended claims relates to a method for determining the presence and/or integrity of a homopolymeric sequence in the 3'-UTR of an mRNA molecule, the mRNA molecule having at least one cleavage site for at least one catalytic nucleic acid molecule, wherein the mRNA molecule is derived from an in vitro transcription reaction and wherein at least one cleavage site is located 5' of the homopolymeric sequence, the method comprising the steps of:
a) providing the mRNA molecule having at least one cleavage site for at least one catalytic nucleic acid molecule, wherein at least one cleavage site is located 5' of the homopolymeric sequence in the 3'-UTR of the mRNA molecule,
b) cleaving the mRNA molecule with the at least one catalytic nucleic acid molecule into a 5' terminal RNA fragment, a 3' terminal RNA fragment and optionally into at least one central RNA fragment by contacting the mRNA molecule with the at least one catalytic nucleic acid molecule under conditions allowing the cleavage of the mRNA molecule, and
c) determining the presence and/or the integrity of the homopolymeric sequence in the 3'-UTR of the mRNA molecule by analyzing the 3' terminal RNA fragment and optionally the at least one optional central RNA fragment obtained in step b), wherein step c) comprises separating the 3' terminal RNA fragment by denaturing gel electrophoresis or liquid chromatography, determining the mass and/or the length of the 3' terminal RNA fragment and comparing the determined mass and/or length of the 3' terminal RNA fragment with a respective mass and/or length of a reference RNA fragment.

In further non-claimed embodiments, the present disclosure also provides a method for analyzing an RNA molecule having at least one cleavage site for at least one catalytic nucleic acid molecule. In particular, the present disclosure relates to a method for analyzing an RNA molecule having at least one cleavage site for at least one catalytic nucleic acid molecule, the method comprising the steps of:
a) providing an RNA molecule having at least one cleavage site for at least one catalytic nucleic acid molecule,
b) cleaving the RNA molecule with the at least one catalytic nucleic acid molecule into a 5' terminal RNA fragment, a 3' terminal RNA fragment and optionally into at least one central RNA fragment by contacting the RNA molecule with the at least one catalytic nucleic acid molecule under conditions allowing the cleavage of the RNA molecule,
c) determining a physical property of the RNA molecule by analyzing the 5' terminal RNA fragment, the 3' terminal RNA fragment and/or the at least one optional central RNA fragment.

The present disclosure relates to a method for analyzing an RNA molecule having at least one cleavage site for at least one catalytic nucleic acid molecule, the method comprising the steps of:
a) providing an RNA molecule having at least one cleavage site for at least one catalytic nucleic acid molecule,
b) cleaving the RNA molecule with the at least one catalytic nucleic acid molecule into a 5' terminal RNA fragment, a 3' terminal RNA fragment and optionally into at least one central RNA fragment by contacting the RNA molecule with the at least one catalytic nucleic acid molecule under conditions allowing the cleavage of the RNA molecule,
c) determining a physical property of the RNA molecule by analyzing the 3' terminal RNA fragment and/or the at least one optional central RNA fragment.

In this context it is particularly preferred that step c) additionally comprises analyzing the 5' terminal RNA fragment.

It has been found by the inventors that the generation of RNA fragments by using a catalytic nucleic acid molecule and subsequent determination of a physical property of said RNA fragments is particularly useful in methods typically employed in quality control of RNA having at least one cleavage site for at least one catalytic nucleic acid molecule.

Advantageously, the method disclosed herein allows reliable, quick and cheap analysis of RNA molecules during or following RNA production, preferably RNA production by *in vitro* transcription.

RNA synthesis, by chemical approaches or by *in vitro* transcription, typically yields RNA molecules having the correct nucleic acid sequence (e.g. the nucleic acid sequence of a template) and by-products, which may differ only slightly from the correct RNA sequence. Many applications involving RNA, particularly for diagnostic or therapeutic purposes, however, require product homogeneity and/or the correct RNA structure and therefore the (sequence) identity and/or integrity needs to be confirmed for quality control reasons. Frequently, the above-mentioned undesired by-products differ from the correct RNA sequence in the presence of one or more additional nucleotides or in the absence of one or more nucleotides that are present in nucleic acid sequence that is used as a template. As a consequence of these changes, the physical properties (e.g. mass, length and/or charge etc.) of the product RNA are changed. However, these changes can typically not be determined reliably by direct analysis of the product RNA as a whole. The method disclosed herein provides a sufficient resolution in order to determine these differences and to distinguish correct product RNA from an erroneous by-product.

Particularly in case the RNA comprises homopolymer sequences such as poly(A) and/or poly(C) sequences or tandem repeats, deletion or partial deletion of these sequences is a problem. Such mutations in homopolymer sequences or tandem repeats can often not be determined directly e.g. by sequencing. The method disclosed herein provides a direct and reliable method to detect such erroneous products.

The present disclosure provides a method for analyzing an RNA molecule as described herein, wherein the method is used as a quality control in the production of the RNA molecule. More preferably, the method disclosed herein is used as a quality control step in a large scale production process of the RNA molecule. Even more preferably, the method disclosed herein is used as a quality control step in a GMP-compliant production process of the RNA molecule as described herein.

In general, the method disclosed herein is not limited with respect to the type of RNA molecule to be analyzed. Preferably, the RNA molecule having at least one cleavage site for at least one catalytic nucleic acid molecule is an RNA molecule as defined herein. For example, the RNA molecule to be analyzed may be a single-stranded or a double-stranded RNA, preferably, whithout being limited thereto, an RNA oligonucleotide (oligoribonucleotide), preferably a short oligonucleotide, a coding RNA, a messenger RNA (mRNA), an immunostimulatory RNA, a ribosomal RNA (rRNA), a transfer RNA (tRNA), a viral RNA (vRNA), a self-replicating RNA (replicon), a small interfering RNA (siRNA), a microRNA, a small nuclear RNA (snRNA), a small-hairpin (sh) RNA or riboswitch, a ribozyme, or an aptamer. Preferably the RNA molecule is a primary microRNA (pri-miRNA) molecule. It is known that miRNAs are first transcribed as a largely unstructured precursor, termed a primary miRNA (pri-miRNA), which is sequentially processed in the nucleus, to give the approximately 65-nt pre-miRNA hairpin intermediate, and then in the cytoplasm, to give the mature miRNA. These pre-miRNA molecules can be capped and polyadenylated (Cai et al., 2004. RNA 10(12):1957-66). Aside from messenger RNA, several non-coding types of RNA exist which may be involved in regulation of transcription and/or translation, and immunostimulation. The term "RNA" further encompass other coding RNA molecules, such as viral RNA, retroviral RNA and replicon RNA, small interfering RNA (siRNA), antisense RNA, CRISPR RNA, ribozymes, aptamers, riboswitches, immunostimulating RNA, transfer RNA (tRNA), ribosomal RNA (rRNA), small nuclear RNA (snRNA), small nucleolar RNA (snoRNA), microRNA (miRNA), and Piwi-interacting RNA (piRNA).

The RNA molecule having at least one cleavage site for at least one catalytic nucleic acid molecule is preferably not a mammalian U6 small nuclear RNA (U6 snRNA). More preferably, the RNA molecule having at least one cleavage site for at least one catalytic nucleic acid molecule is not an eukaryotic U6 snRNA, most preferably not an U6 snRNA. The RNA molecule having at least one cleavage site for at least one catalytic nucleic acid molecule is preferably not an snRNA.

The RNA molecule having at least one cleavage site for at least one catalytic nucleic acid molecule does preferably not comprise or consist of a nucleic acid sequence according to SEQ ID NO: 19, or a fragment or variant thereof. Preferably, the RNA molecule having at least one cleavage site for at least one catalytic nucleic acid molecule does not comprise or consist of a nucleic acid sequence identical to or at least 80% identical to a nucleic acid sequence according to SEQ ID NO: 19.
SEQ ID NO: 19:
GCGCCGAAACACCGUGUCUCGAGC

The RNA molecule having at least one cleavage site for at least one catalytic nucleic acid molecule is derived from an in vitro transcription reaction.

The RNA molecule having at least one cleavage site for at least one catalytic nucleic acid molecule is preferably a single-stranded RNA.

The RNA molecule having at least one cleavage site for at least one catalytic nucleic acid molecule may preferably not comprise a γ-monomethyl phosphate CAP. More preferably, the RNA molecule to be analyzed may not comprise a 5'-cap or a 5'-cap analogue as described herein.

Further preferably, the RNA molecule having at least one cleavage site for at least one catalytic nucleic acid molecule comprises at least one open reading frame (ORF) encoding at least one peptide or protein. More preferably, the RNA molecule is a (linear) single-stranded RNA, even more preferably an mRNA or an immunostimulatory RNA. In the context of the present disclosure, an mRNA is typically an RNA, which is composed of several structural elements, e.g. an optional 5' terminal cap structure, an optional 5'-UTR region, an upstream positioned ribosomal binding site followed by a coding region (open reading frame, ORF), an optional 3'-UTR region, which may be followed by a poly-A tail, a poly-C-tail, and/or a histone stem-loop sequence. An mRNA may occur as a mono-, di-, or even multicistronic RNA, i.e. an RNA, which carries the coding sequences of one, two or more proteins or peptides. Such coding sequences in di-, or even multicistronic mRNA may be separated by at least one IRES sequence, e.g. as defined herein.

More preferably, the RNA molecule having at least one cleavage site for at least one catalytic nucleic acid molecule is an mRNA. It may further be preferred that the RNA molecule having at least one cleavage site for at least one catalytic nucleic acid molecule is not selected from the group consisting of an mRNA encoding a Huntington's Disease (HD) protein, an mRNA encoding human growth hormone (hGH) or an mRNA encoding Alzheimer amyloid precursor (βAPP), and an mRNA encoding a fragment or variant of any of these proteins.

The method is preferably for analyzing an RNA molecule having at least one cleavage site for at least one catalytic nucleic acid molecule, wherein the RNA molecule comprises at least one modification. In the context of the present disclosure, an RNA molecule having at least one modification is also referred to as "modified RNA molecule". Therein, the modification is not limited to any particular structure. Preferably, the structural modification is a structural feature that is typically not found in the respective naturally occurring RNA, but is preferably introduced in an artificial RNA molecule, preferably in an artificial mRNA molecule. Several RNA modifications are known in the art, which can be applied to a given RNA in the context of the present disclosure. In the following, some exemplary modifications are described.

### Chemical modifications:

The term "RNA modification" as used herein may refer to chemical modifications comprising backbone modifications as well as sugar modifications or base modifications.

In this context, the modified RNA molecule as defined herein may contain nucleotide analogues/modifications, e.g. backbone modifications, sugar modifications or base modifications. A backbone modification in connection with the present disclosure is a modification, in which phosphates of the backbone of the nucleotides contained in an RNA molecule as defined herein are chemically modified. A sugar modification in connection with the present disclosure is a chemical modification of the sugar of the nucleotides of the RNA molecule as defined herein. Furthermore, a base modification in connection with the present disclosure is a chemical modification of the base moiety of the nucleotides of the RNA molecule. In this context, nucleotide analogues or modifications are preferably selected from nucleotide analogues which are applicable for transcription and/or translation.

### Sugar Modifications:

The modified nucleosides and nucleotides, which may be incorporated into the modified RNA as described herein, can be modified in the sugar moiety. For example, the 2' hydroxyl group (OH) can be modified or replaced with a number of different "oxy" or "deoxy" substituents. Examples of "oxy" -2' hydroxyl group modifications include, but are not limited to, alkoxy or aryloxy (-OR, e.g., R = H, alkyl, cycloalkyl, aryl, aralkyl, heteroaryl or sugar); polyethyleneglycols (PEG), -O(CH₂CH₂O)nCH₂CH₂OR; "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH₂; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified RNA can include nucleotides containing, for instance, arabinose as the sugar.

### Backbone Modifications:

The phosphate backbone may further be modified in the modified nucleosides and nucleotides, which may be incorporated into the modified RNA, as described herein. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

### Base Modifications:

The modified nucleosides and nucleotides, which may be incorporated into the modified RNA, as described herein, can further be modified in the nucleobase moiety. Examples of nucleobases found in RNA include, but are not limited to, adenine, guanine, cytosine and uracil. For example, the nucleosides and nucleotides described herein can be chemically modified on the major groove face. The major groove chemical modifications can include an amino group, a thiol group, an alkyl group, or a halo group.

The nucleotide analogues/modifications are preferably selected from base modifications, which are preferably selected from 2-amino-6-chloropurineriboside-5'-triphosphate, 2-aminopurine-riboside-5'-triphosphate; 2-aminoadenosine-5'-triphosphate, 2'-amino-2'-deoxycytidine-triphosphate, 2-thiocytidine-5'-triphosphate, 2-thiouridine-5'-triphosphate, 2'-fluorothymidine-5'-triphosphate, 2'-O-methyl inosine-5'-triphosphate, 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-bromo-2'-deoxycytidine-5'-triphosphate, 5-bromo-2'-deoxyuridine-5'-triphosphate, 5-iodocytidine-5'-triphosphate, 5-lodo-2'-deoxycytidine-5'-triphosphate, 5-iodouridine-5'-triphosphate, 5-iodo-2'-deoxyuridine-5'-triphosphate, 5-methylcytidine-5'-triphosphate, 5-methyluridine-5'-triphosphate, 5-propynyl-2'-deoxycytidine-5'-triphosphate, 5-propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

Modified nucleosides preferably include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio- 1-methyl-pseudouridine, 2-thio-I-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

Modified nucleosides preferably include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl- 1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine.

Modified nucleosides preferably include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

Modified nucleosides preferably include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

The nucleotide can be modified on the major groove face and can include replacing hydrogen on C-5 of uracil with a methyl group or a halo group.

In specific embodiments, a modified nucleoside is 5'-O-(1-thiophosphate)-adenosine, 5'-O-(1-thiophosphate)-cytidine, 5'-O-(1-thiophosphate)-guanosine, 5'-O-(1-thiophosphate)-uridine or 5'-O-(1-thiophosphate)-pseudouridine.

The modified RNA may comprise nucleoside modifications selected from 6-aza-cytidine, 2-thio-cytidine, α-thio-cytidine, pseudo-iso-cytidine, 5-aminoallyl-uridine, 5-iodo-uridine, N1-methyl-pseudouridine, 5,6-dihydrouridine, α-thio-uridine, 4-thio-uridine, 6-aza-uridine, 5-hydroxy-uridine, deoxy-thymidine, 5-methyl-uridine, pyrrolo-cytidine, inosine, α-thio-guanosine, 6-methyl-guanosine, 5-methyl-cytdine, 8-oxo-guanosine, 7-deaza-guanosine, N1-methyl-adenosine, 2-amino-6-chloro-purine, N6-methyl-2-amino-purine, pseudo-iso-cytidine, 6-chloro-purine, N6-methyl-adenosine, α-thio-adenosine, 8-azido-adenosine, 7-deaza-adenosine.

### Lipid modification:

The modified RNA as defined herein can contain a lipid modification. Such a lipid-modified RNA typically comprises an RNA as defined herein. Such a lipid-modified RNA molecule as defined herein typically further comprises at least one linker covalently linked with that RNA molecule, and at least one lipid covalently linked with the respective linker. Alternatively, the lipid-modified RNA molecule comprises at least one RNAmolecule as defined herein and at least one (bifunctional) lipid covalently linked (without a linker) with that RNA molecule. According to a third alternative, the lipid-modified RNA molecule comprises an RNA molecule as defined herein, at least one linker covalently linked with that RNA molecule, and at least one lipid covalently linked with the respective linker, and also at least one (bifunctional) lipid covalently linked (without a linker) with that RNA molecule. In this context, it is particularly preferred that the lipid modification is present at the terminal ends of a linear RNA sequence.

### Modification of the 5'-end of the modified RNA:

The modified RNA molecule as defined herein, can be modified by the addition of a so-called "5' CAP" structure.

A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. m7Gppp(N) (wherein "N" is the first transcribed nucleotide) is the 5'-cap structure, which naturally occurs in mRNA transcribed by polymerase II and is therefore not considered as modification comprised in the modified RNA disclosed herein. This means the modified RNA disclosed herein may comprise a m7Gppp(N) as 5'-cap, but additionally the modified RNA comprises at least one further modification as defined herein.

Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. These modified 5'-cap structures are regarded as at least one modification comprised in the modified RNA disclosed herein.

Particularly preferred modified 5'-cap structures are CAP1 (methylation of the ribose of the adjacent nucleotide of m7G), CAP2 (methylation of the ribose of the 2^{nd} nucleotide downstream of the m7G), CAP3 (methylation of the ribose of the 3^{rd} nucleotide downstream of the m7G), CAP4 (methylation of the ribose of the 4^{th} nucleotide downstream of the m7G), ARCA (anti-reverse CAP analogue, modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

The RNA molecule having at least one cleavage site for at least one catalytic nucleic acid molecule preferably comprises a 5'-cap structure, wherein the 5'-cap structure is preferably not a γ-monomethyl phosphate cap.

### Sequence modification of the open reading frame:

### Modification of the G/C content:

The G/C content of the coding region, encoding at least one peptide or protein of the modified RNA as defined herein, is preferably modified, particularly increased, compared to the G/C content of its particular wild type coding region, i.e. the unmodified coding region. The encoded amino acid sequence of the coding region is preferably not modified compared to the coded amino acid sequence of the particular wild type coding region.

The modification of the G/C-content of the coding region of the modified RNA as defined herein is based on the fact that the sequence of any mRNA region to be translated is important for efficient translation of that mRNA. Thus, the composition and the sequence of various nucleotides are important. In particular, mRNA sequences having an increased G (guanosine)/C (cytosine) content are more stable than mRNA sequences having an increased A (adenosine)/U (uracil) content. According to the present disclosure, the codons of the coding region are therefore varied compared to its wild type coding region, while retaining the translated amino acid sequence, such that they include an increased amount of G/C nucleotides. In respect to the fact that several codons code for one and the same amino acid (so-called degeneration of the genetic code), the most favourable codons for the stability can be determined (so-called alternative codon usage).

Depending on the amino acid to be encoded by the coding region of the modified RNA as defined herein, there are various possibilities for modification of the RNA sequence, e.g. the coding region, compared to its wild type coding region. In the case of amino acids, which are encoded by codons, which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U is present.

In contrast, codons which contain A and/or U nucleotides can be modified by substitution of other codons which code for the same amino acids but contain no A and/or U. Examples of these are:
the codons for Pro can be modified from CCU or CCA to CCC or CCG;
the codons for Arg can be modified from CGU or CGA or AGA or AGG to CGC or CGG;
the codons for Ala can be modified from GCU or GCA to GCC or GCG;
the codons for Gly can be modified from GGU or GGA to GGC or GGG.

In other cases, although A or U nucleotides cannot be eliminated from the codons, it is however possible to decrease the A and U content by using codons, which contain a lower content of A and/or U nucleotides. Examples of these are:
the codons for Phe can be modified from UUU to UUC;
the codons for Leu can be modified from UUA, UUG, CUU or CUA to CUC or CUG;
the codons for Ser can be modified from UCU or UCA or AGU to UCC, UCG or AGC;
the codon for Tyr can be modified from UAU to UAC;
the codon for Cys can be modified from UGU to UGC;
the codon for His can be modified from CAU to CAC;
the codon for Gin can be modified from CAA to CAG;
the codons for lie can be modified from AUU or AUA to AUC;
the codons for Thr can be modified from ACU or ACA to ACC or ACG;
the codon for Asn can be modified from AAU to AAC;
the codon for Lys can be modified from AAA to AAG;
the codons for Val can be modified from GUU or GUA to GUC or GUG;
the codon for Asp can be modified from GAU to GAC;
the codon for Glu can be modified from GAA to GAG;
the stop codon UAA can be modified to UAG or UGA.

In the case of the codons for Met (AUG) and Trp (UGG), on the other hand, there is no possibility of sequence modification.

The substitutions listed above can be used either individually or in any possible combination to increase the G/C content of the coding region of the modified RNA as defined herein, compared to its particular wild type coding region (i.e. the original sequence). Thus, for example, all codons for Thr occurring in the wild type sequence can be modified to ACC (or ACG).

Preferably, the G/C content of the coding region of the modified RNA as defined herein is increased by at least 7%, more preferably by at least 15%, particularly preferably by at least 20%, compared to the G/C content of the wild type coding region. Preferably, at least 5%, 10%, 20%, 30%, 40%, 50%, 60%, more preferably at least 70 %, even more preferably at least 80% and most preferably at least 90%, 95% or even 100% of the substitutable codons in the coding region encoding at least one peptide or protein, which comprises a pathogenic antigen or a fragment, variant or derivative thereof, are substituted, thereby increasing the G/C content of said coding region.

In this context, it is particularly preferable to increase the G/C content of the coding region of the modified RNA as defined herein, to the maximum (i.e. 100% of the substitutable codons), compared to the wild type coding region.

### Codon optimization:

According to the present disclosure, a further preferred modification of the coding region encoding at least one peptide or protein of the modified RNA as defined herein, is based on the finding that the translation efficiency is also determined by a different frequency in the occurrence of tRNAs in cells. Thus, if so-called "rare codons" are present in the coding region of the wild type RNA sequence, to an increased extent, the mRNA is translated to a significantly poorer degree than in the case where codons coding for relatively "frequent" tRNAs are present.

In this context, the coding region of the modified RNA is preferably modified compared to the corresponding wild type coding region such that at least one codon of the wild type sequence, which codes for a tRNA which is relatively rare in the cell, is exchanged for a codon, which codes for a tRNA which is relatively frequent in the cell and carries the same amino acid as the relatively rare tRNA. By this modification, the coding region of the modified RNA as defined herein, is modified such that codons, for which frequently occurring tRNAs are available, are inserted. In other words, according to the present disclosure, by this modification all codons of the wild type coding region, which code for a tRNA which is relatively rare in the cell, can in each case be exchanged for a codon, which codes for a tRNA which is relatively frequent in the cell and which, in each case, carries the same amino acid as the relatively rare tRNA.

Which tRNAs occur relatively frequently in the cell and which, in contrast, occur relatively rarely is known to a person skilled in the art; cf. e.g. Akashi, Curr. Opin. Genet. Dev. 2001, 11 (6): 660-666. The codons which use for the particular amino acid the tRNA which occurs the most frequently, e.g. the Gly codon, which uses the tRNA which occurs the most frequently in the (human) cell, are particularly preferred.

According to the present disclosure, it is particularly preferable to link the sequential G/C content, which is increased, in particular maximized, in the coding region of the modified RNA as defined herein, with the "frequent" codons without modifying the amino acid sequence of the peptide or protein encoded by the coding region of the RNA sequence. This allows provision of a particularly efficiently translated and stabilized (modified) RNA sequence as defined herein.

In a non-claimed embodiment, the RNA molecule having at least one cleavage site for at least one catalytic nucleic acid molecule is produced by non-enzymatic chemical RNA synthesis (e.g. Marshall and Kaiser, 2004. Curr. Opin. Chem. Biol. 8(3):222-229). That method is preferably employed in the case of an RNA molecule having a length of about 100 nucleotides or less. According to the invention as defined in the attached claims, the RNA molecule having at least one cleavage site for at least one catalytic nucleic acid molecule is synthesized by an *in vitro* transcription reaction.

Alternatively, the RNA molecule, preferably a single-stranded RNA molecule, more preferably an mRNA, provided in step a) of the method disclosed herein is preferably not associated with another nucleic acid molecule, such as another RNA or a DNA.

The RNA molecule having at least one cleavage site for at least one catalytic nucleic acid molecule is preferably a long RNA molecule comprising at least 100, 150, 200 or more preferably at least 500 nucleotides in length. Preferably, the RNA molecule has a length of from 5 to 30000 nucleotides, 10 to 25000 nucleotides, 50 to 20000 nucleotides, 100 to 18000 nucleotides, 300 to 15000 nucleotides or 500 to 10000 nucleotides.

The RNA molecule, which is analyzed by the method disclosed herein, comprises at least one cleavage site for at least one catalytic nucleic acid molecule. Typically, the RNA molecule is cleaved at the cleavage site by the catalytic nucleic acid molecule, which yields a 3' terminal RNA fragment and a 5' terminal RNA fragment. In case the RNA molecule comprises more than one cleavage sites for at least one catalytic nucleic acid molecule, the RNA molecule is cleaved into a 3' terminal RNA fragment, a 5' terminal RNA fragment and at least one central RNA fragment. In general, the RNA molecule to be analyzed may comprise a cleavage site for any catalytic nucleic acid molecule, wherein the method is not limited with respect to a certain catalytic nucleic acid molecule. Typically, the cleavage site is specifically recognized by the respective catalytic nucleic acid molecule, preferably as defined herein, which is employed in the method disclosed herein. As used herein, the cleavage site for the catalytic nucleic acid molecule is comprised at least once in the RNA molecule.

The RNA molecule has at least one cleavage site, wherein the cleavage site is preferably recognized by the catalytic nucleic acid molecule as described herein in a sequence-specific manner.

Preferably, the sequence of the RNA molecule has been designed or artificially modified in order to comprise at least one cleavage site for at least one catalytic nucleic acid molecule. Methods for changing or introducing nucleotides into DNA molecules to produce specific sites are known in the art. That DNA template can then be used to produce an RNA molecule, e.g. by *in vitro* transcription. These methods are known in the art. Preferably, the RNA molecule to be analyzed comprises a sequence, which is at least 30 %, 40 %, 50 %, 60 %, 70 %, 80 %, 90 % or 95 % identical to the consensus sequence of a cleavage site for a particular catalytic nucleic acid molecule.

For example, hairpin ribozymes cleave 5' of the guanosine in NGUC sequences, wherein N is any nucleotide. Furthermore, for example, a hammerhead ribozyme can be directed to cleave 3' of any NUH sequence, wherein N is any nucleotide, U is conserved, and H can be any nucleotide except G (N=G,A,C,U; H=A,C,U) (Haseloff and Gerlach, 1988. Nature 334: 585-591; McCall et al., 2000. Molecular Biotechnology 14: 5-17).

The RNA molecule to be analyzed comprises at least one cleavage site for at least one catalytic nucleic acid molecule. The RNA molecule may comprise any number of cleavage sites for the at least one catalytic nucleic acid molecule, wherein the location of the at least one cleavage site is preferably selected in order to allow separation and detection of the resulting RNA fragments.

Preferably, the location of the at least one cleavage site is chosen such that cleavage of the RNA molecule at that site generates an RNA fragment that has a suitable size (i.e. number of nucleotides) in order to be separated by methods known in the art.

In case the 3' terminal RNA fragment is analysed it is preferred that the most 3' cleavage site is located in a position between nucleotide positions 1 to 500 in 3' to 5' direction of the RNA molecule (i.e. in a position up to 500 nucleotides 5' of the 3' terminus of the RNA molecule), so that the resulting 3' RNA fragment has a size equal to or smaller than 500 nucleotides. More preferably, the most 3'cleavage site is located between nucleotide positions 1 and 400, 1 and 300, 1 and 200, 1 and 150, 1 and 100 or 1 and 50 in 3' to 5' direction of the RNA molecule, wherein "position 1" corresponds to the 3' terminal nucleotide of the RNA molecule, "position 2" corresponds to the second nucleotide starting from the 3' terminus, and so forth. Most preferably, the cleavage site is located between nucleotide positions 1 and 200, 20 and 150, or 1 and 100 in 3' to 5' direction of the RNA molecule. Furthermore, it is preferred that the RNA molecule is cleaved by the catalytic nucleic acid molecule (in 3' to 5' direction) after nucleotide position 1, 2, 3, 4, 5, 6, 7, 8, 9, 10, 11, 12, 13, 14, 15, 16, 17, 18, 19, 20, 21, 22, 23, 24 or 25. It is further particularly preferred that cleavage occurs between nucleotide position 5 and 15 or between position 8 and 20.

In case the 5' terminal RNA fragment is analysed it is preferred that the most 5' cleavage site is located in a position between nucleotide positions 1 to 500 in 5' to 3' direction of the RNA molecule (i.e. in a position up to 500 nucleotides 3' of the 5' terminus of the RNA molecule), so that the resulting 5' RNA fragment has a size equal to or smaller than 500 nucleotides. More preferably, the most 5' cleavage site is located between nucleotide positions 1 and 400, 1 and 300, 1 and 200, 1 and 150, 1 and 100 or 1 and 50 in 5' to 3' direction of the RNA molecule, wherein "position 1" corresponds to the 5' terminal nucleotide of the RNA molecule, "position 2" corresponds to the second nucleotide starting from the 5' terminus, and so forth. Most preferably, the cleavage site is located between nucleotide positions 20 and 200, 20 and 150, or 1 and 100 in 5' to 3' direction of the RNA molecule.

In case a central RNA fragment is analysed, the RNA molecule having at least one cleavage site for at least one catalytic nucleic acid molecule has to be cleaved at at least two cleaving sites by at least one catalytic nucleic acid molecule. In this context it is preferred that the cleavage sites are located in positions wherein at least 1, 2, 3, 4, 5, 10, 15, 20, 30, 40, 50, 60, 70, 80, 90, 100, 150, 200, 300, 400 or 500 nucleotides are between both cleavage sites.

It is further preferred that the RNA molecule comprises an open reading frame encoding at least one protein or peptide, wherein preferably the most 3' cleavage site for a catalytic nucleic acid molecule is located between the 3' end of the open reading frame and the 3' terminus of the RNA molecule. More preferably, the RNA molecule having a cleavage site is an mRNA molecule and comprises a 3'-UTR as defined herein. Preferably, the most 3' cleavage site is positioned in the 3'-UTR of said mRNA molecule.

Generally, the length of the RNA fragments resulting from the cleavage of the RNA molecule with at least one catalytic nucleic acid molecule is not limited in any way. In particular, according to the present disclosure, the RNA fragment to be analyzed may have any length that allows analysis of the RNA fragment (e.g.separation and resolution of the RNA fragment, preferably separation from another' RNA fragment). Depending, amongst other factors, on the physical property to be determined and depending on the means of separation that are envisaged, the skilled person may adapt the length of the RNA fragment to be analyzed by choosing the respective position of the cleavage site in the RNA molecule to be analyzed. Preferably, the at least onecleavage site in the RNA molecule is chosen such that cleavage with a catalytic nucleic acid molecule results in an RNA fragment (a 5' terminal RNA fragment, a 3' terminal fragement and optionally at least one central RNA fragment), which comprises at least 1, 2, 3, 4, 5, 6, 7, 8, 9, 10, 11, 12, 13, 14, 15, 16, 17, 18, 19 or 20 nucleotides. Alternatively, the length of the RNA fragment to be analysed is from 1 to 500, from 1 to 400, from 1 to 300, from 1 to 200, from 10 to 200, from 10 to 150 or from 20 to 150 nucleotides.

If the 3' terminal fragment is to be analyzed, the location of the most 3' cleavage site in the RNA molecule is preferably chosen such that the length of the 3' terminal RNA fragment resulting from the cleavage is from 5 to 300, from 10 to 250, from 20 to 200 or from 20 to 150 nucleotides. The length of the 3' terminal fragment is preferably 250 nucleotides or less.

The 3' terminal fragment preferably has a length of at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 nucleotides. More preferably, the 3' terminal fragment has a length of at least 30, at least 40, at least 50, at least 60, at least 70, at least 80, at least 90, at least 100 or at least 110 nucleotides.

The skilled person knows that one option to distinguish the RNA fragments of interest from other nucleic acid molecules or fragments may be the choice of an appropriate size of the RNA fragments to be analyzed by choosing an appropriate cleavage site. Alternatively, the RNA fragments to be analysed are labelled with an appropriate marker so that the RNA fragments may be detected and distinguished from non-labelled RNA fragments.

As used herein, the term "labelled" refers to an RNA molecule that is either directly or indirectly labelled with a molecule, which provides a detectable signal, e.g. radioisotope, fluorescent tag, chemiluminescent tag, a peptide or specific binding molecules. Specific binding molecules include pairs, such as biotin and streptavidin, digoxin and antidigoxin. The label can directly or indirectly provide a detectable signal. Radioisotopes (e.g. ¹⁸F, ¹²⁵I, ³⁵S, ¹⁴C, ³H, or ^{99m}Tc) are commonly used in biological applications for the detection of a variety of nucleic acids such as RNA. Methods for the synthesis and labelling of RNA *in vitro* are known in the art (e.g. Huang and Yu, 2013. Synthesis and Labelling of RNA In Vitro. Current Protocols in Molecular Biology. 102:4.15.1-4.15.14).

The method disclosed herein preferably uses a catalytic nucleic acid molecule that has been designed to be able to cleave the RNA molecule at at least one specific cleavage site, preferably at the most 3' cleavage site as described herein. Methods for designing catalytic nucleic acid molecules, in particular ribozymes that cleave RNA substrate molecules at a defined site, are known in the art.

For example, hairpin ribozymes cleave 5' of the guanosine in NGUC sequences, wherein N is any nucleotide. Furthermore, for example, a hammerhead ribozyme can be directed to cleave 3' of any NUH sequence, wherein N is any nucleotide, U is conserved, and H can be any nucleotide except G (N=G,A,C,U; H=A,C,U) (Haseloff and Gerlach, 1988. Nature 334: 585-591; McCall et al., 2000. Molecular Biotechnology 14: 5-17).

According to the substrate requirements of a catalytic nucleic acid molecule described above, an RNA molecule can - in principle - be expected to contain a number of possible sites for sequence-specific cleavage by a catalytic nucleic acid molecule. In addition to the target site, the number of base pairs to be formed between the catalytic nucleic acid molecule and the substrate are preferably chosen (substrate binding region). The affinity of a catalytic nucleic acid molecule towards its substrate can be adjusted by altering the length of the substrate binding region of the catalytic nucleic acid molecule. Although high affinity is usually desirable, an extended substrate binding region may cause problems regarding specificity and catalytic activity. Multiple turnover catalysis may be severely impaired if product release is slow due to strong binding of the target nucleic acid molecule to the catalytic nucleic acid molecule. Catalytic nucleic acid molecules with short binding arms (substrate binding region), however, may lack specificity.

Therefore, catalytic activity on the one hand and specificity on the other hand are preferably balanced when designing a catalytic nucleic acid molecule. Catalytic nucleic acid molecules, which form a larger number of base pairs with the substrate RNA, are less likely to dissociate from the cleaved substrate, and are thus not available for further cleavage. Therefore, the number of base pairs is preferably selected in such a way that the catalytic nucleic acid molecule-substrate complex formed is relatively stable under the conditions allowing the cleavage of the RNA molecule, but is able to dissociate once cleavage of the substrate has occured. This typically requires 11 to 17 base pairs. Depending on the actual requirements in the specific case, that number may vary considerably. As a general rule, for specificity, the number of base pairs formed between the catalytic nucleic acid molecule and the substrate RNA should be high enough to make the target sequence unique, but not so high that imperfectly matched substrates would form stable complexes. Statistically, about 13 nucleotides are required to uniquely define a particular site in an RNA pool.

Methods for the production of catalytic nucleic acid molecules are known in the art. For example, a ribozyme can be chemically synthesized using the standard procedure for RNA synthesis as described (Wincott et al., 1995. Nucleic Acids Res. 23(14):2677-84). Ribozymes can also be synthesized by *in vitro* transcription of suitable DNA templates using e.g. bacteriophage T7 RNA polymerase (Haseloff and Gerlach, 1988. Nature 334: 585-591).

In this context, it is particularly preferred that the catalytic nucleic acid molecule is provided in *trans.* This means that the RNA molecule having at least one cleavage site for at least one catalytic nucleic acid molecule and the at least one catalytic nucleic acid molecule are not part of the same molecule. However, the present disclosure also comprises the use of the catalytic nucleic acid molecule in *cis,* i.e. a situation, where the RNA molecule having at least one cleavage site and the at least one catalytic nucleic acid molecule are part of the same molecule.

As disclosed herein, the catalytic nucleic acid molecule is preferably a ribozyme. In this context it is particularly preferred that the ribozyme is selected from the group consisting of hammerhead ribozymes, hairpin ribozymes, and HDV ribozymes. The ribozyme is preferably a hammerhead ribozyme.

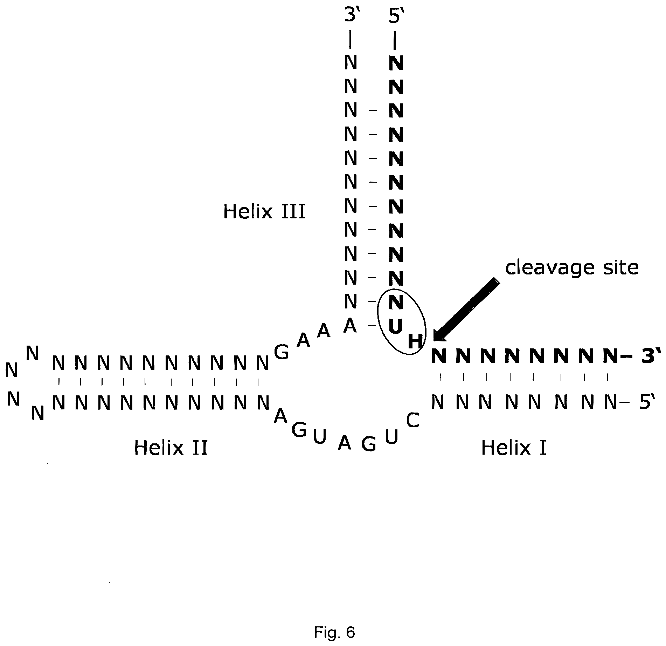

Particularly preferred in this context is a hammerhead ribozyme, which specifically cleaves an RNA molecule 3' of the sequence motif NUH as shown in Figure 6, wherein N is G, A, C, or U, and H is A, C, or U (Haseloff and Gerlach, 1988. Nature 334: 585-591; McCall et al., 2000. Molecular Biotechnology, 14: 5-17).

The ribozyme is preferably selected from the group consisting of 3HH1871_5A (SEQ ID NO: 5), 3HH2989_5A (SEQ ID NO: 6), 3HH_3A_5C_01 (SEQ ID NO: 7), 3HH_3A_5C_02 (SEQ ID NO: 8), 3HH_3C_01 (SEQ ID NO: 9) and 3HH_3C_02 (SEQ ID NO: 10), the nucleic acid sequences of which are listed below.
3HH1871_5A (SEQ ID NO: 5): 
5'-uuuuuuuuuuuuuuuuuuucugaugaggccucgaccgauaggucgaggccgaaauuaaucucggugcaag
gaggggagga-3'
3HH2989_5A (SEQ ID NO: 6): 
5'-uuuuuuuuuuuuuuuuuuucugaugaggccucgaccgauaggucgaggccgaaagaucuagguucuuucc
auuuuuuauu-3'
3HH_3A_5C_01 (SEQ ID NO: 7): 
5'-gggggggggggggggcugaugaggccucgaccgauaggucgaggccgaaaugcauuuuuuuuuuuuuuuu
uuuuuu-3'
3HH_3A_5C_02 (SEQ ID NO: 8): 
5'-ggggggcugaugaggccucgaccgauaggucgaggccgaaaugcaUUUUUUUUUUUUUUUU
UUUU-3'
3HH_3C_01 (SEQ ID NO: 9): 
5'-aauucugguggcucugaaaacugaugaggccucgaccgauaggucgaggccgaaagccuuggggggggg
gggggggg-3'
3HH_3C_02 (SEQ ID NO: 10).
   5'-aauucugguggcucugaaaacugaugaggccucgaccgauaggucgaggccgaaagccuuuggggggg-3'

Alternatively, the catalytic nucleic acid molecule is preferably a ribozyme, wherein the ribozyme is preferably not a hammerhead ribozyme.

The catalytic nucleic acid molecule does preferably not comprise or consist of a nucleic acid sequence according to SEQ ID NO: 20, or of a fragment or variant thereof.
SEQ ID NO: 20:
GGCUCGACUGAUGAGGCGC

It is further preferred that the catalytic nucleic acid molecule does not comprise or consist of a DNA sequence corresponding to SEQ ID NO: 20, or of a fragment or variant thereof.

In another particularly preferred embodiment, the catalytic nucleic acid molecule is a DNAzyme, e.g. a"10-23" DNAzyme.

As used herein, the terms "DNAzyme" or "DNA enzyme" typically refer to a catalytic DNA molecule.

The catalytic nucleic acid molecule does preferably not comprise or consist of a nucleic acid sequence according to any one of SEQ ID NO: 21, 22, 23, or 24, or of a fragment or variant thereof. Preferably, the catalytic nucleic acid molecule does not comprise or consist of a nucleic acid sequence identical to or at least 80% identical to a nucleic acid sequence according to any one of SEQ ID NO: 21, 22, 23, or 24.
SEQ ID NO: 21:
   TGCTGCTGGGCTAGCTACAACGATGCTGCTG
SEQ ID NO: 22:
   GGCTGTTGGGCTAGCTACAACGATGCTGCTG
SEQ ID NO: 23:
   GGCGGTGGGGCTAGCTACAACGAGGCTGTTG
SEQ ID NO: 24:
   GGGCACCAGGCTAGCTACAACGATCTTTTTAATTTC

The catalytic nucleic acid molecule does preferably not comprise or consist of an RNA sequence corresponding to a nucleic acid sequence according to any one of SEQ ID NO: 21, 22, 23, or 24, or of a fragment or variant thereof. Preferably, the catalytic nucleic acid molecule does not comprise or consist of an RNA sequence corresponding to a nucleic acid sequence identical to or at least 80% identical to any one of SEQ ID NO: 21, 22, 23, or 24.

Preferably, the catalytic nucleic acid molecule as described herein, more preferably ribozyme or a catalytic DNA molecule, most preferably a catalytic DNA molecule, does not cleave an RNA encoding Huntington's Disease (HD) protein.

The catalytic nucleic acid molecule is preferably not a catalytic DNA molecule.

By the cleavage with the catalytic nucleic acid molecule, the RNA molecule having at least one cleavage site for the at least one catalytic nucleic acid molecule is specifically cleaved at that (at least one) defined site so that a 3' terminal, a 5' terminal RNA fragment and optionally at leat one central RNA fragement is produced.

Step b) of the methods as defined above comprises cleavage of the RNA molecule having at least one cleavage site for at least one catalytic nucleic acid molecule with the at least one catalytic nucleic acid molecule. Therein, the RNA molecule is contacted with the at least one catalytic nucleic acid molecule under conditions allowing the cleavage of the RNA molecule. Preferably, such conditions allow the specific interaction of the catalytic nucleic acid molecule and the RNA molecule having at least one cleavage site for the at least one catalytic nucleic acid molecule, and the cleavage of the RNA molecule having at least one cleavage site. Such conditions may vary depending on the RNA molecule to be analyzed and the catalytic nucleic acid molecule that is employed. Nevertheless, methods are known in the art to select suitable conditions once a selection has been made concerning the RNA molecule to be analyzed and/or the catalytic nucleic acid molecule. The skilled person knows how to adjust the parameters, such as magnesium ion concentration, buffer composition, pH, temperature and incubation times.

Preferably, step b) of the method disclosed herein comprises denaturing the nucleic acid molecules, preferably by heating, annealing the RNA molecule to be analyzed and the catalytic nucleic acid molecule and cleavage of the RNA molecule to be analyzed, wherein the annealing and the cleavage preferably take place at a lower temperature than the denaturing. Typically, the nucleic acid molecules (i.e. the RNA molecule to be analyzed and the catalytic nucleic acid molecule) are heated either together (i.e. in a mixture) or separately in a suitable buffer that does preferably not contain magnesium ions (Mg⁺⁺). Subsequently, the nucleic acid molecules are cooled to cleavage reaction temperature, either together or separately. Preferably, the heating step involves heating of the buffer containing the nucleic acid molecules to a temperature of at least 70°C, more preferably at least 80°C, 85°C, 90°C, 95°C or at least 96°C, preferably for at least 30 seconds, 60 seconds, 90 seconds or at least 120 seconds. After the heating step, the nucleic acid molecules are typically cooled down to the cleavage reaction temperature, which is typically lower than the temperature in the initial heating step. Preferably, the nucleic acid molecules are cooled in a controlled manner, for instance at a rate of 0.1 °C per second. The cleavage reaction preferably takes place at a temperature from 20°C to 50°C, more preferably from 20°C to 40°C, 24°C to 38°C or 25°C to 37°C, most preferably at 25°C or 37°C, for a period of preferably at least 1, 2, 3, 4, 5, 6, 7, 8, 9, 10, 15, 30, or 60 minutes. After cooling of the heated nucleic acid molecules and before starting the cleavage reaction (e.g. by addition of magnesium ions (Mg⁺⁺)), an optional annealing step is employed, wherein the temperature is preferably equal to the cleavage reaction temperature and which is typically carried out in absence of magnesium ions, preferably for at least 1, 2, 3, 4, 5, 6, 7, 8, 9 or at least 10 minutes.

Preferably, the RNA molecule to be analyzed and the catalytic nucleic acid molecule, preferably a ribozyme, are provided in about the same molar amounts.

The catalytic nucleic acid molecule, preferably a ribozyme, and the RNA molecule to be analyzed are preferably heated together at, for example, 95°C, preferably for 1 to 2 minutes, in the presence of water or buffer without magnesium ions, and subsequently cooled, preferably at a controlled cooling rate, to the reaction temperature of 20-37°C, preferably 25°C, in order to promote annealing. Subsequently, Mg⁺⁺ (e.g. MgCl₂) is added to initiate the cleavage reaction. Alternatively, the catalytic nucleic acid molecule, preferably a ribozyme, and the RNA molecule to be analyzed are preferably heated separately at, for example, 95°C without Mg⁺⁺, preferably for one to two minutes, and are then cooled to the reaction temperature. Mg⁺⁺ is added to both the catalytic nucleic acid molecule and the RNA to be analyzed and the cleavage reaction is started by mixing both. In the method disclosed herein, the cleaving in step b) preferably takes place in the presence of at least 10, 20 or 30 mM Mg⁺⁺, most preferably in presence of 30 mM MgCl₂.

In order to achieve a sufficient degree of cleavage of the RNA molecule to be analyzed, the Mg⁺⁺ concentration, buffer composition, pH value, temperature and reaction time may need to be adjusted. As used herein, the phrase "conditions allowing the cleavage of the RNA molecule" refers to conditions, which - at suitable incubation time - preferably allow cleavage of at least 50%, preferably at least 75%, 80%, 85%, 90%, 95% or 98% of the RNA molecules in a population, which have at least one cleavage site for at least one catalytic nucleic acid molecule. For example, "conditions allowing the cleavage of the RNA molecule" may comprise 50-200 mM NaCl or KCI, 0.1-200 mM Mg⁺⁺, 5-100 mM Tris-HCl, pH 6.5-8.5, 20-37°C for 5 minutes to 2 hours. A non-ionic detergent (Tween, NP-40, Triton-X 100) is preferably present, usually at about 0.001 to 2%, typically 0.05-0.2% (volume/volume).

The cleavage of the RNA molecule having at least one cleavage site for at least one catalytic nucleic acid molecule with the at least one catalytic nucleic acid molecule, leads to the generation of a 3' terminal RNA fragment, a5' terminal RNA fragment and optionally at least one central RNA fragment. The number of central RNA fragments depends on the number of cleavage sites for catalytic nucleic acid molecules. For example, cleavage of an RNA molecule having one cleavage site typically leads to a 3' terminal RNA fragment and a 5' terminal RNA fragment. Therefore no central RNA fragment is generated. On the other hand, cleavage of an RNA molecule having two cleavage sites typically results in three RNA fragments, i.e. a 3' terminal RNA fragment, a 5' terminal RNA fragment and a central RNA fragment. Cleavage of an RNA molecule having three cleavage sites (for the same catalytic nucleic acid molecule or for different catalytic nucleic acid molecules) typically results in four RNA fragments, i.e. a 3' terminal RNA fragment, a 5' terminal RNA fragment and two central RNA fragments.

The method disclosed herein does preferably not involve cleavage of the RNA molecule by a protein enzyme having ribonuclease activity, such as a ribonuclease (RNase), e.g. RNase H, RNase T1 or RNase T2. More preferably, a protein enzyme having ribonuclease activity is not used in the method disclosed herein.

Step b) of the method disclosed herein preferably comprises cleaving the RNA molecule once with each catalytic nucleic acid molecule. A single cleavage by a given catalytic nucleic acid molecule is preferably obtained by (a) designing the cleavage site in the RNA molecule and/or the catalytic nucleic acid molecule and/or (b) carrying out step b) under stringent conditions in order to provide for sufficient specificity of the catalyzed cleavage reaction resulting in a single cleavage of the RNA molecule.

Step c) of the method disclosed herein comprises determining a physical property of the RNA molecule by analyzing at least one RNA fragment.

In the context of the present disclosure, the expression "a physical property" (or "physical properties") typically refers to a physical property or to a structural feature of an RNA molecule. Where the plural ("physical properties") is used, it may likewise refer to a single property or single feature. Preferably, the expression as used herein refers to a physical property or a structural feature of the RNA molecule, which distinguishes the RNA molecule from other, preferably structurally related, RNA molecules. Preferably, a physical property or a structural feature is capable of distinguishing the RNA molecule from a similar, preferably structurally related, RNA molecule lacking the physical property or a structural feature, or differing in that physical property or structural feature. More preferably the RNA molecule is identical apart from the lacking physical property or the lacking structural feature or apart from the difference in the physical property or structural feature. Typically, the distinct physical property reflects a structural feature, such as e.g. a distinct molecular weight, charge, length or specific nucleotide composition. As used herein, a physical property or a structural feature may preferably be determined by standard analytical methods known in the art. Preferably, a physical property or a structural feature can be determined after cleavage of the RNA molecule having at least one cleavage site for at least one catalytic nucleic acid molecule. According to the present disclosure, a distinct physical property or a distinct structural feature of the RNA molecule having at least one cleavage site for at least one catalytic nucleic acid molecule is determined by analysis of at least one RNA fragment obtained after cleavage of the RNA molecule with the at least one catalytic nucleic acid molecule. In other words, the at least one RNA fragment obtained by cleavage of the RNA molecule having at least one cleavage site for at least one catalytic nucleic acid molecule with the at least one catalytic nucleic acid molecule reflects a physical property or a structural feature of the RNA molecule. Thus, by analyzing the at least one RNA fragment, preferably with respect to a distinct physical property or a structural feature as defined herein, a distinct physical property of the RNA molecule, from which the at least one RNA fragment is derived, is determined. The physical property or structural feature that is determined is preferably selected from the mass of the at least one RNA fragment, the molecular weight of the at least one RNA fragment, the charge of the at least one RNA fragment, the nucleotide sequence of the at least one RNA fragment, the length of the at least one RNA fragment, and the presence or absence, respectively, of at least one nucleotide, e.g. a modified nucleotide, a modification as defined herein, or a specific moiety of a nucleotide, preferably of a modified nucleotide, such as a modified base, e.g. in the 3' terminal RNA fragment. In other words, the expression "determining a physical property" as used herein may also refer to determining the identity and/or the integrity of the at least one RNA fragment.

The identity and/or the integrity of the at least one RNA fragment is preferably determined in step c) by a method known in the art, which is suitable for determining the nucleic acid sequence of the at least one RNA fragment. By comparison of the nucleic acid sequence of the at least one RNA fragment with a reference RNA fragment or with the corresponding fragment in the nucleic acid sequence, which was used as a template for synthesis of the RNA molecule having at least one cleavage site for at least one catalytic nucleic acid molecule. By this comparison, the method preferably allows to control successful synthesis of the RNA molecule having at least one cleavage site for at least one catalytic nucleic acid molecule. In this context, the term 'template for synthesis' may refer to a nucleic acid sequence, which is used as a template for chemical synthesis or as a template for *in vitro* transcription. in the method disclosed herein, the RNA having at least one cleavage site for at least one catalytic nucleic acid molecule is produced by *in vitro* transcription and the identity and/or the integrity of the at least one RNA fragment, preferably of the 3' terminal RNA fragment is preferably determined in step c) by comparison of the nucleic acid sequence of the at least one RNA fragment with a reference RNA fragment or with the nucleic acid sequence of the corresponding fragment in a DNA, which was used as a template in *in vitro* transcription.

Step c) preferably comprises determining the mass and/or the length of the at least one RNA fragment, preferably of the 3' terminal RNA fragment. Preferably, the length of the at least one RNA fragment is defined by the number of nucleotides comprised in the at least one RNA fragment. Thus, the length of the at least one RNA fragment is preferably referred to herein in nucleotides. For example, the expression '(nucleic acid molecule) having a length of 127 nucleotides' preferably refers to a nucleic acid molecule consisting of 127 nucleotides.

Step c) preferably involves separating or resolving the at least one RNA fragment from the other resulting RNA fragments. Preferably, the 3' terminal RNA fragment is separated or resolved from the 5' terminal RNA fragment and/or the optional at least one central RNA fragment. In order to determine the physical property of the at least one RNA fragment - or the respective RNA molecule, from which it is derived - it is typically sufficient to resolve the RNA fragment in any manner, i.e. to employ an analytic technique that allows to determine the presence or absence of an RNA fragment with certain physical properties. By determining the presence or absence of said fragment with a certain physical property, the skilled person is capable of determining the physical property of the RNA molecule, from which the RNA fragment is derived. To this end, the RNA fragment does not necessarily need to be physically separated or isolated from another RNA fragment or other fragments that may be present. The resolution of an RNA fragment with a certain physical property may also be achieved in mixture, e.g. by using labelling techniques or molecular markers and relevant methods for detection.

The at least one RNA fragment is preferably separated from another RNA fragment, preferably from the 5' terminal RNA fragment and/or from the optional at least one central RNA fragment. Any suitable method for separating RNA fragments can be used, including, but not limited to, denaturing gel electrophoresis (e.g. agarose gel electrophoresis, polyacrylamide gel electrophoresis, chip gel electrophoresis, etc.) or liquid chromatography. In general, the separation technique is used according to the characteristics, e.g. the size, of the RNA fragments to be separated. The skilled person can thus select a suitable separation technology on the basis of the characteristics of the expected RNA fragment.

In a first aspect disclosed herein, the RNA fragments are preferably separated in step c) by denaturing gel electrophoresis or liquid chromatography, preferably HPLC, FPLC or RPLC. Separation of RNA molecules by denaturing gel electrophoresis has been described (Maniatis et al., 1975. Biochemistry 14(17):3787-3794). For example, polyacrylamide gels that contain a high concentration of a denaturing agent such as urea are capable of resolving short (<500 nucleotides) single-stranded RNA fragments that differ in length by as little as one nucleotide. In this context, polyacrylamide gels comprising urea, preferably 8 M urea, are particularly preferred.

The RNA fragments obtained by cleavage of the RNA molecule having at least one cleavage site for at least one catalytic nucleic acid molecule can also be separated by liquid chromatography. As used herein, the term "liquid chromatography" (LC) preferably refers to a process of selective retardation of one or more components of a fluid solution as the fluid uniformly percolates through a column of a finely divided, preferably porous, substance, or through capillary passageways. The retardation results from the distribution of the components of the mixture between one or more stationary phases and the bulk fluid (i.e. the mobile phase), as this fluid moves relative to the stationary phase(s). LC includes reverse phase liquid chromatography (RPLC), high performance liquid chromatography (HPLC), high turbulence liquid chromatography (HTLC) and fast performance liquid chromatography (FPLC). In contrast to HPLC, the buffer pressure used in FPLC is relatively low, typically less than 5 bar, but the flow rate is relatively high, typically 1-5 ml/min.

Stationary phases for the use in liquid chromatography are known in the art. Preferably, the stationary phase is selected from the group consisting of a porous polystyrene, a porous non-alkylated polystyrene, a polystyrenedi-vinylbenzene, a porous non-alkylated polystyrenedivinylbenzene, a porous silica gel, a porous silica gel modified with non-polar residues, a porous silica gel modified with alkyl containing residues, selected from butyl-, octyl and/or octadecyl containing residues, a porous silica gel modified with phenylic residues, and a porous polymethacrylate (see also WO2008077592). In this context the stationary phase is preferably selected from porous silica gel modified with alkyl containing residues, preferably octadecyl containing residues. More preferably the porous silica gel is selected from polyethoxysilane which is preferably modified with octadecyl containing residues (e.g. XBRIDGE^{™} OST C₁₈ from Waters).

In this context, ethylene-bridged hybrid organic/inorganic stationary phases are particularly preferred (see also Wyndham et al., 2003. Anal. Chem. 75(24):6781-8 and WO2003014450).

For example, the separation process of RNA molecules by HPLC has been described (Weissman et al., 2013. Methods Mol. Biol. 969:43-54).

Preferably, the separation of the at least one RNA fragment in itself already reveals the distinct property of the RNA molecule, from which it is derived and which is to be analyzed. For example, if the absence of nucleotides, the presence of additional nucleotides or a modification in the at least one RNA fragment, which alters a physical property of the RNA fragment, such as its mass or its length, is investigated, then it is typically enough to separate the RNA fragments in order to determine the physical property.

Preferably, step c) comprises comparison of a structural feature or of a physical parameter of the at least one RNA fragment, and the respective feature or parameter of a reference RNA fragment. For example, the at least oneRNA fragment may be compared to a reference RNA fragment, which is known to exhibit a certain property, in order to confirm that property in the at least one RNA fragment obtained in step b). Preferably, this comparison is carried out after separation of the at least one RNA fragment obtained in step b). More preferably, the separated RNA fragment may thus be compared to a reference RNA having a defined value for the physical property (e.g. a known mass or a known length).

The at least one separated RNA fragment is preferably further analyzed by further analytical methods in order to determine the distinct physical property of the at least one RNA fragment.

The physical property of the at least one RNA fragment is preferably determined in step c) by spectroscopic methods, quantitative mass spectrometry, or sequencing.

Spectroscopic methods for RNA analysis include traditional absorbance measurements at 260 nm and more sensitive fluorescence techniques using fluorescent dyes such as ethidium bromide and a fluorometer with an excitation wavelength of 302 or 546 nm (Gallagher, 2011. Quantitation of DNA and RNA with Absorption and Fluorescence Spectroscopy. Current Protocols in Molecular Biology. 93:A.3D.1-A.3D.14).

A mass spectrometer (MS) is a gas phase spectrometer that measures a parameter that can be translated into mass-to-charge ratio of gas phase ions. Examples of mass spectrometers are time-of-flight, magnetic sector, quadrupole filter, ion trap, ion cyclotron resonance, electrostatic sector analyser and hybrids of these. Methods for the application of MS methods to the characterization of nucleic acids are known in the art.

For example, Matrix-Assisted Laser Desorption/Ionization Mass Spectrometry (MALDI-MS) can be used to analyse oligonucleotides at the 120-mer level and below (Castleberry et al., 2008. Matrix-Assisted Laser Desorption/lonization Time-of-Flight Mass Spectrometry of Oligonucleotides. Current Protocols in Nucleic Acid Chemistry. 33:10.1.1-10.1.21).

Electrospray Ionization Mass Spectrometry (ESI-MS) allows the analysis of high-molecular-weight compounds through the generation of multiply charged ions in the gas phase and can be applied to molecular weight determination, sequencing and analysis of oligonucleotide mixtures (Castleberry et al., 2008. Electrospray Ionization Mass Spectrometry of Oligonucleotides. Current Protocols in Nucleic Acid Chemistry. 35:10.2.1-10.2.19). Preferably, the mass spectrometry analysis is conducted in a quantitative manner to determine the amount of RNA.

Methods for sequencing of RNA are known in the art. A recently developed technique called RNA Sequencing (RNA-Seq) uses massively parallel sequencing to allow for example transcriptome analyses of genomes at a far higher resolution than is available with Sanger sequencing- and microarray-based methods. In the RNA-Seq method, complementary DNAs (cDNAs) generated from the RNA of interest are directly sequenced using next-generation sequencing technologies. RNA-Seq has been used successfully to precisely quantify transcript levels, confirm or revise previously annotated 5' and 3' ends of genes, and map exon/intron boundaries (Eminaga et al., 2013. Quantification of microRNA Expression with Next-Generation Sequencing. Current Protocols in Molecular Biology. 103:4.17.1-4.17.14). Consequently, the amount of the RNA fragments can be determined also by RNA sequencing.

Step c) of the method disclosed herein preferably comprises analyzing the at least one RNA fragment without determining its sequence. More preferably, a physical property as defined herein is determined in step c) without using sequence analysis. In that embodiment, the physical property can advantageously be determined without sequencing the RNA fragment, but by merely using one of the other methods (such as chromatographic techniques, e.g. HPLC) described herein to analyze the RNA fragment.

Step c) comprises analyzing the at least one RNA fragment by comparison to a reference RNA fragment. In particular, step c) comprises comparison of a structural feature or of a physical parameter of the at least one RNA fragment and the respective feature or parameter of a reference RNA fragment. Preferably, at least one reference RNA fragment is used as reference. The at least one RNA fragment obtained in step b) of the method disclosed herein is thus compared to one or more reference RNA fragments. For example, the at least one RNA fragment having a physical property of interest (e.g. a defined mass and/or a defined length) may be analyzed in parallel with the at least one RNA fragment derived from the RNA molecule comprising at least one cleavage site for at least one catalytic nucleic acid molecule, which is to be analyzed. Alternatively, the at least one RNA fragment is analysed by comparison with a reference RNA *in silico* which means by comparison with the expected RNA sequence of the at least one RNA fragment.

The method disclosed herein is preferably used for controlling the quality of RNA, preferably for controlling the quality of RNA produced by *in vitro* transcription. Preferably, the method is employed for controlling the quality of artificial RNA, preferably an mRNA, which is preferably synthesized by *in vitro* transcription.

The method is preferably used for determining a physical property or a structural feature in an RNA molecule, having at least one cleavage site for at least one catalytic nucleic acid molecule. The structural feature is preferably located between the 3' terminus of the RNA molecule to be analysed and the cleavage site for the at least one catalytic nucleic acid molecule.

The method is preferably used for determining the presence of a 3' terminal modification, in particular the absence of nucleotides or the presence of additional (non-templated) nucleotides as defined herein. Preferably, the method is used for determining a structural feature selected from the length of the 3' terminal RNA fragment, absence of a nucleotide or of a plurality of nucleotides, presence and/or integrity of a homopolymeric stretch (e.g. a poly(A) or poly(C) sequence), presence of additional nucleotides, e.g. at the 3' terminus of the RNA molecule having at least one cleavage site for at least one catalytic nucleic acid molecule.

Preferably, all RNA fragments (the 5' terminal RNA fragment, the 3' terminal RNA fragment and the optional central RNA fragments) resulting from the cleavage of the RNA molecule having at least one cleavage site for at least one catalytic nucleic acid molecule with at least one catalytic nucleic acid molecule are analysed for at least one structural feature or physical property. In this case modifications (presence or absence of a 5' CAP structure, absence of nucleotides, presence of additional nucleotides etc.) in the whole RNA molecule to be analysed can be detected, particularly by determining the length of all resulting RNA fragments.

In particular, if the RNA molecule to be analysed comprises at least two cleavage sites for at least one catalytic nucleic acid molecule, at least one structural feature or physical property of the optional at least one central RNA fragment is preferably analysed. This may be particularly preferred if in that part of the RNA molecule to be analysed corresponding to the at least one central RNA fragment deletion/absence and/or addition of nucleotides have to be analyzed (e.g. in tandem repeat regions). In this context it is particularly preferred to determine the length of the at least one central RNA fragment.

In case the RNA molecule to be analysed comprises at least two cleavage sites for at least one catalytic nucleic acid molecule it is particularly preferred that the resulting 5' terminal fragment and the at least one central RNA fragment is analysed for at least one structural feature or physical property, preferably the length of the RNA fragments. This might be particularly preferred if mRNA comprising a 5' CAP structure has to be analysed. In this case the presence of a 5' CAP structure, the orientation of the CAP structure or the capping degree might be determined as described in PCT/EP2014/003482.

It is particularly preferred to analyse the 3' terminal RNA fragment and the at least one central RNA fragment of the RNA molecule comprising at least two cleavage sites for at least one catalytic nucleic acid molecule. This might be particularly preferred if e.g. the presence and/or integrity of a homopolymeric stretch (e.g. a poly(A) or poly(C) sequence) in the 3' terminal RNA fragment has to be analysed and e.g. a tandem repeat region in the at least one central RNA fragment.

It is particularly preferred to analyse the 5' terminal RNA fragment and the 3' terminal RNA fragment. This might be particularly preferred to determine simultaneously the presence or absence of a 5' CAP structure or the orientation of the 5' CAP structure in the 5' terminal RNA fragment and the presence or absence of nucleotides in the 3' terminal RNA fragment e.g the presence and/or integrity of a homopolymeric stretch (e.g. a poly(A) or poly(C) sequence). Also in this case it is particularly preferred to determine the length of the 5' terminal RNA fragment and of the 3' terminal RNA fragment. The presence of a 5' CAP structure, the orientation of the CAP structure or the capping degree might be determined as described in PCT/EP2014/003482.

The RNA molecule having at least one cleavage site for at least one catalytic nucleic acid molecule is an mRNA molecule, preferably as described herein.

The RNA molecule having at least one cleavage site for at least one catalytic nucleic acid molecule is an mRNA molecule, which comprises a 3' untranslated region (3'-UTR).

Preferably, the RNA having at least one cleavage site for at least one catalytic nucleic acid molecule is an mRNA comprising a 3'-UTR, wherein the 3'-UTR comprises a poly(A) sequence. The length of the poly(A) sequence may vary. For example, the poly(A) sequence may have a length of about 20 adenine nucleotides up to about 300 adenine nucleotides, preferably of about 40 to about 200 adenine nucleotides, more preferably from about 50 to about 100 adenine nucleotides, such as about 60, 70, 80, 90 or 100 adenine nucleotides. Most preferably, the RNA molecule comprises a poly(A) sequence of about 60 to about 70 nucleotides, most preferably 64 adenine nucleotides. The poly(A) sequence may be located at the 3' terminus of the RNA molecule or within the 3'-UTR.

Preferably, the poly(A) sequence in the RNA molecule having at least one cleavage site for at least one catalytic nucleic acid molecule is derived from a DNA template by *in vitro* transcription. Alternatively, the poly(A) sequence may also be obtained *in vitro* by common methods of chemical-synthesis or by enzymatic polyadenylation (e.g. by poly(A) polymerase from *E. coli*) without being necessarily transcribed from a DNA-progenitor.

Alternatively, the RNA molecule optionally comprises a polyadenylation signal, which is defined herein as a signal, which conveys polyadenylation to a (transcribed) mRNA by specific protein factors (e.g. cleavage and polyadenylation specificity factor (CPSF), cleavage stimulation factor (CstF), cleavage factors I and II (CF I and CF II), poly(A) polymerase (PAP)). In this context, a consensus polyadenylation signal is preferred comprising the NN(U/T)ANA consensus sequence. In a particularly preferred aspect, the polyadenylation signal comprises one of the following sequences: AA(U/T)AAA or A(U/T)(U/T)AAA (wherein uridine is usually present in RNA and thymidine is usually present in DNA).

In addition or as an alternative to a poly(A) sequence as described above, the RNA molecule having at least one cleavage site for at least one catalytic nucleic acid molecule may also comprise a poly(C) sequence, preferably in the region 3' of the coding region of the RNA. A poly(C) sequence is typically a stretch of multiple cytosine nucleotides, typically about 10 to about 200 cytidine nucleotides, preferably about 10 to about 100 cytidine nucleotides, more preferably about 10 to about 70 cytidine nucleotides or even more preferably about 20 to about 50 or even about 20 to about 30 cytidine nucleotides. A poly(C) sequence may preferably be located 3' of an open reading frame comprised in the RNA having at least one cleavage site for at least one catalytic nucleic acid molecule. As disclosed herein, the RNA molecule preferably comprises a poly(A) sequence and a poly(C) sequence, wherein the poly(C) sequence is located 3' of the poly(A) sequence.

The RNA molecule having at least one cleavage site for at least one catalytic nucleic acid molecule preferably comprises in 5'-to-3'-direction, a 5'-UTR, an open reading frame, preferably a modified open reading frame as defined herein, a 3'-UTR element and a poly(A) or a poly(C) sequence. In addition, the RNA preferably comprises a histone stem-loop sequence, preferably as defined herein.

The RNA molecule having at least one cleavage site for at least one catalytic nucleic acid molecule, preferably an mRNA, preferably comprises a 3'-UTR, which may comprise at least one histone stem-loop, such as a histone stem-loop sequence and/or a histone stem-loop structure. Such histone stem-loop sequences are preferably selected from histone stem-loop sequences as disclosed in WO2012/019780. A histone stem-loop structure is a structure of mRNA that is formable or formed by a histone stem-loop sequence of RNA in physiological conditions eg intra-cellular and/or when included pharmaceutical formulation.

A histone stem-loop sequence, suitable to be used within the present disclosure, is preferably selected from at least one of the following formulae (I) or (II):
**formula (I)** (stem-loop sequence without stem bordering elements): 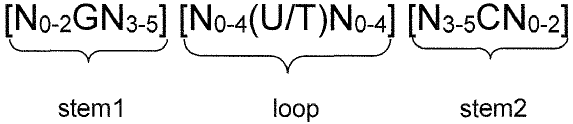
**formula (II)** (stem-loop sequence with stem bordering elements): 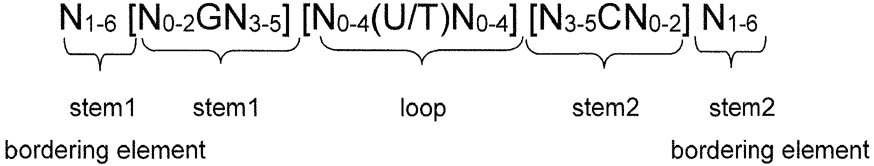
wherein:
   - stem1 or stem2 bordering elements N₁₋₆: is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
   - stem1 [N₀₋₂GN₃₋₅]: is reverse complementary or partially reverse complementary with element stem2, and is a consecutive sequence between of 5 to 7 nucleotides; wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof; wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and wherein G is guanosine or an analogue thereof, and may be optionally replaced by a cytidine or an analogue thereof, provided that its complementary nucleotide cytidine in stem2 is replaced by guanosine;
   - loop sequence [N₀₋₄(U/T)N₀₋₄]: is located between elements stem1 and stem2, and is a consecutive sequence of 3 to 5 nucleotides, more preferably of 4 nucleotides; wherein each N₀₋₄ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and wherein U/T represents uridine, or optionally thymidine;
   - stem2 [N₃₋₅CN₀₋₂]: is reverse complementary or partially reverse complementary with element stem1, and is a consecutive sequence between of 5 to 7 nucleotides; wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof; wherein N₀₋₂ is a consecutive sequence of 0 to 2, preferably of 0 to 1, more preferably of 1 N, wherein each N is independently from another selected from a nucleotide selected from A, U, T, G or C or a nucleotide analogue thereof; and wherein C is cytidine or an analogue thereof, and may be optionally replaced by a guanosine or an analogue thereof provided that its complementary nucleoside guanosine in stem1 is replaced by cytidine;
   wherein
   stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one ore more bases in one stem do not have a complementary base in the reverse complementary sequence of the other stem.

As disclosed herein, at least one histone stem-loop sequence, if included in the mRNA construct, may comprise at least one of the following specific formulae (Ia) or (Ila):
formula (Ia) (stem-loop sequence without stem bordering elements): 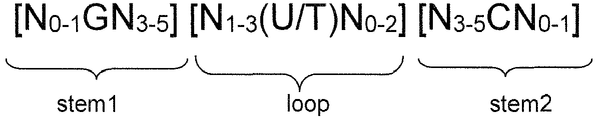
formula (Ila) (stem-loop sequence with stem bordering elements): 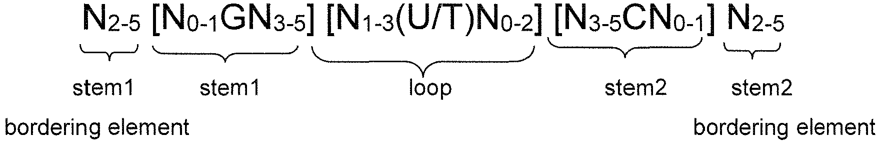
wherein:
   N, C, G, T and U are as defined above.

As disclosed herein, at least one histone stem-loop sequence, if included in the mRNA construct, may comprise at least one of the following specific formulae (Ib) or (IIb):
formula (Ib) (stem-loop sequence without stem bordering elements): 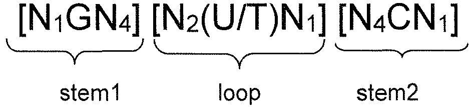
formula (lib) (stem-loop sequence with stem bordering elements): 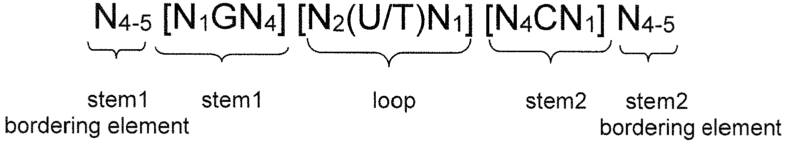
wherein:
   N, C, G, T and U are as defined above.

A particular preferred histone stem-loop sequence is the nucleic acid sequence according to SEQ ID NO. 12 (or a homolog, a fragment or a variant thereof):
Histone stem-loop nucleotide sequence (SEQ ID NO. 12)
CAAAGGCTCTTTTCAGAGCCACCA

More preferably the stem-loop sequence is the corresponding RNA sequence of the nucleic acid sequence according to SEQ ID NO. 12 (or a homolog, a fragment or a variant thereof):
Histone stem-loop RNA sequence (SEQ ID NO. 13)
CAAAGGCUCUUUUCAGAGCCACCA

The RNA molecule having at least one cleavage site for at least one catalytic nucleic acid molecule can (additionally) comprise at least one of the following structural elements: a 5'-and/or 3'- untranslated region element (UTR element), particularly a 5'-UTR element which comprises or consists of a nucleic acid sequence, which is derived from the 5'-UTR of a TOP gene or from a fragment, homolog or a variant thereof, or a 5'- and/or 3'-UTR element, which may be derivable from a gene that provides a stable mRNA or from a homolog, fragment or variant thereof; a histone-stem-loop structure, preferably a histone-stem-loop in its 3' untranslated region; a 5'-CAP structure; a poly(A) sequence or a poly(A) tail; or a poly(C) sequence.

Accordingly, the RNA molecule having at least one cleavage site for at least one catalytic nucleic acid molecule preferably comprises at least one 5'- or 3'-UTR element. In this context, an UTR element comprises or consists of a nucleic acid sequence, which is derived from the 5'- or 3'-UTR of any naturally occurring gene or which is derived from a fragment, a homolog or a variant of the 5'- or 3'-UTR of a gene. Preferably the 5'- or 3'-UTR element used according to the present disclosure is heterologous to the coding region of the mRNA construct. Even if 5'- or 3'-UTR elements derived from naturally occurring genes are preferred, also synthetically engineered UTR elements may be used in the context of the present disclosure.

In respect of a 3'-UTR element, the present disclosure also includes mRNA constructs that include a 3'-UTR element which comprises or consists of a nucleic acid sequence derived from the 3'-UTR of a chordate gene, preferably a vertebrate gene, more preferably a mammalian gene, most preferably a human gene, or from a variant of the 3'-UTR of a chordate gene, preferably a vertebrate gene, more preferably a mammalian gene, most preferably a human gene.

The term '3'-UTR element' refers to a nucleic acid sequence, which comprises or consists of a nucleic acid sequence that is derived from a 3'-UTR or from a variant of a 3'-UTR. A 3'-UTR element in the sense of the present disclosure may represent the 3'-UTR of an mRNA. Thus, in the sense of the present disclosure, preferably, a 3'-UTR element may be the 3'-UTR of an mRNA, preferably of an artificial mRNA, or it may be the transcription template for a 3'-UTR of an mRNA. Thus, a 3'-UTR element preferably is a nucleic acid sequence, which corresponds to the 3'-UTR of an mRNA, preferably to the 3'-UTR of an artificial mRNA, such as an mRNA obtained by transcription of a genetically engineered vector construct. Preferably, the 3'-UTR element fulfils the function of a 3'-UTR or encodes a sequence which fulfils the function of a 3'-UTR.

As disclosed herein, the RNA molecule having at least one cleavage site for at least one catalytic nucleic acid molecule preferably comprises a 3'-UTR, wherein the 3'-UTR comprises or consists of a nucleic acid sequence, which is derived from a 3'-UTR of a gene providing a stable mRNA or from a homolog, or it may be a fragment or a variant of such a gene. In certain embodiments, the mRNA construct comprises a 3'-UTR element, which may be derivable from a gene that relates to an mRNA with an enhanced half-life (that provides a stable mRNA), for example a 3'-UTR element as defined and described below.

Accordingly, the 3'-UTR preferably comprises a nucleic acid sequence, which is heterologous with respect to at least one selected from a 5'-UTR, an ORF and a further nucleic acid sequence comprised in the 3'-UTR. Even more preferably, the 3'-UTR comprises a nucleic acid sequence, which is heterologous to any other element comprised in the artificial nucleic acid as defined herein. For example, if the RNA having at least one cleavage site for at least one catalytic nucleic acid molecule comprises a 3'-UTR element from a given gene, it does preferably not comprise any other nucleic acid sequence, in particular no functional nucleic acid sequence (e.g. coding or regulatory sequence element) from the same gene, including its regulatory sequences at the 5' and 3' terminus of the gene's ORF. The RNA having at least one cleavage site for at least one catalytic nucleic acid molecule preferably comprises an ORF, a 3'-UTR and a 5'-UTR, all of which are heterologous to each other, e.g. they are recombinant as each of them is derived from different genes (and their 5' and 3' UTR's). The 3'-UTR is preferably not derived from a 3'-UTR of a viral gene or is of non-viral origin.

Preferably, the 3'-UTR comprises a nucleic acid sequence derived from the 3'-UTR of a gene selected from the group consisting of an albumin gene, a globin gene and a ribosomal protein gene.

For example, the 3'-UTR element preferably comprises or consists of a nucleic acid sequence, which is derived from a 3'-UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene, or from a variant of a 3'-UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene according to SEQ ID NO. 1369-1390 of the patent application WO2013/143700. The 3'-UTR element preferably comprises or consists of a nucleic acid sequence which is derived from a 3'-UTR of an albumin gene, preferably a vertebrate albumin gene, more preferably a mammalian albumin gene, most preferably a human albumin gene according SEQ ID No: 1369 of the patent application WO2013/143700. The RNA molecule having at least one cleavage site for at least one catalytic nucleic acid molecule may comprise or consist of a nucleic acid sequence which is derived from the 3'-UTR of the human albumin gene according to GenBank Accession number NM_000477.5, or from a fragment or variant thereof.

Accordingly, as disclosed herein the RNA molecule having at least one cleavage site for at least one catalytic nucleic acid molecule preferably comprises a 3'-UTR element that comprises or consists of a nucleic acid sequence derived from a 3'-UTR of a gene selected from the group consisting of an albumin gene, an alpha-globin gene, a beta-globin gene, a tyrosine hydroxylase gene, a lipoxygenase gene, and a collagen alpha gene; or from a homolog, a fragment or a variant thereof.

Most preferably the 3'-UTR element comprises the nucleic acid sequence derived from a fragment of the human albumin gene according to SEQ ID No: 1376 of the patent application WO2013/143700, in the following referred to as SEQ ID NO. 14, or a homolog, a fragment or a variant thereof.

Nucleotide sequence of 3'-UTR element of human albumin gene (SEQ ID NO. 14)

CATCACATTTAAAAGCATCTCAGCCTACCATGAGAATAAGAGAAAGAAAATGAAGATCAATAGCTT
ATTCATCTCTTTTTCTTTTTCGTTGGTGTAAAGCCAACACCCTGTCTAAAAAACATAAATTTCTTT
AATCATTTTGCCTCTTTTCTCTGTGCTTCAATTAATAAAAAATGGAAAGAACCT

The 3'-UTR element preferably comprises or consists of a nucleic acid sequence, which is derived from a 3'-UTR of an alpha-globin gene, preferably a vertebrate alpha- or beta-globin gene, more preferably a mammalian alpha- or beta-globin gene, most preferably a human alpha- or beta-globin gene according to SEQ ID NO. 1370 of the patent application WO2013/143700 (3'-UTR of Homo sapiens hemoglobin, alpha 1 (HBA1)), or according to SEQ ID NO. 1371 of the patent application WO2013/143700 (3'-UTR of Homo sapiens hemoglobin, alpha 2 (HBA2)), or according to SEQ ID NO. 1372 of the patent application WO2013/143700 (3'-UTR of Homo sapiens hemoglobin, beta (HBB)).

For example, the 3'-UTR element may comprise or consist of the center, alpha-complex-binding portion of the 3'-UTR of an alpha-globin gene, such as of a human alpha-globin gene, preferably according to SEQ ID NO. 15 (corresponding to SEQ ID NO. 1393 of the patent application WO2013/143700), or a homolog, a fragment or a variant thereof.

Nucleotide sequence of 3' UTR element of an alpha-globin gene (SEQ ID NO. 15)
GCCCGATGGGCCTCCCAACGGGCCCTCCTCCCCTCCTTGCACCG

Accordingly, in certain embodiments the 3'-UTR element comprises or consists of, and/or is derived or derivable from, a nucleic acid sequence according to SEQ ID NO. 14 or SEQ ID NO. 15, or from a corresponding RNA sequence, a homolog, a fragment or a variant thereof.

The term 'a nucleic acid sequence, which is derived from the 3'-UTR of a [...] gene' preferably refers to a nucleic acid sequence which is based on the 3'-UTR sequence of a [...] gene or on a part thereof, such as on the 3'-UTR of an albumin gene, an alpha-globin gene, a beta-globin gene, a tyrosine hydroxylase gene, a lipoxygenase gene, or a collagen alpha gene, such as a collagen alpha 1(I) gene, preferably of an albumin gene or on a part thereof. This term includes sequences corresponding to the entire 3'-UTR sequence, i.e. the full length 3'-UTR sequence of a gene, and sequences corresponding to a fragment of the 3'-UTR sequence of a gene, such as an albumin gene, alpha-globin gene, beta-globin gene, tyrosine hydroxylase gene, lipoxygenase gene, or collagen alpha gene, such as a collagen alpha 1(I) gene, preferably of an albumin gene.

The term 'a nucleic acid sequence, which is derived from a variant of the 3'-UTR of a [...] gene' preferably refers to a nucleic acid sequence which is based on a variant of the 3'-UTR sequence of a gene, such as on a variant of the 3'-UTR of an albumin gene, an alpha-globin gene, a beta-globin gene, a tyrosine hydroxylase gene, a lipoxygenase gene, or a collagen alpha gene, such as a collagen alpha 1(I) gene, or on a part thereof as described above. This term includes sequences corresponding to the entire sequence of the variant of the 3'-UTR of a gene, i.e. the full length variant 3'-UTR sequence of a gene, and sequences corresponding to a fragment of the variant 3'-UTR sequence of a gene. A fragment in this context preferably consists of a continuous stretch of nucleotides corresponding to a continuous stretch of nucleotides in the full-length variant 3'-UTR, which represents at least 20%, preferably at least 30%, more preferably at least 40%, more preferably at least 50%, even more preferably at least 60%, even more preferably at least 70%, even more preferably at least 80%, and most preferably at least 90% of the full-length variant 3'-UTR. Such a fragment of a variant, in the sense of the present disclosure, is preferably a functional fragment of a variant as described herein.

The RNA having at least one cleavage site for at least one catalytic nucleic acid molecule preferably comprises a 3'-UTR comprising a nucleic acid sequence, which is derived from the 3'-UTR region of a gene encoding a ribosomal protein, preferably from the 3'-UTR region of ribosomal protein L9 (RPL9), ribosomal protein L3 (RPL3), ribosomal protein L4 (RPL4), ribosomal protein L5 (RPL5), ribosomal protein L6 (RPL6), ribosomal protein L7 (RPL7), ribosomal protein L7a (RPL7A), ribosomal protein L11 (RPL11), ribosomal protein L12 (RPL12), ribosomal protein L13 (RPL13), ribosomal protein L23 (RPL23), ribosomal protein L18 (RPL18), ribosomal protein L18a (RPL18A), ribosomal protein L19 (RPL19), ribosomal protein L21 (RPL21), ribosomal protein L22 (RPL22), ribosomal protein L23a (RPL23A), ribosomal protein L17 (RPL17), ribosomal protein L24 (RPL24), ribosomal protein L26 (RPL26), ribosomal protein L27 (RPL27), ribosomal protein L30 (RPL30), ribosomal protein L27a (RPL27A), ribosomal protein L28 (RPL28), ribosomal protein L29 (RPL29), ribosomal protein L31 (RPL31), ribosomal protein L32 (RPL32), ribosomal protein L35a (RPL35A), ribosomal protein L37 (RPL37), ribosomal protein L37a (RPL37A), ribosomal protein L38 (RPL38), ribosomal protein L39 (RPL39), ribosomal protein, large, P0 (RPLP0), ribosomal protein, large, P1 (RPLP1), ribosomal protein, large, P2 (RPLP2), ribosomal protein S3 (RPS3), ribosomal protein S3A (RPS3A), ribosomal protein S4, X-linked (RPS4X), ribosomal protein S4, Y-linked 1 (RPS4Y1), ribosomal protein S5 (RPS5), ribosomal protein S6 (RPS6), ribosomal protein S7 (RPS7), ribosomal protein S8 (RPS8), ribosomal protein S9 (RPS9), ribosomal protein S10 (RPS10), ribosomal protein S11 (RPS11), ribosomal protein S12 (RPS12), ribosomal protein S13 (RPS13), ribosomal protein S15 (RPS15), ribosomal protein S15a (RPS15A), ribosomal protein S16 (RPS16), ribosomal protein S19 (RPS19), ribosomal protein S20 (RPS20), ribosomal protein S21 (RPS21), ribosomal protein S23 (RPS23), ribosomal protein S25 (RPS25), ribosomal protein S26 (RPS26), ribosomal protein S27 (RPS27), ribosomal protein S27a (RPS27a), ribosomal protein S28 (RPS28), ribosomal protein S29 (RPS29), ribosomal protein L15 (RPL15), ribosomal protein S2 (RPS2), ribosomal protein L14 (RPL14), ribosomal protein S14 (RPS14), ribosomal protein L10 (RPL10), ribosomal protein L10a (RPL10A), ribosomal protein L35 (RPL35), ribosomal protein L13a (RPL13A), ribosomal protein L36 (RPL36), ribosomal protein L36a (RPL36A), ribosomal protein L41 (RPL41), ribosomal protein S18 (RPS18), ribosomal protein S24 (RPS24), ribosomal protein L8 (RPL8), ribosomal protein L34 (RPL34), ribosomal protein S17 (RPS17), ribosomal protein SA (RPSA) or ribosomal protein S17 (RPS17). Alternatively, the nucleic acid sequence may be derived from a gene encoding a ribosomal protein or from a gene selected from ubiquitin A-52 residue ribosomal protein fusion product 1 (UBA52), Finkel-Biskis-Reilly murine sarcoma virus (FBR-MuSV) ubiquitously expressed (FAU), ribosomal protein L22-like 1 (RPL22L1), ribosomal protein L39-like (RPL39L), ribosomal protein L10-like (RPL10L), ribosomal protein L36a-like (RPL36AL), ribosomal protein L3-like (RPL3L), ribosomal protein S27-like (RPS27L), ribosomal protein L26-like 1 (RPL26L1), ribosomal protein L7-like 1 (RPL7L1), ribosomal protein L13a pseudogene (RPL13AP), ribosomal protein L37a pseudogene 8 (RPL37AP8), ribosomal protein S10 pseudogene 5 (RPS10P5), ribosomal protein S26 pseudogene 11 (RPS26P11), ribosomal protein L39 pseudogene 5 (RPL39P5), ribosomal protein, large, P0 pseudogene 6 (RPLP0P6) and ribosomal protein L36 pseudogene 14 (RPL36P14). Furthermore, the 3'-UTR of the RNA having at least one cleavage site for at least one catalytic nucleic acid molecule may comprise a nucleic acid sequence derived from the 3'-UTR region of a gene selected from the group consisting of ribosomal protein S4-like (RPS4I), putative 60S ribosomal protein L13a, putative 60S ribosomal protein L37a-like protein, putative 40S ribosomal protein S10-like, putative 40S ribosomal protein S26-like 1, putative 60S ribosomal protein L39-like 5, or 60S acidic ribosomal protein P0-like. The 3'-UTR preferably comprises a nucleic acid sequence derived from a ribosomal protein S9 gene, preferably a human or murine ribosomal protein S9 gene. Exemplary human and murine nucleic acid sequences are shown below:
Homo sapiens ribosomal protein S9 (RPS9) (SEQ ID NO: 16) 
gtccacctgtccctcctgggctgctggattgtctcgttttcctgccaaataaacaggatcagcgct
ttac
Mus musculus ribosomal protein S9 (RPS9) (SEQ ID NO: 17)

TTAATACTTGGCTGAACTGGAGGATTGTCTAGTTTTCCAGCTGAAAAATAAAAAGAATTGATACT
TGG

In various aspects disclosed herein, the RNA molecule having at least one cleavage site for at least one catalytic nucleic acid molecule preferably comprises (such as in a 5' to 3' direction): (a) a 5'-CAP structure (for example, m7GpppN); and (b) an open reading frame (ORF); and (c) a 3'-UTR element comprising or consisting of a nucleic acid sequence, which is preferably derived from an alpha-globin gene (such as one comprising the corresponding RNA sequence of the nucleic acid sequence according to SEQ ID NO. 15, or a homolog, a fragment or a variant thereof); where any of such mRNA molecules may additionally comprise one or more the features (d) to (f) as follows: (d) a poly(A) sequence (such as one comprising about 64 adenosines); (e) a poly(C) sequence (such as one comprising about 30 cytosines); and/or (f) a histone-stem-loop (such as one comprising the corresponding RNA sequence to the nucleic acid sequence according to SEQ ID NO. 12, or a homolog, a fragment or a variant thereof).

In respect of a 3'-UTR element, the present disclosure also includes the RNA molecule having at least one cleavage site for at least one catalytic nucleic acid molecule that comprise at least one 5'-untranslated region element, the mRNA construct comprises additionally at least one 5'-UTR element, which comprises or consists of a nucleic acid sequence, which is derived from the 5'-UTR of a TOP gene, or from a corresponding RNA sequence, a homolog, a fragment, or a variant thereof. The 5'-UTR element preferably does not comprise (e.g. lacks) a 5'TOP motif or a 5'TOP (as defined above).

The RNA molecule having at least one cleavage site for at least one catalytic nucleic acid molecule preferably (additionally) comprises a 5'-UTR element, which comprises or consists of a nucleic acid sequence, which is derived from the 5'-UTR of a TOP gene, or from a corresponding RNA sequence, a homolog, a fragment, or a variant thereof. In certain of such embodiments, the 5'-UTR element preferably does not comprise (eg is lacking) a 5'TOP motif or a 5'TOP (as defined above).

The nucleic acid sequence of the 5'-UTR element, which is derived from a 5'-UTR of a TOP gene preferably terminates at its 3'-end with a nucleotide located at position 1, 2, 3, 4, 5, 6, 7, 8, 9 or 10 upstream of the start codon (e.g. A(U/T)G) of the gene or mRNA it is derived from. Thus, the 5'-UTR element does not comprise any part of the protein coding region. Thus, preferably, the only protein coding part of the RNA molecule having at least one cleavage site for at least one catalytic nucleic acid molecule is provided by the coding region.

The nucleic acid sequence, which is derived from the 5'-UTR of a TOP gene is preferably derived from a eukaryotic TOP gene, preferably a plant or animal TOP gene, more preferably a chordate TOP gene, even more preferably a vertebrate TOP gene, most preferably a mammalian TOP gene, such as a human TOP gene.

For example, the 5'-UTR element is preferably selected from 5'-UTR elements comprising or consisting of a nucleic acid sequence, which is derived from a nucleic acid sequence selected from the group consisting of SEQ ID Nos. 1-1363, SEQ ID NO. 1395, SEQ ID NO. 1421 and SEQ ID NO. 1422 of the patent application WO2013/143700, from the homologs of SEQ ID Nos. 1-1363, SEQ ID NO. 1395, SEQ ID NO. 1421 and SEQ ID NO. 1422 of the patent application WO2013/143700, from a variant thereof, or preferably from a corresponding RNA sequence. The term "homologs of SEQ ID Nos. 1-1363, SEQ ID NO. 1395, SEQ ID NO. 1421 and SEQ ID NO. 1422 of the patent application WO2013/143700" refers to sequences of other species than homo sapiens, which are homologous to the sequences according to SEQ ID Nos. 1-1363, SEQ ID NO. 1395, SEQ ID NO. 1421 and SEQ ID NO. 1422 of the patent application WO2013/143700.

In a preferred embodiment, the 5'-UTR element comprises or consists of a nucleic acid sequence, which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID Nos. 1-1363, SEQ ID NO. 1395, SEQ ID NO. 1421 and SEQ ID NO. 1422 of the patent application WO2013/143700, from the homologs of SEQ ID Nos. 1-1363, SEQ ID NO. 1395, SEQ ID NO. 1421 and SEQ ID NO. 1422 of the patent application WO2013/143700 from a variant thereof, or a corresponding RNA sequence. It is particularly preferred that the 5' UTR element is derived from a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID Nos. 1-1363, SEQ ID NO. 1395, SEQ ID NO. 1421 and SEQ ID NO. 1422 of the patent application WO2013/143700, from the homologs of SEQ ID Nos. 1-1363, SEQ ID NO. 1395, SEQ ID NO. 1421 and SEQ ID NO. 1422 of the patent application WO2013/143700, from a variant thereof, or a corresponding RNA sequence.

The 5'-UTR element preferably comprises or consists of a nucleic acid sequence, which is derived from a 5'-UTR of a TOP gene encoding a ribosomal protein or from a variant of a 5'-UTR of a TOP gene encoding a ribosomal protein. For example, the 5'-UTR element comprises or consists of a nucleic acid sequence, which is derived from a 5'-UTR of a nucleic acid sequence according to any of SEQ ID NOs: 67, 170, 193, 244, 259, 554, 650, 675, 700, 721, 913, 1016, 1063, 1120, 1138, and 1284-1360 of the patent application WO2013/143700, a corresponding RNA sequence, a homolog thereof, or a variant thereof as described herein, preferably lacking the 5'TOP motif. As described above, the sequence extending from position 5 to the nucleotide immediately 5' to the ATG (which is located at the 3'end of the sequences) corresponds to the 5'-UTR of said sequences.

Preferably, the 5'-UTR element comprises or consists of a nucleic acid sequence, which is derived from a 5'-UTR of a TOP gene encoding a ribosomal Large protein (RPL) or from a homolog or variant of a 5'-UTR of a TOP gene encoding a ribosomal Large protein (RPL). For example, the 5'-UTR element comprises or consists of a nucleic acid sequence, which is derived from a 5'-UTR of a nucleic acid sequence according to any of SEQ ID NOs: 67, 259, 1284-1318, 1344, 1346, 1348-1354, 1357, 1358, 1421 and 1422 of the patent application WO2013/143700, a corresponding RNA sequence, a homolog thereof, or a variant thereof as described herein, preferably lacking the 5'TOP motif.

The 5'-UTR element preferably comprises or consists of a nucleic acid sequence, which is derived from the 5'-UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, or from a variant of the 5'-UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, wherein preferably the 5'-UTR element does not comprise the 5'TOP of said gene.

A preferred sequence for a 5'-UTR element corresponds to SEQ ID NO. 1368 of the patent application WO2013/143700 (or a homolog, a fragment or a variant thereof) and reads as follows:
Nucleotide sequence for 5'-UTR element (SEQ ID NO. 18)
GGCGCTGCCTACGGAGGTGGCAGCCATCTCCTTCTCGGCATC

Accordingly, the 5'-UTR element preferably comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO. 1368 of the patent application WO2013/143700 (5'-UTR of human ribosomal protein Large 32 lacking the 5' terminal oligopyrimidine tract, SEQ ID NO. 18).

The RNA molecule having at least one cleavage site for at least one catalytic nucleic acid molecule preferably comprises a 5'-UTR element, which comprises or consists of a nucleic acid sequence, which is derived from the 5'-UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB, or from a homolog or variant thereof, wherein preferably the 5'-UTR element does not comprise a TOP-motif or the 5'TOP of said genes, and wherein optionally the 5'-UTR element starts at its 5'-end with a nucleotide located at position 1, 2, 3, 4, 5, 6, 7, 8, 9 or 10 downstream of the 5'terminal oligopyrimidine tract (TOP) and wherein further optionally the 5'-UTR element which is derived from a 5'-UTR of a TOP gene terminates at its 3'-end with a nucleotide located at position 1, 2, 3, 4, 5, 6, 7, 8, 9 or 10 upstream of the start codon (A(U/T)G) of the gene it is derived from.

The 5'-UTR element preferably comprises or consists of a nucleic acid sequence which is derived from the 5'-UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene (HSD17B4), an androgen-induced 1 gene (AIG1), cytochrome c oxidase subunit Vlc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit Vlc gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cytochrome c oxidase subunit Vic gene (COX6C), or a mammalian N-acylsphingosine amidohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP synthase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1), a human cytochrome c oxidase subunit Vic gene (COX6C), or a human N-acylsphingosine amidohydrolase (acid ceramidase) 1 gene (ASAH1) or from a variant thereof, wherein preferably the 5'-UTR element does not comprise the 5'TOP of said gene.

Accordingly, the 5'-UTR element preferably comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO. 1368, or SEQ ID NOs 1412-1420 of the patent application WO2013/143700, or a corresponding RNA sequence, or wherein the at least one 5'-UTR element comprises or consists of a fragment of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO. 1368, or SEQ ID NOs 1412-1420 of the patent application WO2013/143700, wherein, preferably, the fragment is as described above, i.e. being a continuous stretch of nucleotides representing at least 20% etc. of the full-length 5'-UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Accordingly, the 5'-UTR element preferably comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according SEQ ID NO. 1414 of the patent application WO2013/143700 (5'-UTR of ATP5A1 lacking the 5' terminal oligopyrimidine tract) or preferably to a corresponding RNA sequence, or wherein the at least one 5'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO. 1414 of the patent application WO2013/143700or more preferably to a corresponding RNA sequence, wherein, preferably, the fragment is as described above, i.e. being a continuous stretch of nucleotides representing at least 20% etc. of the full-length 5'-UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

The RNA having at least one cleavage site for at least one catalytic nucleic acid molecule comprises at least one homopolymeric sequence. As used herein, the term 'homopolymeric sequence' is used with respect to any nucleic acid sequence (which is a part of the RNA molecule having at least one cleavage site for at least one catalytic nucleic acid molecule) that comprises at least 10, preferably at least 15, at least 20, at least 25, more preferably at least 30 consecutive nucleotides (e.g. adenosine, cytidine, guanosine or uridine of the same type (e.g. a nucleic acid sequence comprising 10 consecutive adenosines or a nucleic acid sequence comprising 10 consecutive cytosines). In a preferred embodiment, a 'homopolymeric sequence' as used herein is a poly(A) or a poly(C) sequence, preferably as defined herein. As used herein, the term 'homopolymeric sequence' may refer to a nucleic acid sequence as described above, independent of the position of that sequence in the RNA molecule having at least one cleavage site for at least one catalytic nucleic acid molecule. In embodiments, where the RNA molecule having at least one cleavage site for at least one catalytic nucleic acid molecule is an mRNA, that mRNA may comprise a homopolymeric sequence, for example, in the 5'-UTR, the ORF, or in the 3'-UTR.

Accordingly, the RNA molecule having at least one cleavage site for at least one catalytic nucleic acid molecule is an mRNA, which comprises a 3'-UTR, wherein the 3'-UTR comprises at least one homopolymeric sequence, wherein the homopolymeric sequence is preferably a poly(A) sequence or a poly(C) sequence, preferably as defined herein.

According to the method disclosed herein, the RNA molecule or the sample containing the population of RNA molecules is produced by *in vitro* transcription, wherein the in vitro transcription is preferably carried out by using an RNA polymerase, preferably a bacteriophage RNA polymerase. More preferably, the bacteriophage RNA polymerase is selected from the group consisting of T3 RNA polymerase, T7 RNA polymerase and SP6 RNA polymerase.

Optionally, the *in vitro* transcription reaction may be carried out in the presence of a cap analog (co-transcriptional capping). Capped *in vitro* transcripts can be synthesized by substituting a cap analog such as a m7G(5')ppp(5')G (m7G) for a portion of the GTP in the transcription reaction, typically the cap analog is used at a four-fold excess compared to GTP. Methods for *in vitro* transcription are known in the art (Geall et al., 2013. Semin. Immunol. 25(2): 152-159) and preferably include:
1) a linearized DNA template with a promoter sequence that has a high binding affinity for its respective RNA polymerase such as bacteriophage-encoded RNA polymerases,
2) ribonucleotide triphosphates (NTPs) for the four bases (adenine, cytosine, guanine and uracil);
3) (optionally) a cap analog as defined above (e.g. m7G(5')ppp(5')G (m7G));
4) a DNA-dependent RNA polymerase (e.g. T7, T3 or SP6 RNA polymerase);
5) a ribonuclease (RNase) inhibitor to inactivate any contaminating RNase;
6) a pyrophosphatase to degrade pyrophosphate, which may inhibit transcription;
7) MgCl₂, which supplies Mg²⁺ as a co-factor for the polymerase;
8) a buffer to maintain a suitable pH value, which can also contain antioxidants and polyamines such as spermidine at optimal concentrations.

The cap analog is preferably selected from the group consisting of G[5']ppp[5']G, m⁷G[5']ppp[5']G, m₃^{2,2,7}G[5']ppp[5']G, m₂^{7,3'-O}G[5']ppp[5']G (3'-ARCA), m₂^{7,2'-O}GpppG (2'-ARCA), m₂^{7,2-O}GppspG D1 (β-S-ARCA D1) and m₂^{7,2-O}GppspG D2 (β-S-ARCA D2).

The RNA molecule, preferably the mRNA molecule, to be analyzed is preferably produced by *in vitro* transcription and subsequent enzymatic capping (e.g. post-transcriptional capping). Vaccinia Virus Capping Enzyme (VCE) possesses all three enzymatic activities necessary to synthesize an m7G cap structure (RNA 5'-triphosphatase, guanylyltransferase, and guanine-7-methyltransferase). *In vitro* transcripts can be capped in the presence of the capping enzyme, reaction buffer, GTP, and the methyl donor S-adenosylmethionine (SAM). Using GTP as substrate the VCE reaction yields RNA caps in the correct orientation. In addition, a type 1 cap can be created by adding a second Vaccinia enzyme, 2'-O-methyltransferase, to the capping reaction. RNA carrying type I caps are reported to have enhanced translational activity compared to type 0 caps (Tcherepanova et al., 2008. BMC Mol. Biol. 9:90).

The position of the at least one cleavage site in the RNA molecule is preferably such that the resulting RNA fragments can be separated or resolved, as described herein. Any size is possible for the RNA fragments to be analyzed, as long as a physical property, preferably the identity and/or integrity, more preferably the mass and/or the length of the RNA fragments can be identified. The skilled person will understand that one option to distinguish the RNA fragment to be analysed from other nucleic acid molecules may be the selection of an appropriate size of the RNA fragment by choosing an appropriate cleavage site. Alternatively or in addition to the aforementioned, the RNA fragment may also be labeled, preferably as described herein, with an appropriate marker allowing specific detection of the RNA fragment. In addition or alternatively to the separation methods mentioned above, any suitable further analytical method, preferably as described herein, may be employed in order to determine the physical property of the obtained RNA fragment(s).

The method disclosed herein preferably comprises determining the mass and/or the length of the RNA fragment(s), preferably of the 3' terminal RNA fragment and/or of the optional at least one central RNA fragment.

The method disclosed herein preferably allows distinguishing of at least two different 3'-terminal RNA fragments, of at least two different 5' terminal RNA fragments and/or of at least two different central RNA fragments (corresponding to the same part of the RNA molecule having at least one cleavage site for at least one catalytic nucleic acid molecule), which differ in length by at least 40 nucleotides, preferably by at least 20 nucleotides, more preferably by at least 10 nucleotides or even more preferably by at least 1 nucleotide, wherein the RNA fragments preferably have a size in a range from 1 to 500, from 1 to 400, from 1 to 300, from 1 to 200, from 10 to 200, from 10 to 150 or from 20 to 150 nucleotides.

RNA fragments can preferably be distinguished that differ by at least 5, 4, 3, 2 or 1 nucleotide, wherein the RNA fragments preferably have a size in a range from 1 to 75 nucleotides, more preferably from 1 to 50 nucleotides or even more preferably from 5 to 50 or from 5 to 30 nucleotides.

Preferably, the method disclosed herein preferably comprises determining a structural feature of a 3' terminal RNA fragment, wherein the structural feature is located at the 3' terminus of the 3' terminal RNA fragment or between the 3' terminus and the 5' terminus of the 3' terminal RNA fragment. In other words, the method disclosed herein allows determining of a structural feature in an RNA molecule having at least one cleavage site for at least one catalytic nucleic acid molecule, wherein the structural feature is preferably located at the 3' terminus of the RNA molecule or between the most 3' cleavage site for the catalytic nucleic acid molecule and the 3' terminus of the RNA molecule.

As disclosed herein, the RNA molecule having at leat one cleavage site for at least one catalytic nucleic acid molecule is preferably an mRNA having a 3'-UTR, wherein the at least one cleavage site for the at least one catalytic nucleic acid molecule is located in the 3'-UTR, preferably at a distance from the 3' terminus of the RNA as described above. Preferably, the method disclosed herein comprises determining the identity and/or the integrity of the 3'-UTR or a fragment thereof, wherein typically the identity and/or the integrity of the nucleic acid sequence between the cleavage site for the catalytic nucleic acid molecule and the 3' terminus of the RNA molecule is determined.

In the method disclosed herein, the presence or absence of a structural feature of the 3' terminal RNA fragment is preferably determined, wherein the structural feature is located between the most 3' cleavage site for the catalytic nucleic acid molecule and the 3' terminus of the RNA molecule. The structural feature is preferably located in the 3'-UTR of an mRNA, wherein the most 3' cleavage site is on the 5' side of the structural feature, more preferably on the 5' side of the structural feature and within the 3'-UTR.

The structural feature of the 3' terminal RNA fragment is preferably the identity and/or integrity of a homopolymeric sequence, preferably a homopolymeric sequence comprised in the 3'-UTR of an mRNA. In RNA synthesis, homopolymeric sequences are typically errorprone, so that the homopolymeric sequences in the product RNA are frequently not identical to corresponding homopolymeric sequence in the template nucleic acid sequence. In particular, if RNA is produced by *in vitro* transcription, a homopolymeric sequence in the product RNA may differ from the homopolymeric sequence by the presence of one or more additional nucleotides or by the absence of one or more nucleotides that are present in the template nucleic acid sequence. As a consequence of these changes in the homopolymeric sequences, the physical properties (e.g. mass, length and/or charge) of the product RNA are changed. The method disclosed herein allows resolution of even minor structural differences and allows distinguishing an RNA molecule comprising the correct homopolymeric sequence from an RNA molecule comprising an erroneous homopolymeric sequence. Furthermore enzymatic polyadenylation results in RNA molecules having different poly(A) tails. In this context, the method disclosed herein allows determination of the different poly(A) tails comprised in the RNA molecules of an RNA population.

The 3'-UTR preferably comprises at least one selected from a histone stem-loop sequence and a homopolymeric sequence, preferably a poly(A) sequence or a poly(C) sequence, more preferably as described herein. Further preferably, the cleavage site for the catalytic nucleic acid molecule is located 5' of the homopolymeric sequence or 5' of the histone stem-loop sequence. Alternatively, the cleavage site for the catalytic nucleic acid molecule is located in the homopolymeric sequence or in the histone stem-loop sequence.

Preferably, the RNA having at least one cleavage site for at least one catalytic nucleic acid molecule comprises a homopolymeric sequence in the 3'-UTR, preferably a poly(A) sequence or a poly(C) sequence, wherein the cleavage site of the catalytic nucleic acid is located 5' of the homopolymeric sequence, preferably within the 3'-UTR. Accordingly, the 3' terminal RNA fragment is preferably separated, preferably as described herein, and analyzed.

The method disclosed herein thus preferably comprises determining a structural feature, in particular the number of nucleotides, comprised in a 3'-terminal fragment, preferably in a homopolymeric sequence in the 3'-UTR of an mRNA.

The method disclosed herein preferably comprises determining an additional structural element at the 3' terminus of the 3' terminal RNA fragment or at the 3' terminus of the RNA molecule having at least one cleavage site for at least one catalytic nucleic acid molecule. The method preferably comprises determining the presence of one or more additional nucleotides at the 3' terminus of the 3' terminal RNA fragment. In this context, additional nucleotide' refers to a (non-templated) nucleotide, which is present in the RNA molecule to be analyzed, while it is absent from the template, for example a DNA template used in *in vitro* transcription. Such additional nucleotides may be added to the 3' terminus of the RNA molecule to be analyzed, for example, post transcriptionally or during *in vitro* transcription. For instance, nucleotides may be added to the 3' terminus in an enzymatic reaction, such as, e.g. in an enzymatic polyadenylation reaction.

The method disclosed herein preferably comprises determining the number of adenosine nucleotides that have been added to the 3' terminus of an RNA molecule having at least one cleavage site for at least one catalytic nucleic acid molecule. Preferably, the length and/or the mass of the separated 3' terminal RNA fragment after enzymatic polyadenylation of the RNA is compared to the length and/or mass of the corresponding nucleic acid sequence in the template, which is used for synthesizing said RNA.

In analogous manner, it is preferably determined by the method disclosed herein, whether the 3' terminal RNA fragment is identical to the respective template that is used for RNA synthesis or whether additional nucleotides are present in the 3' terminal RNA fragment.

The method disclosed herein preferably comprises determining whether non-templated nucleotides are present in the 3' terminal RNA fragment, preferably at the 3' terminus of the 3' terminal RNA fragment. In a preferred embodiment, the method disclosed herein comprises determining the presence of additional (non-templated) nucleotides at the 3' terminus of the 3' terminal RNA fragment, which were (erroneously) added during synthesis, preferably by terminal transferase activity of an RNA polymerase during *in vitro* transcription.

According to another aspect, the method disclosed herein is used for characterizing a population of RNA molecules, as defined by the attached claims. Preferably, the method is for analyzing a modified RNA molecule as defined herein. Specifically, method method is provided for determining the presence and/or integrity of a homopolymeric sequence in the 3'-UTR of a population of mRNA molecules, wherein the population comprises at least one mRNA molecule comprising a 3'-UTR, which comprises at least one homopolymeric sequence, wherein the mRNA molecules are derived from in vitro transcription reactions and wherein the mRNA molecule has at least one cleavage site for at least one catalytic nucleic acid molecule, wherein at least one cleavage site is located 5' of the homopolymeric sequence, the method comprising the steps of:
a) providing the sample containing the population of mRNA molecules, wherein the at least one mRNA molecule comprising a 3'-UTR, which comprises at least one homopolymeric sequence, has at least one cleavage site for at least one catalytic nucleic acid molecule, wherein at least one cleavage site is located 5' of the homopolymeric sequence in the 3'-UTR of the mRNA molecule,
b) cleaving the at least one mRNA molecule having at least one cleavage site for at least one catalytic nucleic acid molecule, wherein at least one cleavage site is located 5' of the homopolymeric sequence, with at least one catalytic nucleic acid molecule into a 3' terminal RNA fragment, a 5' RNA fragment and optionally into at least one central RNA fragment by contacting the sample with at least one catalytic nucleic acid molecule under conditions allowing the cleavage of the mRNA molecule,
c) determining the presence and/or the integrity of the homopolymeric sequence in the 3'-UTR of the at least one mRNA molecule having at least one cleavage site for at least one catalytic nucleic acid molecule by analyzing the 3' terminal RNA fragment, and optionally the at least one optional central RNA fragment obtained in step b), wherein step c) comprises separating the 3' terminal RNA fragment by denaturing gel electrophoresis or liquid chromatography, determining the mass and/or the length of the 3' terminal RNA fragment, comparing the determined mass and/or length of the 3' terminal RNA fragment with a respective mass and/or length of a reference RNA fragment and
d) optionally determining the relative amount of different mRNA molecules in the population.

In further non-claimed embodiments, the present disclosure also provides a method for analyzing a population of RNA molecules, wherein the population comprises at least one RNA molecule that has at least one cleavage site for at least one catalytic nucleic acid molecule, the method comprising the steps of:
a) providing a sample containing the population of RNA molecules,
b) cleaving the at least one RNA molecule having at least one cleavage site for at least one catalytic nucleic acid molecule with at least one catalytic nucleic acid molecule into a 3' terminal RNA fragment, a 5' RNA fragment and optionally into at least one central RNA fragment by contacting the sample with at least one catalytic nucleic acid molecule under conditions allowing the cleavage of the RNA molecule,
c) determining a physical property of the at least one RNA molecule having at least one cleavage site for at least one catalytic nucleic acid molecule by analyzing the 3' terminal RNA fragment, the 5' terminal RNA fragment and/or the at least one optional central RNA fragment obtained in step b), and
d) optionally determining the relative amount of different RNA molecules in the population.

While steps a), b) and c) are typically as defined for the method for analyzing an RNA molecule having at least one cleavage site for at least one catalytic nucleic acid molecule, step d) of the method for analyzing a population of RNA molecules is specific for the latter. Hence, all the features described above for steps a), b) and c) apply in analogous manner to the method for analyzing an RNA population. The method for analyzing an RNA population, however, additionally comprises the optional step d), which comprises determining the relative amount of different RNA molecules in the population by measuring the relative amount of the different 3' terminal RNA fragments, the different 5' terminal fragments and/or of the different central RNA fragment corresponding to the same part of the RNA molecule to be analysed.

As used herein, the population of RNA molecules typically comprises at least one RNA molecule, preferably a modified RNA molecule, having at least one cleavage site for at least one catalytic nucleic acid molecule, wherein the at least one RNA molecule is characterized by a distinct physical property or a distinct structural feature, which may preferably be determined by analyzing the RNA fragment(s) obtained in step b) of the method for analyzing the RNA population. Preferably, a population of RNA molecules comprises at least one first RNA molecule having at least one cleavage site for at least one catalytic nucleic acid molecule, and further comprises at least one second RNA molecule having at least one cleavage site for at least one catalytic nucleic acid molecule, wherein the first RNA molecule and the second RNA molecule differ in a physical property or a structural feature that may be determined by analyzing the respective RNA fragments. By measuring the relative amounts of those different RNA fragments corresponding to the same part of the RNA molecule to be analyzed, the relative amounts of the different RNA molecules in the population of RNA molecules are determined. Therein, the relative amounts of the RNA fragments are measured by using any suitable technique for nucleic acid molecule quantitation, preferably by using the techniques described herein. The amounts of the RNA fragments are preferably measured in step c) by spectroscopic methods, quantitative mass spectrometry, or sequencing. Step d) preferably comprises calculating the ratio of the amount of an RNA molecule with a distinct physical property to the amount of another RNA molecule in the population or to the total amount of RNA molecules in the population.

In the method for analyzing an RNA population, the population preferably comprises at least one RNA molecule having at least one cleavage site for at least one catalytic nucleic acid molecule, wherein the mass and/or the length of the corresponding RNA fragment resulting from the cleavage with the at least one catalytic nucleic acid molecule is equal to the respective mass and/or length of a reference RNA fragment or of the corresponding nucleic acid sequence in the template that was used for synthesis of said RNA molecule. In other words, the population preferably comprises at least one RNA molecule, wherein the RNA fragment resulting from the cleavage with the at least one catalytic nucleic acid molecule is identical to the respective reference RNA fragment or to the respective nucleic acid sequence in the template. Therein, step d) preferably comprises determining the relative amount of RNA molecules in the population, wherein the RNA fragment resulting from the cleavage with the at least one catalytic nucleic acid molecule is identical to the respective reference RNA fragment or to the respective nucleic acid sequence in the template, preferably by measuring the total amount of RNA fragments resulting from the cleavage with the at least one catalytic nucleic acid molecule and the amount of RNA fragments that are identical to the respective reference RNA fragment or to the respective nucleic acid sequence in the template.

The present disclosure concerns a method, wherein the population preferably comprises at least two different RNA molecules having at least one cleavage site for at least one catalytic nucleic acid molecule, wherein the at least two different RNA molecules having at least one cleavage site for at least one catalytic nucleic acid molecule have different lengths, and wherein step c) comprises separating the RNA fragments resulting from the cleavage with the at least one catalytic nucleic acid molecule depending on their respective lengths. In this context it is particularly preferred that the difference in length of the at least two different RNA molecules arise from a difference in length in the part of the RNA molecules which correspond to the RNA fragments which were analyzed after cleavage with the at least one catalytic nucleic acid molecule.

Preferably, the difference between the length of the at least two different RNA fragments separated in step c) is 75 nucleotides or less. More preferably, the at least two different RNA fragments separated in step c) differ in length by at least 40 nucleotides, preferably by at least 20 nucleotides, more preferably by at least 15 nucleotides or even more preferably by at least 10 nucleotides, wherein the RNA fragments to be analyzed preferably have a size in a range from 1 to 300 nucleotides, more preferably from 10 to 150 nucleotides or even more preferably from 50 to 150 or from 40 to 100 nucleotides. Preferably, RNA fragments are distinguished that differ by at least 5, 4, 3, 2 or 1 nucleotide, wherein the RNA fragments to be analysed preferably have a size in a range from 1 to 75 nucleotides, more preferably from 1 to 50 nucleotides or even more preferably from 5 to 50 or from 5 to 30 nucleotides.

Preferably, step c) comprises a chromatography technique, more preferably a liquid chromatography technique as described herein or most preferably a HPLC technique. In a particularly preferred embodiment, step c) comprises a chromatography technique and a spectrometry technique, such as a mass spectrometry technique.

Step d) of the method disclosed herein preferably comprises calculating the ratio of the amount of a first RNA molecule having at least one cleavage site for at least one catalytic nucleic acid molecule and having a distinct length to the amount of a second RNA molecule having at least one cleavage site for at least one catalytic nucleic acid molecule and having a length that differs from the length of the first RNA molecule. Preferably, the at least one RNA molecule having at least one cleavage site for at least one catalytic nucleic acid molecule is produced by *in vitro* transcription and step d) comprises calculating the ratio of the amount of RNA molecules having at least one cleavage site for at least one catalytic nucleic acid molecule and having the length of a reference RNA or of the corresponding nucleic acid sequence in the nucleic acid molecule used as template in *in vitro* transcription to the amount of RNA molecules having at least one cleavage site for at least one catalytic nucleic acid molecule and having a length that differs from the length of a reference RNA or of the corresponding nucleic acid sequence in the nucleic acid molecule used as template in *in vitro* transcription.

The method disclosed herein is preferably used as a quality control, preferably in the production of RNA for diagnostic or therapeutic applications. In particular, the method disclosed herein is used for controlling the quality of an RNA molecule or RNA population obtained by chemical synthesis or by *in vitro* transcription. Furthermore the method disclosed herein may be used as quality control in the production of modified RNA after chemical synthesis or *in vitro* transcription e.g. in the production of enzymatic capped RNA or enzymatic polyadenylated RNA.

In a further aspect, the invention concerns the use of a catalytic nucleic acid molecule in a method as defined in the attached claims.

In further non-claimed embodiments, the present disclosure also concerns the use of a catalytic nucleic acid molecule in a method for analyzing an RNA molecule having at least one cleavage site for at least one catalytic nucleic acid molecule, wherein the catalytic nucleic acid molecule is used to cleave the RNA molecule into a 3' terminal RNA fragment, a 5' RNA fragment, and optionally in at least one central RNA fragment, and wherein the 3' terminal RNA fragment, the 5' terminal fragment and/or the optional at least one central RNA fragment is analyzed. The features and descriptions provided above with respect to the methods disclosed herein likewise apply to the use disclosed herein. Preferably, the use disclosed herein comprises an analysis of the 3' terminal RNA fragment, the 5' terminal fragment and/or the optional at least one central RNA fragment, preferably of the 3' terminal fragment and/or the optional at least one central RNA fragment which comprises determining a physical property or a structural feature of the 3' terminal RNA fragment, the 5' terminal fragment and/or the optional at least one central RNA fragment preferably determining the mass and/or the length of the 3' terminal RNA fragment, the 5' terminal fragment and/or the optional at least one central RNA fragment.

In this context, the catalytic nucleic acid molecules used for analyzing an RNA molecule having at least one cleavage site for at least one catalytic nucleic acid molecule may be used in the quality control of the production process of RNA molecules, preferably under GMP conditions, more preferably in the production process of RNA molecules that involves *in vitro* transcription.

### Brief Description of the Figures

The figures shown in the following are merely illustrative and shall describe the present invention as defined by the appended claims in a further way.

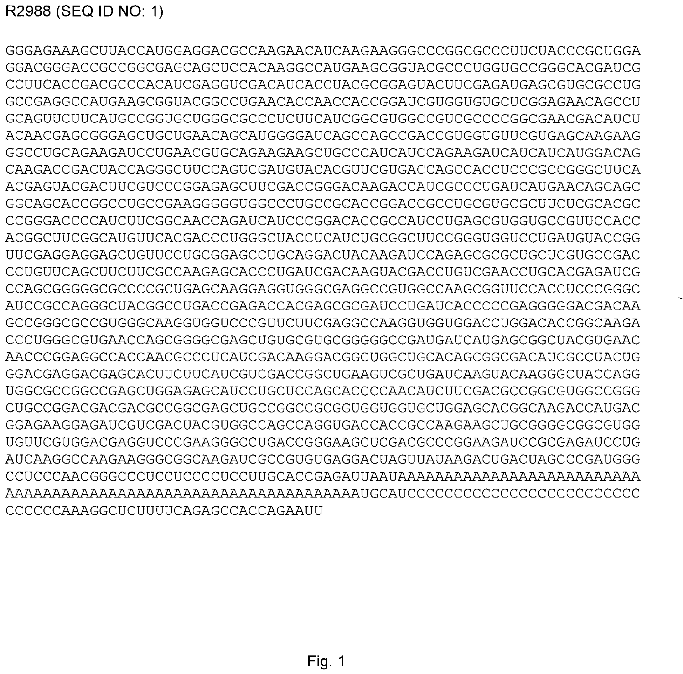

- **Figure 1:**: G/C optimized mRNA sequence encoding Photinus pyralis luciferase (PpLuc) (R2988, SEQ ID NO: 1).

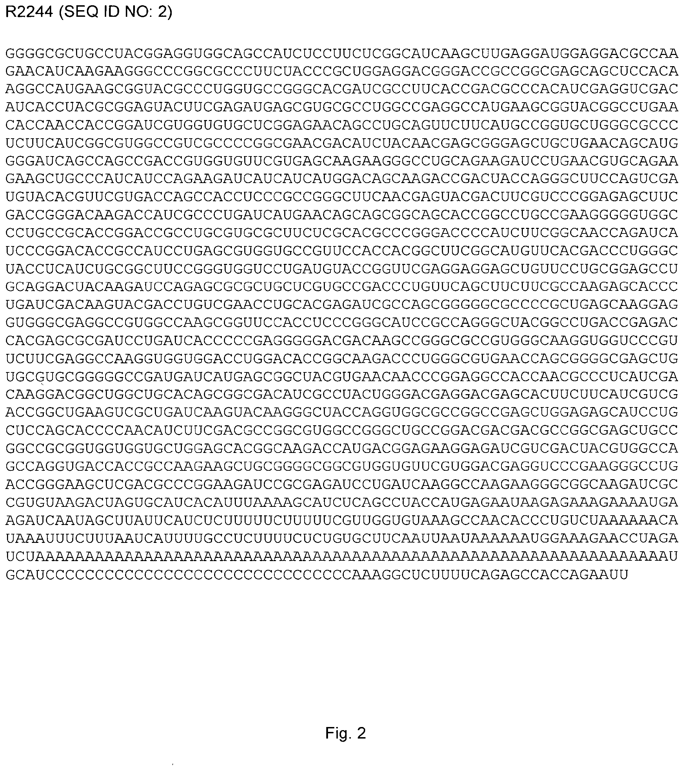

- **Figure 2:**: G/C optimized mRNA sequence encoding Photinus pyralis luciferase (PpLuc) (R2244, SEQ ID NO: 2).

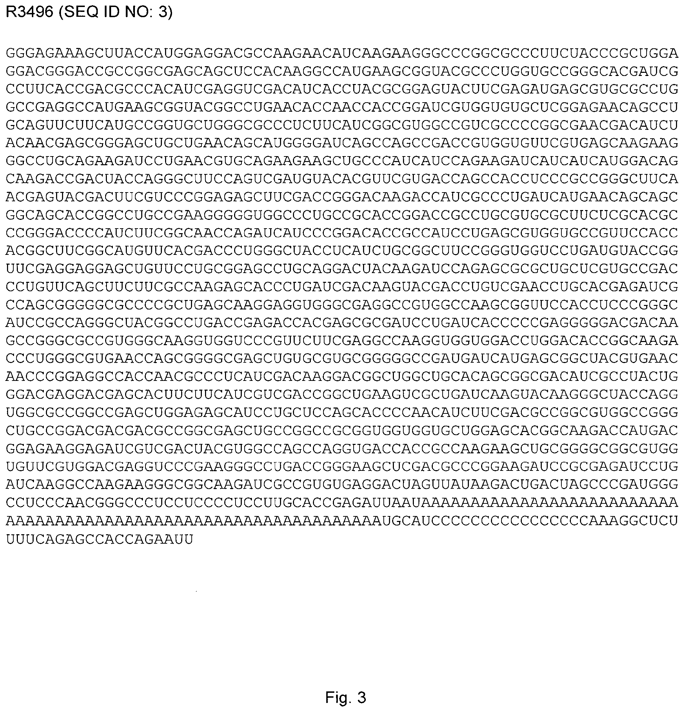

- **Figure 3:**: G/C optimized mRNA sequence encoding Photinus pyralis luciferase (PpLuc) (R3496, SEQ ID NO: 3).

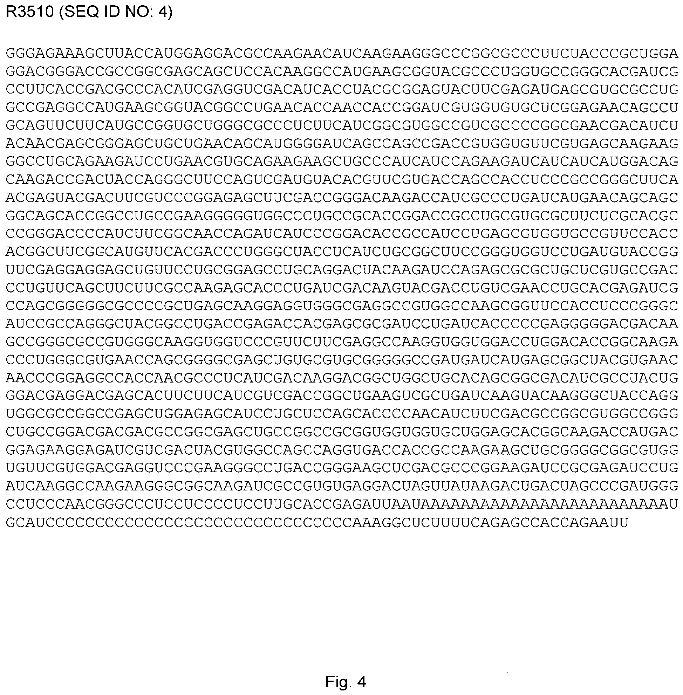

- **Figure 4:**: G/C optimized mRNA sequence encoding Photinus pyralis luciferase (PpLuc) (R3510, SEQ ID NO: 4).

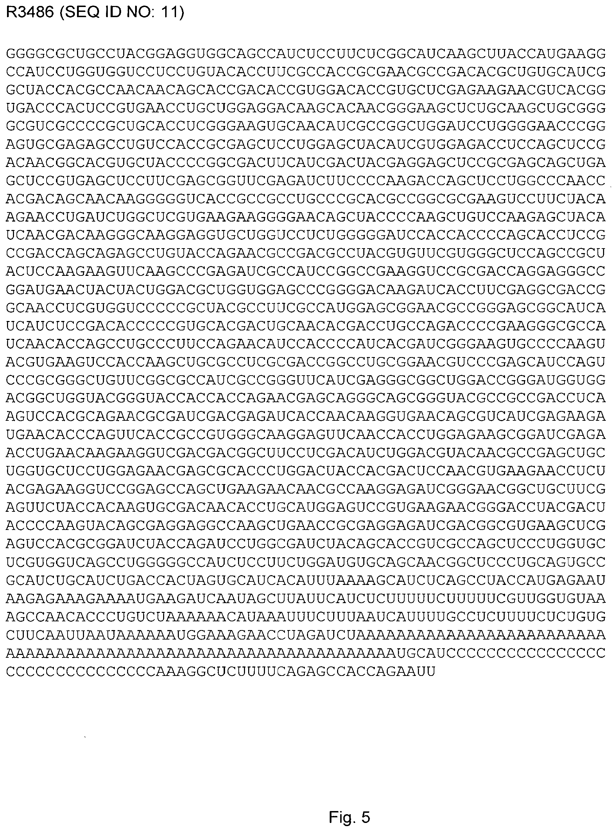

- **Figure 5:**: G/C optimized mRNA sequence encoding Hemagglutinin from Influenza virus H1N1(Netherlands2009). (R3486, SEQ ID NO: 11)
- **Figure 6:**: Diagram of hammerhead ribozyme annealed to target RNA sequence (highlighted in bold).
- **Figure 7:**: Acrylamide gel analysis of RNA digested with ribozyme (Example 3).
- **Figure 8:**: HPLC analysis of 3' terminal fragments obtained by incubating RNA R2988 (SEQ ID NO: 1) with ribozyme 3HHR1871_5A. The expected 127 nt-fragment is represented by the corresponding peak in the bottom panel.
- **Figure 9:**: HPLC analysis of 3' terminal fragments obtained by incubating RNA R3496 (SEQ ID NO: 3) with ribozyme 3HHR1871_5A. The expected 112 nt-fragment is represented by the corresponding peak in the bottom panel.
- **Figure 10:**: HPLC analysis of 3' terminal fragments obtained by incubating RNA R3510 (SEQ ID NO: 4) with ribozyme 3HHR1871_5A. The expected 88 nt-fragment is represented by the corresponding peak in the bottom panel.
- **Figure 11:**: Comparison of 3' terminal fragments obtained by incubating RNA R2988 (SEQ ID NO: 1), RNA R3496 (SEQ ID NO: 3) or RNA R3510 (SEQ ID NO: 4), respectively, with ribozyme 3HHR1871_5A. The alignment of the respective panels shows that not only the lack of 39 adenosine nucleotides (see bottom panel vs. top panel), but also the lack of 15 cytosine nucleotides (see middle panel vs. top panel) in the obtained 3'-terminal fragment can be determined by the assay.
- **Figure 12:**: HPLC analysis of 3' terminal fragments obtained by incubating RNA R2244 (SEQ ID NO: 2) with ribozyme 3HH2989_5A.

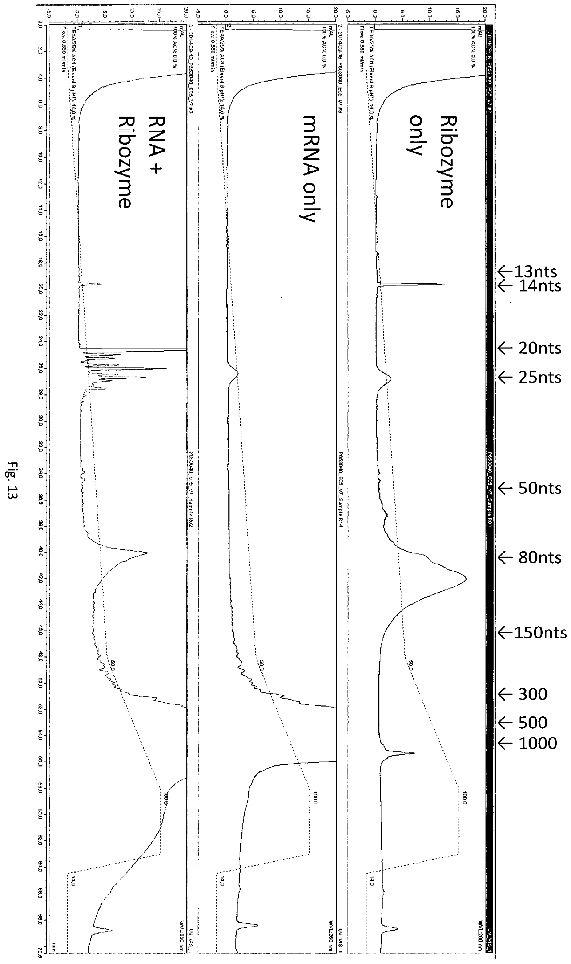

- **Figure 13:**: HPLC analysis of 3' terminal fragments obtained by incubating RNA R2244 (SEQ ID NO: 2) with ribozyme 3HH_3C_02. In the bottom panel, the peak corresponding to the expected 20 nt 3'-terminal fragment is separated from further peaks corresponding to further 3'-terminal fragments, which are slightly longer (about 25 to 30 nt).

### Examples

The Examples shown in the following are merely illustrative and shall describe the present invention as defined by the appended claims.

### Example 1: Preparation of hammerhead ribozymes

The ribozymes used in the experiments were synthesized and PAGE purified by Biomers.net GmbH (Ulm, Germany).

**Table 1: Examplary ribozymes**

| **Name** | **Sequence** | **SEQ ID NO** | **Description** |
|---|---|---|---|
| 3HH1871_5A | 5'-uuuuuuuuuuuuuuuuu uucugaugaggccucgacc gauaggucgaggccgaaau uaaucucggugcaaggagg ggagga-3' | 5 | Hammerhead ribozyme designed for cleavage 5' of the polyA sequence in e.g. R2988 (SEQ ID NO: 1), R3496 (SEQ ID NO: 3), R3510 (SEQ ID NO: 4) |
| 3HH2989_5A | 5'-uuuuuuuuuuuuuuuu uucugaugaggccucgacc gauaggucgaggccgaaag | 6 | Hammerhead ribozyme designed for cleavage 5' of the polyA |
| | aucuagguucuuuccauuu uuuauu-3' | | sequence in R2244 (SEQ ID NO: 2) and R3486 (SEQ ID NO: 11) |
| 3HH_3A_5C_01 | 5'-ggggggggggggggggcu gaugaggccucgaccgaua ggucgaggccgaaaugcau uuuuuuuuuuuuuuuuuuu uu-3' | 7 | Hammerhead ribozyme designed for cleavage 3' of polyA in all listed RNAs (SEQ ID NO: 1-4 and 11)(e.g. nucleotide position 1812 in R2988 (SEQ ID NO: 1)) |
| 3HH_3A_5C_02 | 5'-ggggggcugaugaggc cucgaccgauaggucgagg ccgaaaugcaUUUUUU UUUUUUUUUUUUUU U-3' | 8 | Hammerhead ribozyme designed for cleavage 3' of polyA in all listed RNAs (SEQ ID NO: 1-4 and 11) (e.g. nucleotide position 1812 in R2988 (SEQ ID NO: 1)) |
| 3HH_3C_01 | 5'-aauucugguggcucuga aaacugaugaggccucgac cgauaggucgaggccgaaa gccuuuggggggggggggg gggg-3' | 9 | Hammerhead ribozyme designed for cleavage within the stem-loop region of all listed RNAs (SEQ ID NO: 1-4 and 11) (e.g.nucleotide position1850 in R2988 (SEQ ID NO: 1)) |
| 3HH_3C_02 | 5'-aauucugguggcucuga aaacugaugaggccucgac cgauaggucgaggccgaaa gccuuuggggggg-3' | 10 | Hammerhead ribozyme designed for cleavage within the stem-loop region of all listed RNAs (SEQ ID NO: 1-4 and 11) (e.g.nucleotide position 1850 in R2988 (SEQ ID NO: 1)) |

Potential hammerhead ribozymes towards each cleavage site, respective helix lengths and fragment sizes are depicted in Table 2.

**Table 2: Position and sequence of potential hammerhead target sites (NUH) in 3'-UTR of RNAs, e.g. R2988, R2244, R3510, R3496, R3486**

| Name | NUH sequence | Length Helix I/III | Cleavage product sizes (if single cleavage reaction) |
|---|---|---|---|
| 3HH1871_5A | AUA | 19/27 nt | 127 nt |
| 3HH2989_5A | CUA | 19/27 nt | 127 nt |
| 3HH_3A_5C_01 | AUC | 15/27 nt | 58 nt |
| 3HH_3A_5C_02 | AUC | 7/27 nt | 58 nt |
| 3HH_3C_01 | CUC | 20/24 nt | 20 nt |
| 3HH_3C_02 | CUC | 20/14 nt | 20 nt |

### Example 2: Preparation of mRNA

### 1. Preparation of DNA and mRNA constructs

Four different DNA sequences, each encoding a Photinus pyralis luciferase (PpLuc) mRNA (R2988, R3496, R3510, R2244), were prepared and used for subsequent in vitro transcription reactions. One further DNA sequence (R3486) was prepared, encoding Hemagglutinin from Influenza virus H1N1(Netherlands2009).

The open reading frames (ORF) of each of the DNA constructs were modified with respect to the wild type coding sequence by introducing a GC-optimized sequence for stabilization.

The RNAs encoded by the DNA constructs comprised one the following combinations of features:
5'-CAP - GC-optimized ORF - globin-3'-UTR - poly(A) sequence - poly(C) sequence - histone stem-loop sequence; (R2988 (SEQ ID NO: 1); R3496 (SEQ ID NO: 3); R3510 (SEQ ID NO: 4)) or
5'-CAP - 32L-5'-UTR - GC-optimized ORF - albumin-3'-UTR - poly(A) sequence - poly(C) sequence - histone stem-loop sequence (R2244 (SEQ ID NO: 2) and R3486 (SEQ ID NO: 11)).

### 2. In vitro transcription

The respective DNA plasmids prepared according to paragraph 1 were transcribed in vitro using T7 polymerase. Subsequently, the mRNA was purified using PureMessenger^{®} (CureVac, Tubingen, Germany; WO2008/077592A1).

Linearized DNA plasmid templates (50 µg/ml) were transcribed at 37°C for 3-5 hours in 80 mM HEPES/KOH, pH 7.5, 24 mM MgCl₂, 2 mM spermidine, 40 mM DTT, 5 U/ml pyrophosphatase (Thermo Fisher Scientific), 200 U/ml Ribolock RNase inhibitor (Thermo Fisher Scientific), 5000 U/ml T7 RNA polymerase (Thermo Fisher Scientific). Nucleotide triphosphates were added according to section 3 below. Following transcription, DNA templates were removed by DNasel (Roche) (100 U/ml, 1 mM CaCl₂, 1 hour at 37°C). RNAs were precipitated in 2.86 M LiCI for 16 hours at -20°C, followed by centrifugation (30 min, 16.000 g, 4°C). Pellets were washed in 0.1 transcription reaction volumes of 75% ethanol (invert, centrifuge 5 min, 16.000 g, 4°C), dried and re-dissolved in 10 transcription reaction volumes H₂O.

### 3. In vitro transcription in the presence of CAP analog

For the production of 5'-capped RNAs using CAP analog, transcription was carried out in 5.8 mM m7G(5')ppp(5')G Cap Analog, 4 mM ATP, 4 mM CTP, 4 mM UTP, and 1.45 mM GTP (all Thermo Fisher Scientific).

### Example 3: Assay for analysis of RNA 3'-terminus

### 1. Principle of the assay

The hammerhead ribozymes of Example 1 were incubated with the in vitro transcribed RNAs of Example 2 and the cleavage products were separated (e.g. by polyacrylamidegel-electrophoresis (PAGE) or chromatographic methods).

### 2. Ribozyme cleavage reaction

Reaction scales for gel analysis were usually 1x (10 pmol RNA). For HPLC analysis, 15x reactions (150 pmol RNA) were set up, allowing a more sensitive detection and thus a more precise determination of the respective mRNA populations. Per reaction, 10 pmol of ribozyme and 10 pmol of the respective RNA were annealed in 0.625 mM EDTA in a total volume of 7.5 µl (3 min at 95°C, 0.1 °C/sec to 25°C, 10 min at 25°C). After addition of 2.5 µl of 160 mM MgCl₂, 200 mM Tris/HCl, pH 7.5 (final concentration 40 mM MgCl₂, 50 mM Tris/HCl), the reaction was incubated at 25°C for 1 hour.

For analysis via PAGE, the 1x reaction was stopped with 30 µl 95% formamide, 20 mM EDTA. For HPLC analysis, the 15x reaction was stopped with 450 µl 20 mM EDTA (final concentration 15 mM).

### 3. Gel separation and analysis of cleavage products

Stopped reactions were heat-denatured (heated to 80°C for 2 min, immediately put on ice for 5 min) and separated on a 10 cm x 8 cm x 1.5 mm 20% denaturing PAGE (8 M urea (Appli-Chem), 20% acrylamid:bisacrylamid 19:1 (AppliChem), 1x TBE, 0.05% APS (AppliChem), 0.05% TEMED (AppliChem); 180 V, 2 hours, Mini-PROTEAN^{®} Tetra Cell (BioRad)). Gels were stained for 10 minutes in 1:10,000 SYBR Gold (Invitrogen) in TBE and documented on an E-BOX VX2 gel documentation system with 312 nm-UV Transilluminator (Peqlab) (excitation maximum for SYBR Gold: -300 nm, emission: ~537 nm).

To determine the size of the RNA fragments, cleavage products were analysed using Quantity One 1-D Analysis Software (BioRad) and compared to a reference RNA of known size.

### 4. HPLC separation, quantification of cleavage products and calculation of ratio

Analysis was performed via ion-pair, reversed-phase chromatography on a Dionex Parallel-HPLC U3000 CV-P-1247, equipped with analytical pump (DPG-3600SD), column oven (TCC-3000SD) and UV/Vis-4-channel-detectors (2x VWD-3400RS) with analytical SST measuring cell (11µL, 10 mm, for VWD-3x00 detector). An AQUITY UPLC OST C18 column (2.1 × 50mm, 1.7 µm particle size, Waters) was used. Column temperature was set to 60°C. Buffer A contained 0.1 M triethylammonium acetate (TEAA), pH 6.8, buffer B 0.1 M TEAA, pH 7.3, 25% acetoni-trile. The column was equilibrated with 14% buffer B.

For sample preparation, HPLC equilibration buffer (86% buffer A, 14% buffer B) was added to the stopped hammerhead ribozyme reactions to obtain a final volume of 1700 µl.

1650 µl of the RNA solution were loaded using a SEMIPREP-Autosampler (WPS-3000SL, Dionex) and run with a stepped gradient beginning with 14% buffer B for 3 minutes, increasing to 50% buffer B over 45 minutes, then increased to 100% B over 10 minutes, held for 5 minutes, then decreased to 14% buffer B over 1.5 minutes.

Signal integration was done using Chromeleon software 6.80 SR11 Build 3161 (Dionex), and the size of the RNA fragments was determined by comparing the retention time with a known control of the correct length.

### 5. Results

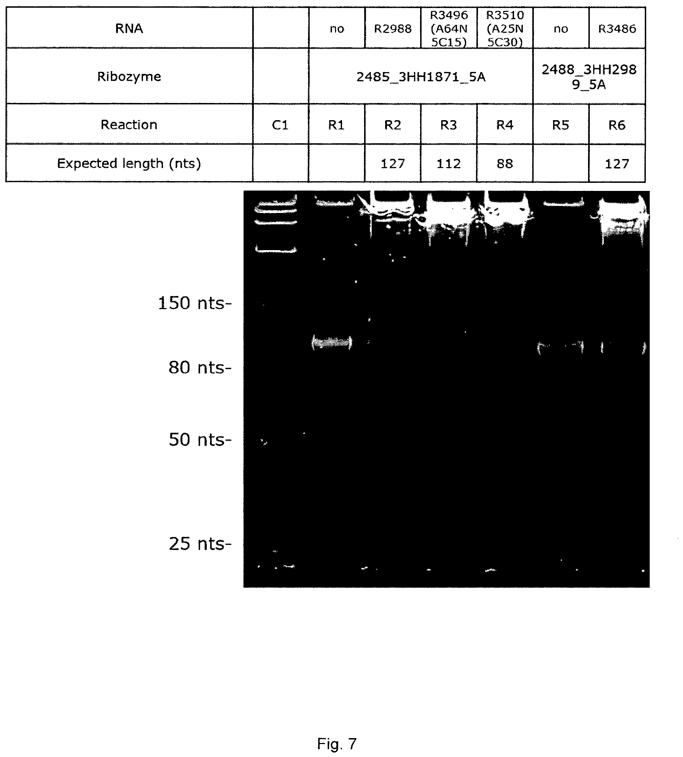

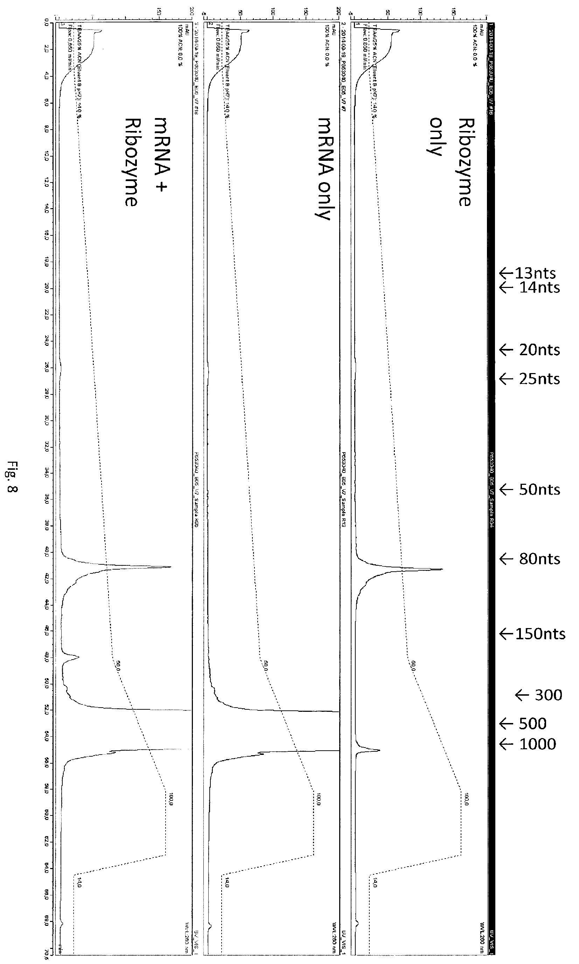

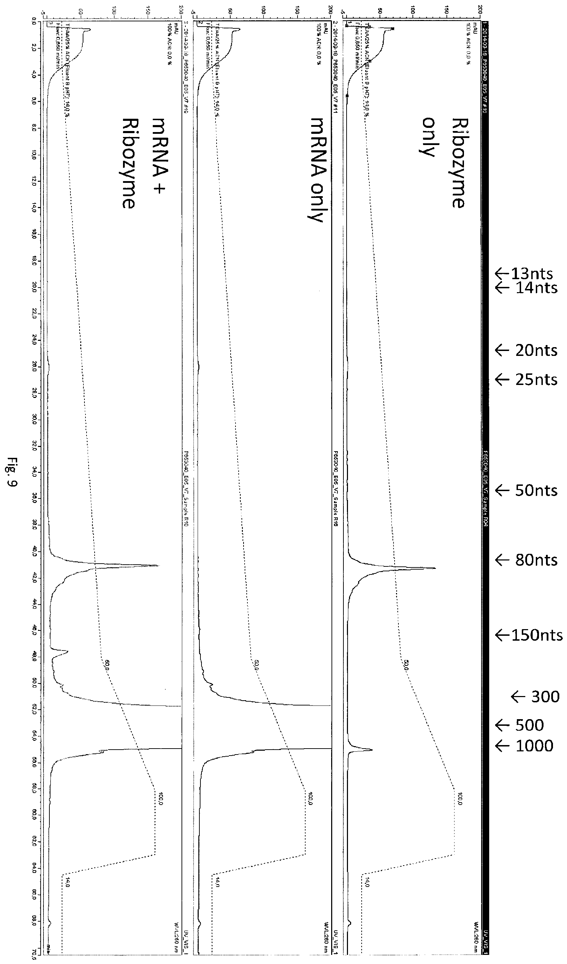

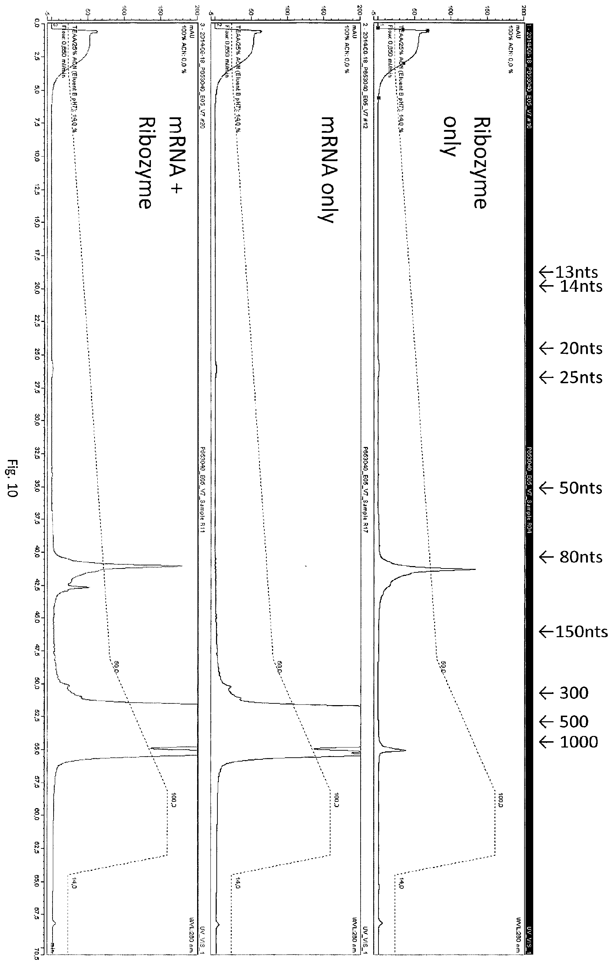

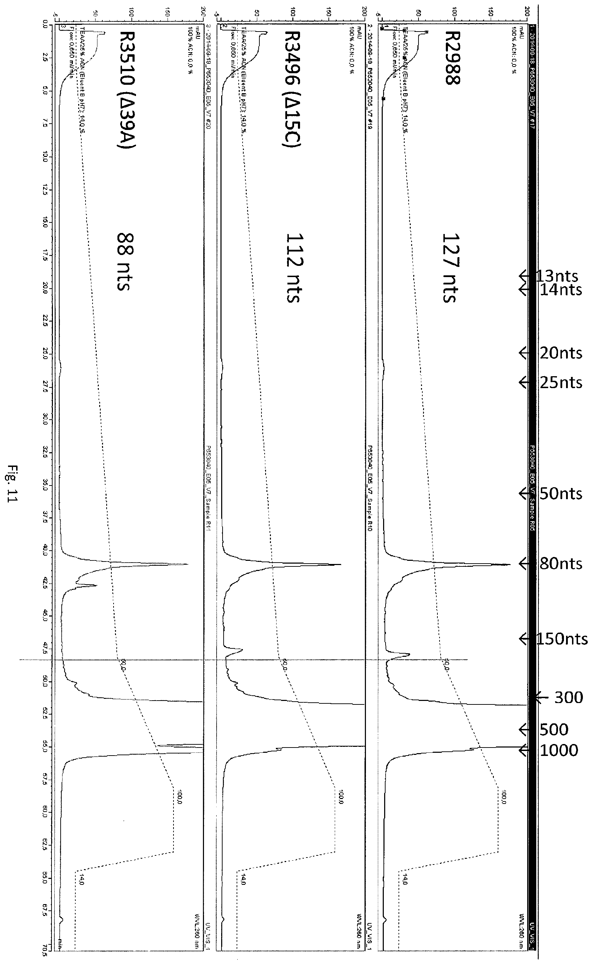

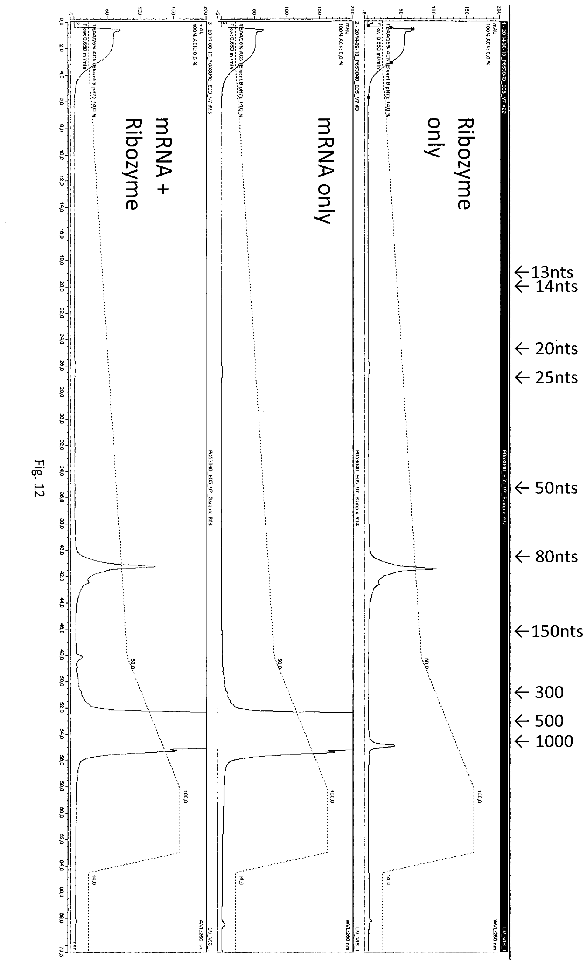

As can be seen in Figures 7 to 12, fragments produced by ribozyme cleavage of long mRNA molecules can be resolved by HPLC.

## Claims

1. A method for determining the presence and/or integrity of a homopolymeric sequence in the 3'-UTR of an mRNA molecule, the mRNA molecule having at least one cleavage site for at least one catalytic nucleic acid molecule, wherein the mRNA molecule is derived from an in vitro transcription reaction and wherein at least one cleavage site is located 5' of the homopolymeric sequence, the method comprising the steps of:
a) providing the mRNA molecule having at least one cleavage site for at least one catalytic nucleic acid molecule, wherein at least one cleavage site is located 5' of the homopolymeric sequence in the 3'-UTR of the mRNA molecule,
b) cleaving the mRNA molecule with the at least one catalytic nucleic acid molecule into a 5' terminal RNA fragment, a 3' terminal RNA fragment and optionally into at least one central RNA fragment by contacting the mRNA molecule with the at least one catalytic nucleic acid molecule under conditions allowing the cleavage of the mRNA molecule, and
c) determining the presence and/or the integrity of the homopolymeric sequence in the 3'-UTR of the mRNA molecule by analyzing the 3' terminal RNA fragment and optionally the at least one optional central RNA fragment obtained in step b), wherein step c) comprises separating the 3' terminal RNA fragment by denaturing gel electrophoresis or liquid chromatography, determining the mass and/or the length of the 3' terminal RNA fragment and comparing the determined mass and/or length of the 3' terminal RNA fragment with a respective mass and/or length of a reference RNA fragment.

2. A method for determining the presence and/or integrity of a homopolymeric sequence in the 3'-UTR of a population of mRNA molecules, wherein the population comprises at least one mRNA molecule comprising a 3'-UTR, which comprises at least one homopolymeric sequence, wherein the mRNA molecules are derived from in vitro transcription reactions and wherein the mRNA molecule has at least one cleavage site for at least one catalytic nucleic acid molecule, wherein at least one cleavage site is located 5' of the homopolymeric sequence, the method comprising the steps of:
a) providing the sample containing the population of mRNA molecules, wherein the at least one mRNA molecule comprising a 3'-UTR, which comprises at least one homopolymeric sequence, has at least one cleavage site for at least one catalytic nucleic acid molecule, wherein at least one cleavage site is located 5' of the homopolymeric sequence in the 3'-UTR of the mRNA molecule,
b) cleaving the at least one mRNA molecule having at least one cleavage site for at least one catalytic nucleic acid molecule, wherein at least one cleavage site is located 5' of the homopolymeric sequence, with at least one catalytic nucleic acid molecule into a 3' terminal RNA fragment, a 5' RNA fragment and optionally into at least one central RNA fragment by contacting the sample with at least one catalytic nucleic acid molecule under conditions allowing the cleavage of the mRNA molecule,
c) determining the presence and/or the integrity of the homopolymeric sequence in the 3'-UTR of the at least one mRNA molecule having at least one cleavage site for at least one catalytic nucleic acid molecule by analyzing the 3' terminal RNA fragment, and optionally the at least one optional central RNA fragment obtained in step b), wherein step c) comprises separating the 3' terminal RNA fragment by denaturing gel electrophoresis or liquid chromatography, determining the mass and/or the length of the 3' terminal RNA fragment, comparing the determined mass and/or length of the 3' terminal RNA fragment with a respective mass and/or length of a reference RNA fragment and
d) optionally determining the relative amount of different mRNA molecules in the population.

3. The method according to claim 1 or 2, wherein the catalytic nucleic acid molecule has been designed to be able to cleave the mRNA molecule at at least one specific cleavage site.

4. The method according to any one of claims 1 to 3, wherein the mRNA molecule having at least one cleavage site for at least one catalytic nucleic acid molecule has been designed to have at least one cleavage site for at least one catalytic nucleic acid molecule.

5. The method according to any one of claims 1 to 4, wherein at least one cleavage site of the catalytic nucleic acid molecule is located within 250 nucleotides from the 3' terminus of the mRNA molecule.

6. The method according to any one of claims 1 to 5, wherein the at least one catalytic nucleic acid molecule is a ribozyme, preferably selected from the group consisting of hammerhead ribozymes, hairpin ribozymes, and HDV ribozymes.

7. The method according to any one of claims 1 to 6, wherein the at least one catalytic nucleic acid molecule is provided in step b) in *trans.*

8. The method according to any one of claims 1 to 7, wherein step b) comprises denaturation of the mRNA molecule having at least one cleavage site for at least one catalytic nucleic acid molecule and annealing of the at least one catalytic nucleic acid molecule to said mRNA molecule.

9. The method according to any one of claims 1 to 8, wherein step c) comprises separating the RNA fragments by HPLC, FPLC or RPLC,
and/or
wherein step c) comprises separating the optional central RNA fragments and wherein the optional central RNA fragments are separated by denaturing gel electrophoresis or liquid chromatography, preferably HPLC, FPLC or RPLC, and/or
wherein step c) comprises analysis of a structural feature or of a physical parameter of the at least one optional central RNA fragment,
and/or
wherein step c) comprises comparison of a structural feature or of a physical parameter of the at least one optional central RNA fragment, with a respective feature or parameter of a reference RNA fragment,
and/or
wherein step c) comprises determining the identity and/or the integrity of the 3' terminal RNA fragment and/or of the at least one optional central RNA fragment, and/or
wherein step c) comprises determining the mass and/or the length of the at least one optional central RNA fragment, wherein the length of the at least one optional central RNA fragment is preferably 250 nucleotides or less,
and/or
wherein step c) involves spectroscopic analysis, quantitative mass spectrometry, or sequence analysis.

10. The method according to any one of claims 1 to 9, wherein the *in vitro* transcription is carried out by using a bacteriophage RNA polymerase, wherein the bacteriophage RNA polymerase is preferably selected from the group consisting of T3 RNA polymerase, T7 RNA polymerase and SP6 RNA polymerase.

11. The method of any one of claims 1 to 10, wherein the 3'-UTR comprises a cleavage site for at least one catalytic nucleic acid molecule.

12. The method according to any one of claims 1 to 11, wherein the 3'-UTR comprises at least one histone stem-loop sequence,
wherein the at least one cleavage site for the at least one catalytic nucleic acid molecule is preferably located 5' of the histone stem-loop sequence, or
wherein the at least one cleavage site for the at least one catalytic nucleic acid molecule is preferably located in the histone stem-loop sequence.

13. The method according to any one of claims 1 to 12, wherein the homopolymeric sequence is preferably a poly(A) sequence or a poly(C) sequence.

14. The method according to any one of claims 1 to 13, wherein the 3'-UTR comprises a nucleic acid sequence, which is heterologous with respect to at least one selected from the group consisting of a 5'-UTR, an ORF and a further nucleic acid sequence comprised in the 3'-UTR,
and/or
wherein the 3'-UTR comprises a nucleic acid sequence derived from the 3'-UTR of a gene selected from the group consisting of an albumin gene, a globin gene and a ribosomal protein gene,
and/or
wherein the method comprises determining the length of the 3'-UTR or a fragment thereof.

15. The method according to any of claims 1 to 14, wherein the homopolymeric sequence is located between the most 3' cleavage site for the catalytic nucleic acid molecule and the 3' end of the mRNA molecule having a cleavage site for the catalytic nucleic acid molecule.

16. The method according to any one of claims 1 to 14, wherein the mRNA molecule having at least one cleavage site for at least one catalytic nucleic acid molecule comprises at least one modification.

17. The method according to any one of claims 1 to 16, wherein step c) additionally comprises analyzing of the 5' terminal RNA fragment,
wherein analyzing of the 5' terminal RNA fragment preferably comprises determining the presence of a 5'-CAP structure, wherein the orientation of the 5'-CAP structure is more preferably determined.

18. The method according to any one of claims 2 to 17, wherein the population comprises at least two different mRNA molecules having at least one cleavage site for at least one catalytic nucleic acid molecule, wherein the at least two different mRNA molecules have different lengths, and wherein step c) comprises separating the 3' terminal RNA fragments and/or the optional central RNA fragments of the at least two different mRNA molecules depending on their respective lengths.

19. The method according to any one of claims 1 to 18, wherein the 3' terminal RNA fragment and/or the at least one optional central RNA fragment has a length that differs from the length of a reference RNA fragment,
wherein the different length is preferably due to the deletion or addition of at least one nucleotide in the mRNA molecule having at least one cleavage site for at least one catalytic nucleic acid molecule,
and/or
wherein the different length is preferably due to the deletion or addition of at least one nucleotide at the 3' end of the mRNA or preferably due to the deletion or addition of at least one nucleotide within the 3' terminal RNA fragment, wherein the different length is more preferably due to the deletion or addition of at least one nucleotide in a homopolymeric sequence.

20. The method according to any one of claims 18 or 19, wherein the difference in length of 3' terminal RNA fragments or optional central RNA fragments separated in step c) is 75 nucleotides or less,
wherein step d) preferably comprises calculating the ratio of the amount of a first mRNA molecule having at least one cleavage site for at least one catalytic nucleic acid molecule and having a distinct length to the amount of a second mRNA molecule having at least one cleavage site for at leat one catalytic nucleic acid molecule and having a length that differs from the length of the first mRNA molecule,
or
wherein the at least one mRNA molecule having at least one cleavage site for at least one catalytic nucleic acid molecule is preferably produced by *in vitro* transcription and step d) preferably comprises calculating the ratio of the amount of mRNA molecules having at least one cleavage site for at least one catalytic nucleic acid molecule and having the length of the corresponding nucleic acid sequence in the nucleic acid molecule used as template in *in vitro* transcription to the amount of mRNA molecules having at least one cleavage site for at least one catalytic nucleic acid molecule and having a length that differs from the length of the corresponding nucleic acid sequence in the nucleic acid molecule used as template in *in vitro* transcription,
and/or
wherein the amounts of the 3' terminal RNA fragments or of the optional central RNA fragments having a distinct length are determined in step c) by spectroscopic methods, quantitative mass spectrometry, or sequencing.

21. Use of a catalytic nucleic acid molecule in a method as defined in any one of claims 1 to 20.

## Patentansprüche

1. Ein Verfahren zur Bestimmung des Vorhandenseins und/oder der Integrität einer homopolymeren Sequenz in der 3'-UTR eines mRNA-Moleküls, wobei das mRNA-Molekül mindestens eine Schnittstelle für mindestens ein katalytisches Nukleinsäuremolekül aufweist, wobei das mRNA-Molekül aus einer in vitro-Transkriptionsreaktion stammt und wobei mindestens eine Schnittstelle 5' von der homopolymeren Sequenz angeordnet ist, wobei das Verfahren die folgenden Schritte umfasst:
a) Bereitstellen des mRNA-Moleküls mit mindestens einer Schnittstelle für mindestens ein katalytisches Nukleinsäuremolekül, wobei mindestens eine Schnittstelle 5' von der homopolymeren Sequenz in der 3'-UTR des mRNA-Moleküls angeordnet ist,
b) Schneiden des mRNA-Moleküls mit dem mindestens einen katalytischen Nukleinsäuremolekül in ein 5'-terminales RNA-Fragment, ein 3'-terminales RNA-Fragment und gegebenenfalls in mindestens ein zentrales RNA-Fragment durch In-Kontakt-Bringen des mRNA-Moleküls mit dem mindestens einen katalytischen Nukleinsäuremolekül unter Bedingungen, die das Schneiden des mRNA-Moleküls erlauben, und
c) Bestimmen des Vorhandenseins und/oder der Integrität der homopolymeren Sequenz in der 3'-UTR des mRNA-Moleküls durch Analysieren des 3'-terminalen RNA-Fragments und gegebenenfalls des mindestens einen optionalen zentralen RNA-Fragments, das in Schritt b) erhalten wurde, wobei Schritt c) das Trennen des 3'-terminalen RNA-Fragments durch denaturierende Gelelektrophorese oder Flüssigkeitschromatographie, das Bestimmen der Masse und/oder der Länge des 3'-terminalen RNA-Fragments und das Vergleichen der bestimmten Masse und/oder Länge des 3'-terminalen RNA-Fragments mit einer entsprechenden Masse und/oder Länge eines Referenz-RNA-Fragments umfasst.

2. Ein Verfahren zur Bestimmung des Vorhandenseins und/oder der Integrität einer homopolymeren Sequenz in der 3'-UTR einer Population von mRNA-Molekülen, wobei die Population mindestens ein mRNA-Molekül umfasst, das eine 3'-UTR aufweist, die mindestens eine homopolymere Sequenz umfasst, wobei die mRNA-Moleküle aus in vitro-Transkriptionsreaktionen stammen und wobei das mRNA-Molekül mindestens eine Schnittstelle für mindestens ein katalytisches Nukleinsäuremolekül aufweist, wobei mindestens eine Schnittstelle 5' von der homopolymeren Sequenz angeordnet ist, wobei das Verfahren die folgenden Schritte umfasst:
a) Bereitstellen der Probe, die die Population von mRNA-Molekülen enthält, wobei das mindestens eine mRNA-Molekül, das eine 3'-UTR umfasst, die mindestens eine homopolymere Sequenz umfasst, mindestens eine Schnittstelle für mindestens ein katalytisches Nukleinsäuremolekül aufweist, wobei mindestens eine Schnittstelle 5' von der homopolymeren Sequenz in der 3'-UTR des mRNA-Moleküls angeordnet ist,
b) Schneiden des mindestens einen mRNA-Moleküls mit mindestens einer Schnittstelle für mindestens ein katalytisches Nukleinsäuremolekül, wobei mindestens eine Schnittstelle 5' von der homopolymeren Sequenz liegt, mit mindestens einem katalytischen Nukleinsäuremolekül in ein 3'-terminales RNA-Fragment, ein 5'-RNA-Fragment und gegebenenfalls in mindestens ein zentrales RNA-Fragment, indem die Probe mit mindestens einem katalytischen Nukleinsäuremolekül unter Bedingungen in Kontakt gebracht wird, die das Schneiden des mRNA-Moleküls ermöglichen,
c) Bestimmen des Vorhandenseins und/oder der Integrität der homopolymeren Sequenz in der 3'-UTR des mindestens einen mRNA-Moleküls mit mindestens einer Schnittstelle für mindestens ein katalytisches Nukleinsäuremolekül durch Analysieren des 3'-terminalen RNA-Fragments und gegebenenfalls des mindestens einen optionalen zentralen RNA-Fragments, das in Schritt b) erhalten wurde, wobei Schritt c) das Trennen des 3'-terminalen RNA-Fragments durch denaturierende Gelelektrophorese oder Flüssigkeitschromatographie, das Bestimmen der Masse und/oder der Länge des 3'-terminalen RNA-Fragments, das Vergleichen der bestimmten Masse und/oder Länge des 3'-terminalen RNA-Fragments mit einer entsprechenden Masse und/oder Länge eines Referenz-RNA-Fragments und
d) gegebenenfalls die Bestimmung der relativen Menge der verschiedenen mRNA-Moleküle in der Population.

3. Verfahren nach Anspruch 1 oder 2, wobei das katalytische Nukleinsäuremolekül so konzipiert ist, dass es das mRNA-Molekül an mindestens einer spezifischen Schnittstelle schneiden kann.

4. Verfahren nach einem der Ansprüche 1 bis 3, wobei das mRNA-Molekül mit mindestens einer Schnittstelle für mindestens ein katalytisches Nukleinsäuremolekül so konzipiert ist, dass es mindestens eine Schnittstelle für mindestens ein katalytisches Nukleinsäuremolekül aufweist.

5. Verfahren nach einem der Ansprüche 1 bis 4, wobei mindestens eine Schnittstelle des katalytischen Nukleinsäuremoleküls innerhalb von 250 Nukleotiden vom 3'-Terminus des mRNA-Moleküls angeordnet ist.

6. Verfahren nach einem der Ansprüche 1 bis 5, wobei das mindestens eine katalytische Nukleinsäuremolekül ein Ribozym ist, das vorzugsweise aus der Gruppe ausgewählt ist, die aus Hammerhead-Ribozymen, Haarnadel-Ribozymen und HDV-Ribozymen besteht.

7. Verfahren nach einem der Ansprüche 1 bis 6, wobei das mindestens eine katalytische Nukleinsäuremolekül in Schritt b) in *trans* bereitgestellt wird.

8. Verfahren nach einem der Ansprüche 1 bis 7, wobei Schritt b) die Denaturierung des mRNA-Moleküls mit mindestens einer Schnittstelle für mindestens ein katalytisches Nukleinsäuremolekül und das Annealing des mindestens einen katalytischen Nukleinsäuremoleküls an das mRNA-Molekül umfasst.

9. Verfahren nach einem der Ansprüche 1 bis 8, wobei Schritt c) die Auftrennung der RNA-Fragmente durch HPLC, FPLC oder RPLC umfasst,
und/oder
wobei Schritt c) die Trennung der optionalen zentralen RNA-Fragmente umfasst und wobei die optionalen zentralen RNA-Fragmente durch denaturierende Gelelektrophorese oder Flüssigkeitschromatographie, vorzugsweise HPLC, FPLC oder RPLC, getrennt werden,
und/oder
wobei Schritt c) die Analyse eines Strukturmerkmals oder eines physikalischen Parameters des mindestens einen optionalen zentralen RNA-Fragments umfasst, und/oder
wobei Schritt c) den Vergleich eines Strukturmerkmals oder eines physikalischen Parameters des mindestens einen optionalen zentralen RNA-Fragments mit einem entsprechenden Merkmal oder Parameter eines Referenz-RNA-Fragments umfasst,
und/oder
wobei Schritt c) die Bestimmung der Identität und/oder der Integrität des 3'-terminalen RNA-Fragments und/oder des mindestens einen optionalen zentralen RNA-Fragments umfasst,
und/oder
wobei Schritt c) die Bestimmung der Masse und/oder der Länge des mindestens einen optionalen zentralen RNA-Fragments umfasst, wobei die Länge des mindestens einen optionalen zentralen RNA-Fragments vorzugsweise 250 Nukleotide oder weniger beträgt,
und/oder
wobei Schritt c) die spektroskopische Analyse, die quantitative Massenspektrometrie oder die Sequenzanalyse umfasst.

10. Verfahren nach einem der Ansprüche 1 bis 9, wobei die in vitro-Transkription unter Verwendung einer Bakteriophagen-RNA-Polymerase durchgeführt wird, wobei die Bakteriophagen-RNA-Polymerase vorzugsweise ausgewählt ist aus der Gruppe, bestehend aus T3-RNA-Polymerase, T7-RNA-Polymerase und SP6-RNA-Polymerase.

11. Verfahren nach einem der Ansprüche 1 bis 10, wobei die 3'-UTR eine Schnittstelle für mindestens ein katalytisches Nukleinsäuremolekül umfasst.

12. Verfahren nach einem der Ansprüche 1 bis 11, wobei die 3'-UTR mindestens eine Histon-Stamm-Schleifen-Sequenz umfasst,
wobei die mindestens eine Schnittstelle für das mindestens eine katalytische Nukleinsäuremolekül vorzugsweise 5' von der Histon-Stammschleifen-Sequenz angeordnet ist, oder
wobei die mindestens eine Schnittstelle für das mindestens eine katalytische Nukleinsäuremolekül vorzugsweise in der Histon-Stammschleifen-Sequenz angeordnet ist.

13. Verfahren nach einem der Ansprüche 1 bis 12, wobei die homopolymere Sequenz vorzugsweise eine Poly(A)-Sequenz oder eine Poly(C)-Sequenz ist.

14. Verfahren nach einem der Ansprüche 1 bis 13, wobei die 3'-UTR eine Nukleinsäuresequenz umfasst, die heterolog ist in Bezug auf mindestens eine aus der Gruppe, bestehend aus einer 5'-UTR, einem ORF und einer weiteren Nukleinsäuresequenz, die in der 3'-UTR enthalten ist,
und/oder
wobei die 3'-UTR eine Nukleinsäuresequenz umfasst, die sich von der 3'-UTR eines Gens ableitet, das aus der Gruppe ausgewählt ist, die aus einem Albumin-Gen, einem Globin-Gen und einem ribosomalen Protein-Gen besteht,
und/oder
wobei das Verfahren die Bestimmung der Länge der 3'-UTR oder eines Fragments davon umfasst.

15. Verfahren nach einem der Ansprüche 1 bis 14, wobei die homopolymere Sequenz zwischen der am meisten 3' gelegenen Schnittstelle für das katalytische Nukleinsäuremolekül und dem 3'-Ende des mRNA-Moleküls mit einer Schnittstelle für das katalytische Nukleinsäuremolekül angeordnet ist.

16. Verfahren nach einem der Ansprüche 1 bis 14, wobei das mRNA-Molekül mit mindestens einer Schnittstelle für mindestens ein katalytisches Nukleinsäuremolekül mindestens eine Modifikation umfasst.

17. Verfahren nach einem der Ansprüche 1 bis 16, wobei Schritt c) zusätzlich das Analysieren des 5'-terminalen RNA-Fragments umfasst,
wobei das Analysieren des 5'-terminalen RNA-Fragments vorzugsweise das Bestimmen des Vorhandenseins einer 5'-CAP-Struktur umfasst, wobei besonders bevorzugt die Orientierung der 5'-CAP-Struktur bestimmt wird.

18. Verfahren nach einem der Ansprüche 2 bis 17, wobei die Population mindestens zwei verschiedene mRNA-Moleküle umfasst, die mindestens eine Schnittstelle für mindestens ein katalytisches Nukleinsäuremolekül aufweisen, wobei die mindestens zwei verschiedenen mRNA-Moleküle unterschiedliche Längen aufweisen, und wobei Schritt c) das Abtrennen der 3'-terminalen RNA-Fragmente und/oder der optionalen zentralen RNA-Fragmente der mindestens zwei verschiedenen mRNA-Moleküle in Abhängigkeit von ihren jeweiligen Längen umfasst.

19. Verfahren nach einem der Ansprüche 1 bis 18, wobei das 3'-terminale RNA-Fragment und/oder das mindestens eine optionale zentrale RNA-Fragment eine Länge aufweist, die sich von der Länge eines Referenz-RNA-Fragments unterscheidet,
wobei die unterschiedliche Länge vorzugsweise auf die Deletion oder Addition von mindestens einem Nukleotid in dem mRNA-Molekül mit mindestens einer Schnittstelle für mindestens ein katalytisches Nukleinsäuremolekül zurückzuführen ist,
und/oder
wobei die unterschiedliche Länge vorzugsweise auf die Deletion oder Addition von mindestens einem Nukleotid am 3'-Ende der mRNA oder vorzugsweise auf die Deletion oder Addition von mindestens einem Nukleotid innerhalb des 3'-terminalen RNA-Fragments zurückzuführen ist, wobei die unterschiedliche Länge noch bevorzugter auf die Deletion oder Addition von mindestens einem Nukleotid in einer homopolymeren Sequenz zurückzuführen ist.

20. Verfahren nach einem der Ansprüche 18 oder 19, wobei der Längenunterschied der in Schritt c) getrennten 3'-terminalen RNA-Fragmente oder optionalen zentralen RNA-Fragmente 75 Nukleotide oder weniger beträgt,
wobei Schritt d) vorzugsweise das Berechnen des Verhältnisses der Menge eines ersten mRNA-Moleküls mit mindestens einer Schnittstelle für mindestens ein katalytisches Nukleinsäuremolekül und mit einer abweichenden Länge zu der Menge eines zweiten mRNA-Moleküls mit mindestens einer Schnittstelle für mindestens ein katalytisches Nukleinsäuremolekül und mit einer Länge, die sich von der Länge des ersten mRNA-Moleküls unterscheidet, umfasst,
oder
wobei das mindestens eine mRNA-Molekül mit mindestens einer Schnittstelle für mindestens ein katalytisches Nukleinsäuremolekül vorzugsweise durch in vitro-Transkription hergestellt wird und Schritt d) vorzugsweise das Berechnen des Verhältnisses der Menge der mRNA-Moleküle mit mindestens einer Schnittstelle für mindestens ein katalytisches Nukleinsäuremolekül und mit der Länge der entsprechenden Nukleinsäuresequenz in dem Nukleinsäuremolekül, das als Matrize bei der in vitro-Transkription verwendet wird, zu der Menge der mRNA-Moleküle mit mindestens einer Schnittstelle für mindestens ein katalytisches Nukleinsäuremolekül und mit einer Länge, die sich von der Länge der entsprechenden Nukleinsäuresequenz in dem Nukleinsäuremolekül, das als Matrize bei der in vitro-Transkription verwendet wird, unterscheidet,
und/oder
wobei die Mengen der 3'-terminalen RNA-Fragmente oder der optionalen zentralen RNA-Fragmente mit einer unterschiedlichen Länge in Schritt c) durch spektroskopische Methoden, quantitative Massenspektrometrie oder Sequenzierung bestimmt werden.

21. Verwendung eines katalytischen Nukleinsäuremoleküls in einem Verfahren, wie in einem der Ansprüche 1 bis 20 definiert.

## Revendications

1. Procédé de détermination de la présence et/ou de l'intégrité d'une séquence homopolymère dans la région 3'-UTR d'une molécule d'ARNm, la molécule d'ARNm ayant au moins un site de clivage pour au moins une molécule d'acide nucléique catalytique, dans lequel la molécule d'ARNm est dérivée d'une réaction de transcription *in vitro* et dans lequel au moins un site de clivage est situé du côté 5' de la séquence homopolymère, le procédé comprenant les étapes de :
a) fourniture de la molécule d'ARNm ayant au moins un site de clivage pour au moins une molécule d'acide nucléique catalytique, dans lequel au moins un site de clivage est situé du côté 5' de la séquence homopolymère dans la région 3'-UTR de la molécule d'ARNm,
b) clivage de la molécule d'ARNm avec au moins une molécule d'acide nucléique catalytique en un fragment d'ARN de 5'-terminal, un fragment d'ARN 3'-terminal et facultativement en un au moins un fragment d'ARN central par mise en contact de la molécule d'ARNm avec au moins une molécule d'acide nucléique catalytique dans des conditions permettant le clivage de la molécule d'ARNm, et
c) détermination de la présence et/ou de l'intégrité de la séquence homopolymère dans la région 3'-UTR de la molécule d'ARNm par analyse du fragment d'ARN 3'-terminal et facultativement du au moins un fragment d'ARN central facultatif obtenu dans l'étape b), dans lequel l'étape c) comprend la séparation du fragment d'ARN 3'-terminal par électrophorèse sur gel dénaturant ou chromatographie en phase liquide, détermination de la masse et/ou de la longueur du fragment d'ARN 3'-terminal et comparaison de la masse et/ou longueur déterminée(s) du fragment d'ARN 3'-terminal avec une masse et/ou longueur respective(s) d'un fragment d'ARN de référence.

2. Procédé de détermination de la présence et/ou de l'intégrité d'une séquence homopolymère dans la région 3'-UTR d'une population de molécules d'ARNm, dans lequel la population comprend au moins une molécule d'ARNm comprenant une région 3'-UTR, qui comprend au moins une séquence homopolymère, dans lequel les molécules d'ARNm sont dérivées de réactions de transcription *in vitro* et dans lequel la molécule d'ARNm a au moins un site de clivage pour au moins une molécule d'acide nucléique catalytique, dans lequel au moins un site de clivage est situé du côté 5' de la séquence homopolymère, le procédé comprenant les étapes de :
a) fourniture de l'échantillon contenant la population de molécules d'ARNm, dans lequel l'au moins une molécule d'ARNm comprenant une région 3'-UTR, qui comprend au moins une séquence homopolymère, a au moins un site de clivage pour au moins une molécule d'acide nucléique catalytique, dans lequel au moins un site de clivage est situé du côté 5' de la séquence homopolymère dans la région 3'-UTR de la molécule d'ARNm,
b) clivage de l'au moins une molécule d'ARN ayant au moins un site de clivage pour au moins une molécule d'acide nucléique catalytique, dans lequel au moins un site de clivage est situé du côté 5' de la séquence homopolymère, avec au moins une molécule d'acide nucléique catalytique en un fragment d'ARN 3'-terminal, un fragment d'ARN 5' et facultativement dans au moins un fragment d'ARN central par mise en contact de l'échantillon avec au moins une molécule d'acide nucléique catalytique dans des conditions permettant le clivage de la molécule d'ARNm,
c) détermination de la présence et/ou de l'intégrité de la séquence homopolymère dans la région 3'-UTR de l'au moins une molécule d'ARNm ayant au moins un site de clivage pour au moins une molécule d'acide nucléique catalytique par analyse du fragment d'ARN 3'-terminal et facultativement du au moins un fragment d'ARN central facultatif obtenu dans l'étape b), dans lequel l'étape c) comprend la séparation du fragment d'ARN 3'-terminal par électrophorèse sur gel dénaturant ou chromatographie en phase liquide, détermination de la masse et/ou de la longueur du fragment d'ARN 3'-terminal et comparaison de la masse et/ou longueur déterminée(s) du fragment d'ARN 3'-terminal avec une masse et/ou longueur respective(s) d'un fragment d'ARN de référence et
d) facultativement, détermination de la quantité relative des différentes molécules d'ARN dans la population.

3. Procédé selon la revendication 1 ou 2, dans lequel la molécule d'acide nucléique catalytique a été conçue pour être capable de cliver la molécule d'ARNm au niveau d'au moins un site de clivage spécifique.

4. Procédé selon l'une quelconque des revendications 1 à 3, dans lequel la molécule d'ARNm ayant au moins un site de clivage pour au moins une molécule d'acide nucléique catalytique a été conçue pour avoir au moins un site de clivage pour au moins une molécule d'acide nucléique catalytique.

5. Procédé selon l'une quelconque des revendications 1 à 4, dans lequel au moins un site de clivage de la molécule d'acide nucléique catalytique est situé parmi les 250 nucléotides à partir de l'extrémité 3' de la molécule d'ARNm.

6. Procédé selon l'une quelconque des revendications 1 à 5, dans lequel l'au moins une molécule d'acide nucléique catalytique est un ribozyme, de préférence choisi dans le groupe constitué par les ribozymes en tête de marteau, les ribozymes en épingle à cheveux et les ribozymes VHD.

7. Procédé selon l'une quelconque des revendications 1 à 6, dans lequel l'au moins une molécule d'acide nucléique catalytique est fournie dans l'étape b) sous forme *trans.*

8. Procédé selon l'une quelconque des revendications 1 à 7, dans lequel l'étape b) comprend la dénaturation de la molécule d'ARNm ayant au moins un site de clivage pour au moins une molécule d'acide nucléique catalytique et l'hybridation d'au moins une molécule d'acide nucléique catalytique à ladite molécule d'ARNm.

9. Procédé selon l'une quelconque des revendications 1 à 8, dans lequel l'étape c) comprend la séparation des fragments d'ARN par HPLC, FPLC ou RPLC,
et/ou
dans lequel l'étape c) comprend la séparation des fragments d'ARN central facultatifs et dans lequel les fragments d'ARN central facultatifs sont séparés par électrophorèse sur gel dénaturant ou chromatographie en phase liquide, de préférence HPLC, FPLC ou RPLC,
et/ou
dans lequel l'étape c) comprend l'analyse d'une caractéristique structurelle ou d'un paramètre physique de l'au moins un fragment d'ARN central facultatif, et/ou
dans lequel l'étape c) comprend la comparaison d'une caractéristique structurelle ou d'un paramètre physique de l'au moins un fragment d'ARN central facultatif, avec une caractéristique ou un paramètre respectif d'un fragment d'ARN de référence,
et/ou
dans lequel l'étape c) comprend la détermination de l'identité et/ou l'intégrité du fragment d'ARN 3'-terminal et/ou de l'au moins un fragment d'ARN central facultatif,
et/ou
dans lequel l'étape c) comprend la détermination de la masse et/ou de la longueur de l'au moins un fragment d'ARN central facultatif, dans lequel la longueur de l'au moins un fragment d'ARN central facultatif est de préférence de 250 nucléotides ou moins,
et/ou
dans lequel l'étape c) implique l'analyse spectroscopique, la spectrométrie de masse quantitative ou l'analyse de séquences.

10. Procédé selon l'une quelconque des revendications 1 à 9, dans lequel la transcription *in vitro* est effectuée par l'utilisation d'une ARN polymérase de bactériophage, dans lequel l'ARN polymérase de bactériophage est de préférence choisie dans le groupe constitué par l'ARN polymérase T3, l'ARN polymérase T7 et l'ARN polymérase SP6.

11. Procédé selon l'une quelconque des revendications 1 à 10, dans lequel la région 3'-UTR comprend un site de clivage pour au moins une molécule d'acide nucléique catalytique.

12. Procédé selon l'une quelconque des revendications 1 à 11, dans lequel la région 3'-UTR comprend au moins une séquence tige-boucle d'histone,
dans lequel l'au moins un site de clivage pour l'au moins une molécule d'acide nucléique catalytique est de préférence situé du côté 5' de la séquence tige-boucle d'histone, ou
dans lequel l'au moins un site de clivage pour l'au moins une molécule d'acide nucléique catalytique est de préférence situé dans la séquence tige-boucle d'histone.

13. Procédé selon l'une quelconque des revendications 1 à 12, dans lequel la séquence homopolymère est de préférence une séquence poly(A) ou une séquence poly(C).

14. Procédé selon l'une quelconque des revendications 1 à 13, dans lequel la région 3'-UTR comprend une séquence d'acides nucléiques, qui est hétérologue par rapport à au moins une choisie dans le groupe constitué par une région 5'-UTR, un cadre ORF et une autre séquence d'acides nucléiques comprise dans la région 3'-UTR,
et/ou
dans lequel la région 3'-UTR comprend une séquence d'acides nucléiques dérivée de la région 3'-UTR d'un gène choisi dans le groupe constitué par un gène d'albumine, un gène de globine et un gène de protéine ribosomale,
et/ou
dans lequel le procédé comprend la détermination de la longueur de la région 3'-UTR ou d'un fragment de celle-ci.

15. Procédé selon l'une quelconque des revendications 1 à 14, dans lequel la séquence homopolymère est située entre le site de clivage le plus du côté 3' pour la molécule d'acide nucléique catalytique et l'extrémité 3' de la molécule d'ARNm ayant un site de clivage pour la molécule d'acide nucléique catalytique.

16. Procédé selon l'une quelconque des revendications 1 à 14, dans lequel la molécule d'ARNm ayant au moins un site de clivage pour au moins une molécule d'acide nucléique catalytique comprend au moins une modification.

17. Procédé selon l'une quelconque des revendications 1 à 16, dans lequel l'étape c) comprend en plus l'analyse du fragment d'ARN 5'-terminal,
dans lequel l'analyse du fragment d'ARN 5'-terminal comprend de préférence la détermination de la présence d'une structure 5'-CAP, dans lequel l'orientation de la structure 5'-CAP est plus préférablement déterminée.

18. Procédé selon l'une quelconque des revendications 2 à 17, dans lequel la population comprend au moins deux différentes molécules d'ARNm ayant au moins un site de clivage pour au moins une molécule d'acide nucléique catalytique, dans lequel les au moins deux différentes molécules d'ARNm ont différentes longueurs, et dans l'étape c) comprend la séparation des fragments d'ARN 3'-terminal et/ou des fragments d'ARN central facultatifs de les au moins deux différentes molécules d'ARN en fonction de leurs longueurs respectives.

19. Procédé selon l'une quelconque des revendications 1 à 18, dans lequel le fragment d'ARN 3'-terminal et/ou l'au moins un fragment d'ARN central facultatif a une longueur différente de la longueur d'un fragment d'ARN de référence, dans lequel la différence de longueur est de préférence due à la délétion ou l'addition d'au moins un nucléotide dans la molécule d'ARNm ayant au moins un site de clivage pour au moins une molécule d'acide nucléique catalytique, et/ou
dans lequel la différence de longueur est de préférence due à la délétion ou l'addition d'au moins un nucléotide à l'extrémité 3' de l'ARNm ou de préférence due à la délétion ou l'addition d'au moins un nucléotide dans le fragment d'ARN 3'-terminal, dans lequel la différence de longueur est plus préférablement due à la délétion ou l'addition d'au moins un nucléotide dans une séquence homopolymère.

20. Procédé selon l'une quelconque des revendications 18 ou 19, dans lequel la différence de longueur des fragments d'ARN 3'-terminal ou des fragments d'ARN central facultatifs séparés dans l'étape c) est de 75 nucléotides ou moins, dans lequel l'étape d) comprend de préférence le calcul du rapport de la quantité d'une première molécule d'ARNm ayant au moins un site de clivage pour au moins une molécule d'acide nucléique catalytique et ayant une longueur distincte, à la quantité d'une seconde molécule d'ARNm ayant au moins un site de clivage pour au moins une molécule d'acide nucléique catalytique et ayant une longueur différente de la longueur de la première molécule d'ARNm,
ou
dans lequel l'au moins une molécule d'ARNm ayant au moins un site de clivage pour au moins une molécule d'acide nucléique catalytique est de préférence produite par transcription *in vitro* et l'étape d) comprend de préférence le calcul du rapport de la quantité de molécules d'ARNm ayant au moins un site de clivage pour au moins une molécule d'acide nucléique catalytique et ayant la longueur de la séquence d'acides nucléiques correspondante dans la molécule d'acide nucléique utilisée comme modèle dans la transcription *in vitro,* à la quantité de molécules d'ARNm ayant au moins un site de clivage pour au moins une molécule d'acide nucléique catalytique et ayant une longueur différente de la longueur de la séquence d'acides nucléiques correspondante dans la molécule d'acide nucléique utilisée comme modèle dans la transcription *in vitro,*
et/ou
dans lequel les quantités des fragments d'ARN 3'-terminal ou des fragments d'ARN central facultatifs ayant une longueur distincte sont déterminées dans l'étape c) par des méthodes spectroscopiques, une spectrométrie de masse quantitative ou un séquençage.

21. Utilisation d'une molécule d'acide nucléique catalytique dans un procédé tel que défini dans l'une quelconque des revendications 1 à 20.
